(19) Europäisches Patentamt
European Patent Office
Office européen des brevets

(11) Publication number: 0 238 178 B1

# (12) EUROPEAN PATENT SPECIFICATION

(45) Date of publication of the patent specification: 27.06.90

(51) Int. Cl.[5]: C07H 17/08, A61K 31/70

(21) Application number: 87300807.2

(22) Date of filing: 29.01.87

(54) **Novel derivatives of erythromycylamine.**

(30) Priority: 03.02.86 US 824911

(43) Date of publication of application: 23.09.87 Bulletin 87/39

(45) Publication of the grant of the patent: 27.06.90 Bulletin 90/26

(84) Designated Contracting States: AT BE CH DE ES FR GB GR IT LI LU NL SE

(56) References cited:
FR-A- 2 306 704
GB-A- 1 345 524

JOURNAL OF MEDICINAL CHEMISTRY, vol. 16, no. 9, September 1973, pages 1059-1060; R. RYDEN et al.: "N-substituted derivatives of erythromycylamine"

(73) Proprietor: ELI LILLY AND COMPANY, Lilly Corporate Center, Indianapolis Indiana 46285(US)

(72) Inventor: Bonjouklian, Rosanne, 318 Dominion Drive, Zionsville Indiana 46077(US)
Inventor: Debono, Manuel, 5257 Hinesley Avenue, Indianapolis Indiana 46208(US)
Inventor: Kirst, Herbert Andrew, 1819 Madison Village Drive Apt. B-6, Indianapolis Indiana 46227(US)
Inventor: Wind, Julie Ann, 898 Dover Drive Apt. 909, Greenwood Indiana 46142(US)

(74) Representative: Hudson, Christopher Mark et al, Erl Wood Manor, Windlesham Surrey GU20 6PH(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to new 9-N-substituted derivatives of erythromycylamine. The new derivatives are orally active against Gram-positive microorganisms and are, therefore, quite useful for treating infections caused by these organisms. The activity of the new derivatives is superior to that of erythromycylamine. Some of the new derivatives are also useful intermediates to other active compounds.

Some cyclic carbinolamine ether and arylidene derivatives at the 9-position of erythromycylamine are disclosed in J. Med. Chem., Vol. 16, pages 1059–1060 (1973). Other 9-N-substituted heteroarylidene and alkylamino derivatives are disclosed in British Patent 1 345 524 and French Patent 2 306 704.

This invention also provides new processes for preparing derivatives of erythromycylamine and aliphatic aldehydes. One of the new processes involves controlling the pH during the reduction step, and the other involves catalytic hydrogenation, preferably at higher temperatures.

In other aspects, this invention relates to novel compositions comprising the new erythromycylamine derivatives.

New 9-N-substituted derivatives of erythromycylamine are provided by this invention. The first derivatives have the structure shown in formula 1.

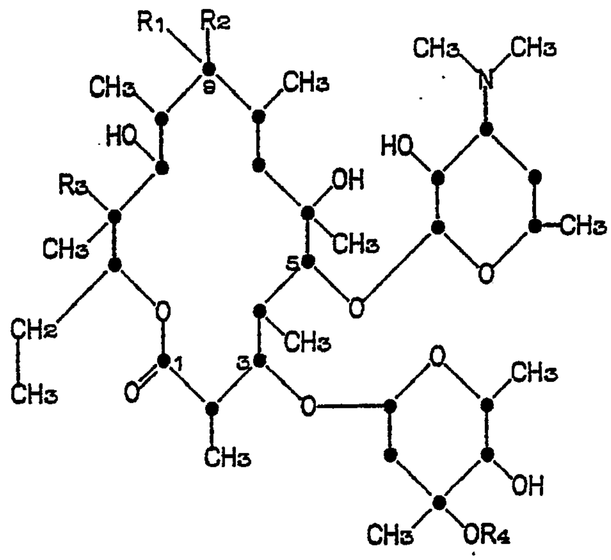

1

wherein $R_1$ and $R_2$ are different and are hydrogen or $-NHCH_2R_5$;
$R_3$ is hydrogen or hydroxyl;
$R_4$ is hydrogen or methyl; and
$R_5$ is hydrogen or a $C_1$-$C_{14}$-alkyl or $-(CH_2)_lX(CH_2)_mY$ group, either of which group may have from one to three substituents selected from halo, hydroxyl, $C_1$-$C_4$-alkyl, $C_1$-$C_4$-alkoxy, $C_1$-$C_4$-alkylthio, cyano, $C_1$-$C_4$-alkoxycarbonyl, mono- or di($C_1$-$C_4$-alkyl)amino, $-N(CH_2)_s$, $R_6$-substituted-phenyl, or an $R_6$-substituted-monocyclic heterocyclic group having from 3 to 7 ring atoms;
X is oxygen or sulfur;
Y is $-X(CH_2)_nCH_3$, $-N(CH_2)_s$ or $-N[(CH_2)_nCH_3]_2$;
l is 1 or 2;
m is an integer from 1 to 3;
n is an integer from 0 to 3;
s is an integer from 2 to 7; and
$R_6$ is hydrogen, halo, $C_1$-$C_4$-alkyl or $C_1$-$C_4$-alkoxy;
provided that, when the substituent on the $R_5$ group is selected from hydroxyl, cyano, alkoxycarbonyl, mono- or dialkylamino or $-N(CH_2)_s$, it cannot be located on the second or third carbon atom from the nitrogen of the $-NHCH_2R_5$ group unless the second carbon atom is quaternary;
or a salt of these compounds.

Other new derivatives of this invention are (9-N-alkyl-erythromycylamine)-formaldehyde adducts which have the structure shown in formula 2:

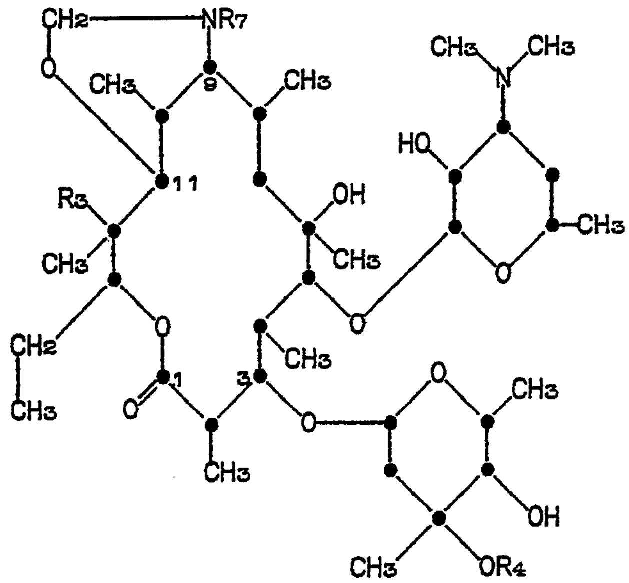

2

wherein $R_3$ and $R_4$ are as defined supra;
$R_7$ is $CH_2R_5$ as defined supra, $C_3$-$C_8$-cycloalkyl, $-CHR_8(CH_2)_pR_9$, $-(CH_2)_qR_{10}$ or $-CH_2(CH=CH)_rAr$;
$R_8$ is $C_1$-$C_4$-alkyl, phenyl or benzyl;
$R_9$ is hydrogen, halo, hydroxy, $C_1$-$C_4$-alkoxy, mono- or di($C_1$-$C_4$-alkyl)amino, $-N(CH_2)_s$ or phenyl
$R_{10}$ is hydroxy, cyano, $C_1$-$C_4$-alkoxycarbonyl, mono- or di($C_1$-$C_4$-alkyl)amino or $-N(CH_2)_s$;
Ar is phenyl; phenyl having one or more halo, $C_1$-$C_4$-alkyl, $C_1$-$C_4$-alkoxy or hydroxy substituents; or an $R_6$-substituted monocyclic aromatic heterocyclic group having from 5 to 7 ring atoms;
p is an integer from 1 to 5;
q is 2 or 3;
r is 0 or 1; and
s is as defined supra;
or a salt of these compounds.

The third group of erythromycylamine compounds of this invention have formula 3:

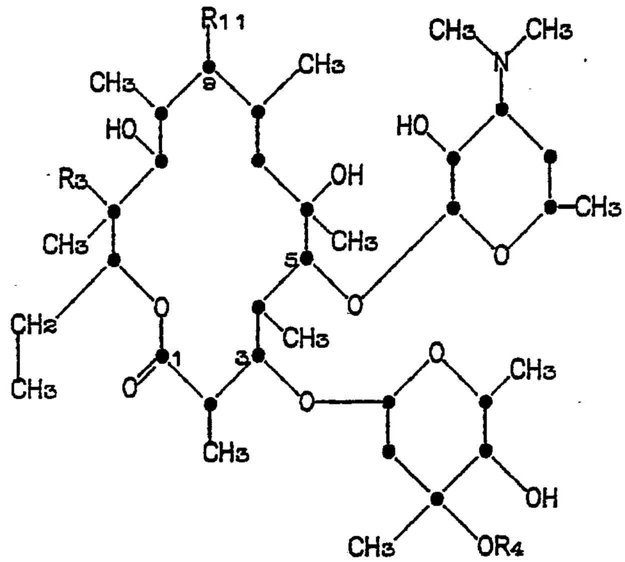

3

wherein $R_{11}$ is $-N(CH_3)R_7$ or $-N(CH_2)_s$; and
$R_3$, $R_4$, $R_7$ and s are as defined supra; or a salt of these compounds.

Although no stereochemical assignments are indicated in the structures given herein, the stereochemistry is identical to that of the antibiotics from which the compounds are prepared, e.g., erythromycins A, B, C and D. In formula 1, the $R_2$-substituent is on the same side of the ring as the 6-, 11- and 12-hydroxyl groups. In formulas 2 and 3, the stereochemistry of the 9-amino group is that of erythromycylamine, i.e., the 9-amino group and the 11-hydroxyl group are on the same side of the lactone ring.

"Alkyl" includes straight, branched and cyclic hydrocarbon moieties and combinations thereof containing the specified number of carbon atoms. Such groups can be saturated or unsaturated (alkenyl or alkynyl). When unsaturated, the alkyl group may contain from 1 to 3 double and/or triple bonds. The double bonds can be in either the cis or trans configuration.

"Halo" means chloro, bromo, iodo or fluoro.

"Monocyclic heterocyclic group containing from 3 to 7 ring atoms" refers to a heterocyclic ring which may be saturated or unsaturated and which contains at least 2 carbon atoms. In these groups, the heteroatom or atoms are selected from nitrogen, oxygen and sulfur. A "monocyclic aromatic heterocyclic group" refers to a heterocyclic ring which is unsaturated and aromatic in nature, but is otherwise as defined supra.

Typical monocyclic aromatic heterocyclic groups are pyridinyl, pyrimidinyl, pyridazinyl, pyrazinyl, imidazolyl, pyrazolyl, pyrrolyl, furanyl, oxazolyl, thiazolyl, thienyl and the like. Typical monocyclic saturated heterocyclic groups are piperidinyl, pyrrolidinyl, tetrahydrofuranyl, tetrahydropyranyl, morpholinyl, thiomorpholinyl, N-methylpiperazinyl and the like.

As used " a quaternary carbon atom" is a fully substituted carbon atom, that is, a carbon atom which has no hydrogen atoms bonded to it.

The new derivatives of this invention are prepared from the well-known erythromycin antibiotics, which have the structures shown in formulas 4a-4d:

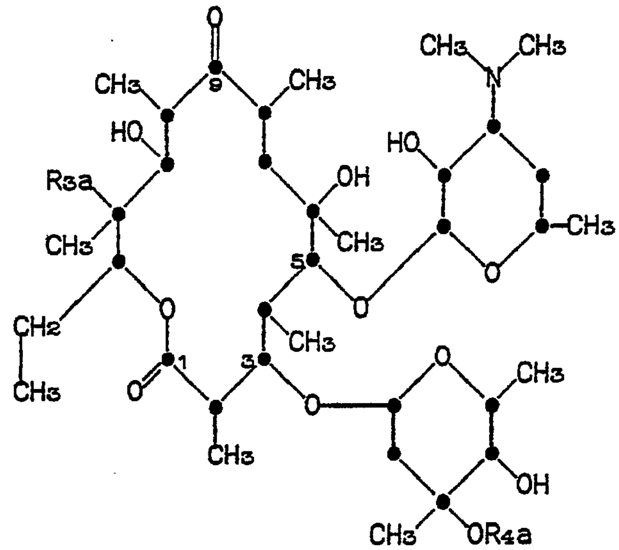

| | | $R_3a$ | $R_4a$ |
|---|---|---|---|
| 4a: | Erythromycin A | OH | $CH_3$ |
| 4b: | Erythromycin B | H | $CH_3$ |
| 4c: | Erythromycin C | OH | H |
| 4d: | Erythromycin D | H | H |

Erythromycin A, also known as erythromycin (see U.S. Patent 2,653,899), is a successful commercial antibiotic. Erythromycins B, C and D are minor components of the erythromycin fermentation (see U.S. Patents 2,834,714 and 4,496,546).

The erythromycylamines are prepared from erythromycins A-D [see, for example, Massey and Kitchell, U.S. Patent 3,652,537; and Wildsmith, U.S. Patent Nos. 3,790,559 and 3,780,019]. The erythromycylamines are shown in formulas 5a through 5h:

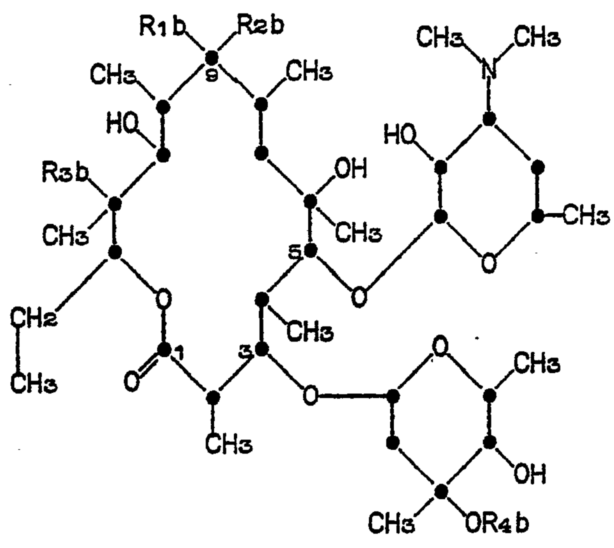

| | | $R_1b$ | $R_2b$ | $R_3b$ | $R_4b$ |
|---|---|---|---|---|---|
| 5a | Erythromycylamine A | H | $NH_2$ | OH | $CH_3$ |
| 5b | epi-Erythromycylamine A | $NH_2$ | H | OH | $CH_3$ |
| 5c | Erythromycylamine B | H | $NH_2$ | H | $CH_3$ |
| 5d | epi-Erythromycylamine B | $NH_2$ | H | H | $CH_3$ |
| 5e | Erythromycylamine C | H | $NH_2$ | OH | H |
| 5f | epi-Erythromycylamine C | $NH_2$ | H | OH | H |
| 5g | Erythromycylamine D | H | $NH_2$ | H | H |
| 5h | epi-Erythromycylamine D | $NH_2$ | H | H | H |

Prior to this invention, it was known that erythromycylamine could be condensed with ketones and aromatic aldehydes to produce the corresponding Schiff's bases. These Schiff's bases could then be reduced by standard methods, using a standard reducing agent such as sodium cyanoborohydride, sodium borohydride or hydrogenation, to give the corresponding N-alkyl derivatives (see, for example, U.S. 3,794,635). When erythromycylamine was condensed with aliphatic aldehydes, however, the corresponding Schiff's bases were not obtained. Instead, an oxazine was formed (see, for example, Kitchell and Gerzon, U.S. Patent 3,681,322). Kitchell and Gerzon believed that the oxazine involved the hydroxyl group at C-6, but suggested that it could alternately involve the C-11 or C-12 hydroxyl group (see U.S. 3,681,322 at column 2). Later, evidence led to the conclusion that the Kitchell and Gerzon compounds involve the C-11 hydroxyl group as shown in formula 6:

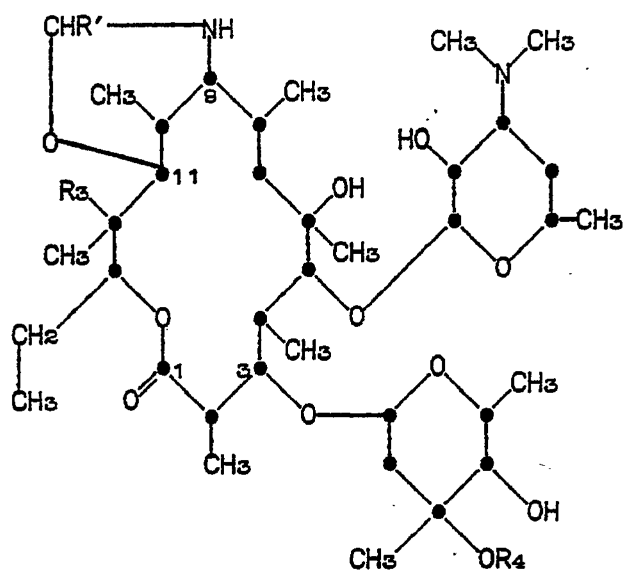

6

After Kitchell and Gerzon's work, Maier and others found that an even wider range of aliphatic aldehydes did not form Schiff's bases, but instead formed oxazine derivatives (see U.S. Patent 4,048,306). It is critical to note that the oxazine derivatives were inert to standard reducing agents under the general conditions used by all these workers. Thus, until the present invention, alkyl derivatives derived from erythromycylamine and aliphatic aldehydes generally, were not obtainable by previously known methodology.

Our invention now provides a general solution to the preparation of alkyl derivatives of erythromycylamine and aliphatic aldehydes. The key to this solution involves controlling the pH of the reaction, using either a pre-formed oxazine derivative which has been independently isolated or a reaction in which an oxazine may or may not be pre-formed in situ but is not separated. Thus, when either a pre-formed oxazine or an erythromycylamine-aldehyde mixture is maintained at a pH in the range of from about 4 to

about 6 in the presence of reducing agents such as sodium borohydride or sodium cyanoborohydride, the corresponding N-alkyl derivative can be readily obtained, using any aliphatic aldehyde.

Alternatively, we have also discovered that catalytic hydrogenation of the oxazine or of the erythromycylamine-aldehyde adduct can be used to accomplish the same chemical transformation. With these new procedures, a number of erythromycylamine derivatives which were previously unobtainable have now been prepared. The new group of derivatives made possible by these procedures is shown in formula 1.

In carrying out the first process of this invention, the key point is controlling the pH so that the intermediate Schiff's base between erythromycylamine and the aliphatic aldehyde is present to some degree. The Schiff's base can then be reduced by reducing agents to the corresponding alkyl derivative. The reaction conditions thus promote or establish the presence of some Schiff's base in the equilibrium between the oxazine, the Schiff's base and dissociated starting materials. The pH control is most conveniently performed in the presence of the reducing agent so that any Schiff's base which is formed can be rapidly reduced to the N-alkyl derivative. The equilibrium is thus constantly being displaced to give the desired product.

Controlling the pH is accomplished by standard methods, i.e., additions of acids, bases or buffer solutions to adjust the reaction mixture to the desired pH, using standard methodology such as a pH meter or pH indicator paper. Reaction progress can be followed by standard methods such as thin-layer chromatography (TLC) or high performance liquid chromatography (HPLC) until the reduction is complete. The product can be isolated by standard methods such as extraction, precipitation, crystallization, and/or chromatographic procedures.

The second process of this invention involves hydrogenation over standard catalysts, such as palladium, platinum and nickel. The catalyst is typically presented on a carrier such as charcoal. The hydrogenation is conveniently carried out in the presence of a solvent or mixture of solvents in which the reactants are soluble. Moderate pressure ranges, e.g. 1 to 50 atmospheres, are suitable for the reaction. The pressure used will vary, depending upon other factors such as the catalyst used. Although different temperatures may be used in the reaction, it has been found that the reductive alkylation is more selective when hydrogenation is performed at higher temperatures, e.g. from about 80° to about 150°C.

Thus, in accordance with the invention, there is provided a process for preparing a compound of formula (I):

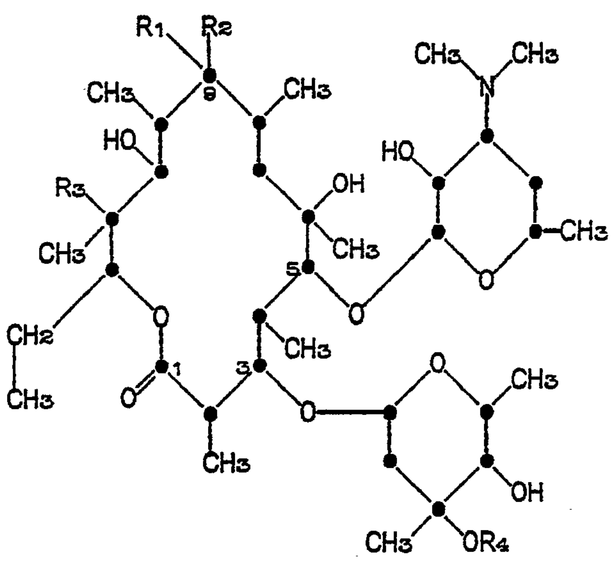

1

wherein $R_1$ and $R_2$ are different and are hydrogen or $-NHCH_2R_5$;

$R_3$ is hydrogen or hydroxyl;

$R_4$ is hydrogen or methyl; and

$R_5$ is hydrogen or a $C_1$-$C_{14}$-alkyl or $-(CH_2)_lX(CH_2)_mY$ group, either of which group may have from one to three substituents selected from halo, hydroxyl, $C_1$-$C_4$-alkyl, $C_1$-$C_4$-alkoxy, $C_1$-$C_4$-alkylthio, cyano, $C_1$-$C_4$-alkoxycarbonyl, mono- or di($C_1$-$C_4$-alkyl)amino, $-N(CH_2)_s$, $R_6$-substituted-phenyl, or an $R_6$-substituted-monocyclic heterocyclic group having from 3 to 7 ring atoms;

X is oxygen or sulfur;

Y is $-X(CH_2)_nCH_3$, $-N(CH_2)_s$ or $-N[(CH_2)_nCH_3]_2$;

l is 1 or 2;

m is an integer from 1 to 3;
n is an integer from 0 to 3;
s is an integer from 2 to 7; and
$R_6$ is hydrogen, halo, $C_1$-$C_4$-alkyl or $C_1$-$C_4$-alkoxy;
provided that, when the substituent on the $R_5$' group is selected from hydroxyl, cyano, alkoxycarbonyl, mono- or dialkylamino or -$N(CH_2)_s$, it cannot be located on the second or third carbon atom from the nitrogen of the -$NHCH_2R_5$ group unless the second carbon atom is quaternary;
or a salt thereof, which comprises reducing, by catalytic hydrogenation at a temperature of from about 80°C to about 150°C or chemical reduction at a pH of about 4 to about 6, a compound of Formula (1a):

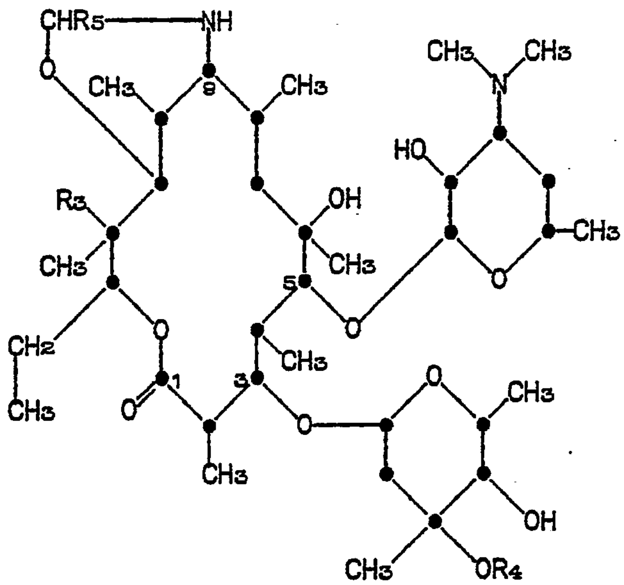

**1a**

and, if desired, salifying the product.

Furthermore, we have discovered that it is possible to react monoalkyl derivatives of erythromycylamine with formaldehyde to make the methylene bridged compounds of formula 2. These compounds represent the first examples of 9-N-disubstituted derivatives of erythromycylamine. Aldehydes other than formaldehyde react poorly, if at all, with monoalkyl derivatives of erythromycylamine.

In this regard, therefore, there is provided a process for preparing a compound of Formula (2):

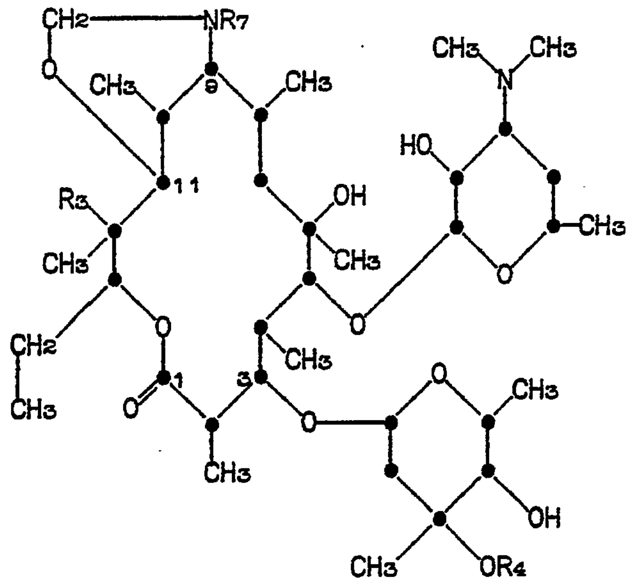

2

wherein $R_3$ is hydrogen or hydroxyl and $R_4$ is hydrogen or methyl;

$R_7$ is

(a) $-CH_2R_5$ in which $R_5$ is hydrogen or a $C_1$-$C_{14}$-alkyl or $-(CH_2)_lX(CH_2)_mY$ group, either of which group may have from one to three substituents selected from halo, hydroxyl, $C_1$-$C_4$-alkyl, $C_1$-$C_4$-alkoxy, $C_1$-$C_4$-alkylthio, cyano, $C_1$-$C_4$-alkoxycarbonyl, mono- or di($C_1$-$C_4$-alkyl)amino, $-N(CH_2)_s$, $R_6$-substituted-phenyl, or an $R_6$-substituted-monocyclic heterocyclic group having from 3 to 7 ring atoms;

X is oxygen or sulfur;

Y is $-X(CH_2)_nCH_3$, $-N(CH_2)_s$ or $-N[(CH_2)_nCH_3]_2$;

l is 1 or 2;

m in an integer from 1 to 3;

n is an integer from 0 to 3;

s is an integer from 2 to 7; and

$R_6$ is hydrogen, halo, $C_1$-$C_4$-alkyl or $C_1$-$C_4$-alkoxy; or

(b) $C_3$-$C_8$-cycloalkyl, $-CHR_8(CH_2)_pR_9$, $-(CH_2)_qR_{10}$ or $-CH_2(CH=CH)_rAr$;

$R_8$ is $C_1$-$C_4$-alkyl, phenyl or benzyl;

$R_9$ is hydrogen, halo, hydroxy, $C_1$-$C_4$-alkoxy, mono- or di($C_1$-$C_4$-alkyl)amino, $-N(CH_2)_s$ or phenyl

$R_{10}$ is hydroxy, cyano, $C_1$-$C_4$-alkoxycarbonyl, mono- or di($C_1$-$C_4$-alkyl)amino or $-N(CH_2)_s$;

Ar is phenyl; phenyl having one or more halo, $C_1$-$C_4$-alkyl, $C_1$-$C_4$-alkoxy or hydroxy substituents; or an $R_6$-substituted monocyclic aromatic heterocyclic group having from 5 to 7 ring atoms;

p is an integer from 1 to 5;

q is 2 or 3;

r is 0 or 1; and

s is as defined above in (a);

or a salt thereof, which comprises:

reacting a compound of Formula (2a):

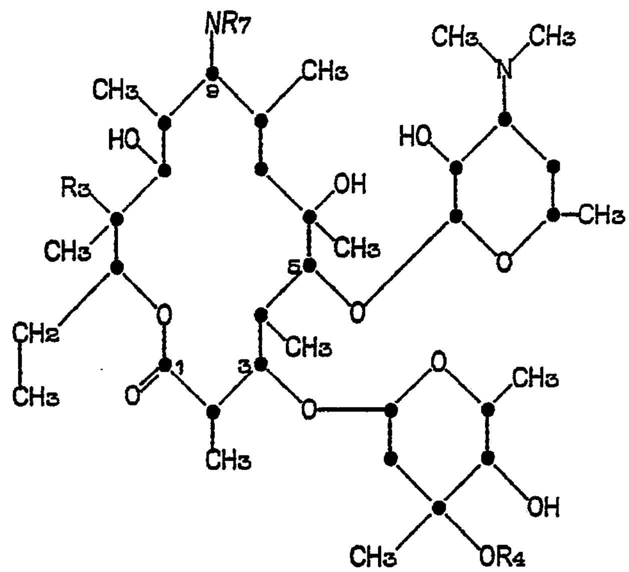

**(2a)**

in which $R_3$, $R_4$ and $R_7$ are as defined above, with formaldehyde.

We have further discovered that the bridged methylene compounds of formula 2 can be opened via reductive techniques, such as those described supra, to make tertiary amines, thus providing a method for obtaining the previously unknown tertiary amine derivatives of erythromycylamine shown in formula 3.

Finally, we have discovered that it is possible to cyclize erythromycylamine with dialdehydes to make cyclic tertiary amino derivatives, i.e., the formula 3 compounds wherein $R_{11}$ is $-N(CH_2)_s$.

Further, therefore, there is provided a process for preparing a compound of Formula (3):

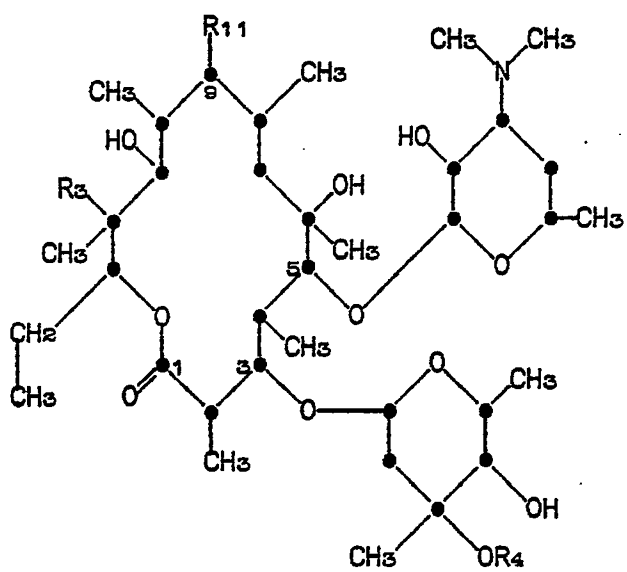

**3**

in which $R_3$ is hydrogen or hydroxyl;
$R_4$ is hydrogen or methyl;
$R_{11}$ is $-N(CH_2)_s$ in which s in an integer from 2 to 7 or $-N(CH_3)R_7$ in which $R_7$ is:

(a) $-CH_2R_5$ in which $R_5$ is hydrogen or a $C_1$-$C_4$-alkyl or $-(CH_2)_lX(CH_2)_mY$ group, either of which group may have from one to three substituents selected from halo, hydroxyl, $C_1$-$C_4$-alkyl, $C_1$-$C_4$-alkoxy, $C_1$-$C_4$-alkylthio, cyano, $C_1$-$C_4$-alkoxycarbonyl, mono- or di($C_1$-$C_4$-alkyl)amino, $-N(CH_2)_s$, $R_6$-substituted-phenyl, or an $R_6$-substituted-monocyclic heterocyclic group having from 3 to 7 ring atoms;
X is oxygen or sulfur;
Y is $-X(CH_2)_nCH_3$, $-N(CH_2)_s$ or $-N[(CH_2)_nCH_3]_2$;
l is 1 or 2;
m is an integer from 1 to 3;
n is an integer from 0 to 3;
s is an integer from 2 to 7; and
$R_6$ is hydrogen, halo, $C_1$-$C_4$-alkyl or $C_1$-$C_4$-alkoxy; or
(b) $C_3$-$C_8$-cycloalkyl, $-CHR_8(CH_2)_pR_9$, $-(CH_2)_qR_{10}$ or $-CH_2(CH{=}CH)_rAr$;
$R_8$ is $C_1$-$C_4$-alkyl, phenyl, or benzyl;
$R_9$ is hydrogen, halo, hydroxy, $C_1$-$C_4$-alkoxy, mono- or di($C_1$-$C_4$-alkyl)amino, $-N(CH_2)_s$ or phenyl
$R_{10}$ is hydroxy, cyano, $C_1$-$C_4$-alkoxycarbonyl, mono- or di($C_1$-$C_4$-alkyl)amino or $-N(CH_2)_s$;
Ar is phenyl; phenyl having one or more halo, $C_1$-$C_4$-alkyl, $C_1$-$C_4$-alkoxy or hydroxy substituents; or an $R_6$-substituted monocyclic aromatic heterocyclic group having from 5 to 7 ring atoms;
p is an integer from 1 to 5;
q is 2 or 3;
r is 0 to 1; and
s is as defined above, or a salt thereof, which comprises:
(a) reducing, by catalytic hydrogenation at a temperature of from about 80°C to about 150°C or chemical reduction at a pH of about 4 to about 6, a compound of Formula (2) as defined above, to produce a compound of Formula (3) in which $R_{11}$ is a $-N(CH_3)R_7$ and, if desired, salifying the product; or
(b) reacting an erythromycylamine with a dialdehyde of the formula $OHC-(CH_2)_b-CHO$ where b is an integer from 1 to 5, followed by reducing the product, by catalytic hydrogenation at a temperature of from about 80°C to about 150°C or chemical reduction at a pH of about 4 to about 6;
and if desired, salifying the product.

The derivatives of this invention form salts, particularly acid addition salts. These acids addition salts are also useful as antibiotics and are a part of this invention. In another aspect, such salts are useful as intermediates, for example, for separating and purifying the derivatives. In addition, the salts have improved solubility in water.

Representative suitable salts include those salts formed by standard reactions with both organic and inorganic acids such as, for example, sulfuric, hydrochloric, phosphoric, acetic, succinic, citric, lactic, maleic, fumaric, palmitic, cholic, pamoic, mucic, D-glutamic, d-camphoric, glutaric, glycolic, phthalic, tartaric, formic, lauric, stearic, salicyclic, methanesulfonic, benzenesulfonic, sorbic, picric, benzoic, cinnamic, and like acids.

Pharmaceutically acceptable acid addition salts are an especially preferred group of salts of this invention. Pharmaceutically acceptable acid addition salts are those salts useful in the chemotherapy of a warm-blooded animal.

Typical formula 1compounds are shown in Table I.

Table I: Illustrative Formula 1 Compounds

| Compound No. | $R_1$ | $R_2$ | $R_3$ | $R_4$ |
|---|---|---|---|---|
| 1 | H | –NHMe | OH | Me |
| 2 | H | –NHEt | OH | Me |
| 3 | H | –NHPr | OH | Me |
| 4 | H | –NH(n-Bu) | OH | Me |
| 5 | H | $-NH(CH_2)_4Me$ | OH | Me |
| 6 | H | $-NH(CH_2)_5Me$ | OH | Me |
| 7 | H | $-NH(CH_2)_6Me$ | OH | Me |
| 8 | H | $-NH(CH_2)_7Me$ | OH | Me |
| 9 | H | $-NH(CH_2)_9Me$ | OH | Me |
| 10 | H | $-NH(CH_2)_{11}Me$ | OH | Me |
| 11 | H | $-NH(CH_2)_2CH(Me)_2$ | OH | Me |
| 12 | H | $-NHCH_2CH(Et)_2$ | OH | Me |
| 13 | H | –NH(cis-dec-4-enyl) | OH | Me |
| 14 | H | –NH(trans-dec-4-enyl) | OH | Me |
| 15 | H | $-NHCH_2$(cyclohex-3-enyl) | OH | Me |
| 16 | H | $-NHCH_2$(cyclooctyl) | OH | Me |
| 17 | H | $-NH(CH_2)_3Ph$ | OH | Me |
| 18 | H | $-NHCH_2C{\equiv}CPh$ | OH | Me |
| 19 | H | $-NHCH_2$(endo-5-norbornen-2-yl) | OH | Me |
| 20 | H | $-NHCH_2$(exo-5-norbornen-2-yl) | OH | Me |
| 21 | H | $-NH(CH_2)_5OH$ | OH | Me |
| 22 | H | $-NHCH_2C(Me)_2CH_2OH$ | OH | Me |
| 23 | H | $-NH(CH_2)_3OMe$ | OH | Me |
| 24 | H | –NH(3,7-diMe-7-MeO-octyl) | OH | Me |
| 25 | H | $-NH(CH_2)_2O(CH_2)_2OMe$ | OH | Me |
| 26 | H | $-NH(CH_2)_3SMe$ | OH | Me |
| 27 | H | $-NHCH_2$(2-COOEt-cycloprop-1-yl) | OH | Me |
| 28 | H | –NH(undec-10-en-1-yl) | OH | Me |
| 29 | H | $-NH(CH_2)_3CN$ | OH | Me |
| 30 | H | $-NH(CH_2)_2$(2,6,6-triMe-cyclohex-1-en-1-yl) | OH | Me |
| 31 | H | $-NH(CH_2)_2OCH_3$ | OH | Me |
| 32 | H | $-NH(CH_2)_2O(CH_2)_3OEt$ | OH | Me |
| 33 | H | $-NH(CH_2)_3O(CH_2)_2OMe$ | OH | Me |
| 34 | H | $-NH(CH_2)_2O(CH_2)_2NMe_2$ | OH | Me |
| 35 | H | $-NH(CH_2)_3O(CH_2)_2N(CH_2)_6$ | OH | Me |
| 36 | H | $-NH(CH_2)_2S(CH_2)_2OMe$ | OH | Me |
| 37 | H | –NHEt | H | Me |
| 38 | H | –NHPr | H | Me |
| 39 | H | $-NH(CH_2)_5Me$ | H | Me |
| 40 | H | $-NH(CH_2)_2CH(Me)_2$ | H | Me |
| 41 | H | $-NH(CH_2)_9CH_3$ | H | Me |
| 42 | H | –NH(cis-dec-4-enyl) | H | Me |
| 43 | H | $-NHCH_2$(cyclohex-3-enyl) | H | Me |
| 44 | H | $-NH(CH_2)_3Ph$ | H | Me |
| 45 | H | $-NHCH_2C{\equiv}CPh$ | H | Me |
| 46 | H | $-NHCH_2$(pyridin-2-yl) | H | Me |
| 47 | H | $-NHCH_2$(furan-2-yl) | H | Me |
| 48 | H | $-NHCH_2$(thien-3-yl) | H | Me |

Table I: Illustrative Formula 1 Compounds (continued)

| Compound No. | $R_1$ | $R_2$ | $R_3$ | $R_4$ |
|---|---|---|---|---|
| 49 | H | $-NH(CH_2)_2O(CH_2)_2OMe$ | H | Me |
| 50 | H | $-NH(CH_2)_5$(morpholin-4-yl) | H | Me |
| 51 | H | $-NHCH_2$(cyclopropyl) | H | Me |
| 52 | H | $-NH(CH_2)_3OMe$ | H | Me |
| 53 | –NHPr | H | OH | Me |
| 54 | –NHPr | H | H | Me |
| 55 | H | –NHPr | OH | H |
| 56 | H | –NHPr | H | H |
| 57 | H | $-NH(CH_2)_2O(CH_2)_2OMe$ | OH | H |

Typical formula 2 compounds are shown in Table II.

Table II: Illustrative Formula 2 Compounds

| Compound Number | $R_3$ | $R_4$ | $R_7$ |
|---|---|---|---|
| 58 | OH | $CH_3$ | iPr |
| 59 | OH | $CH_3$ | Pr |
| 60 | OH | $CH_3$ | cyclopentyl |
| 61 | OH | $CH_3$ | cyclohexyl |
| 62 | OH | $CH_3$ | benzyl |
| 63 | OH | $CH_3$ | 5-dimethylamino-2-pentyl |
| 64 | H | $CH_3$ | Pr |
| 65 | H | $CH_3$ | cyclohexyl |
| 66 | H | $CH_3$ | $-(CH2)_3OMe$ |
| 67 | OH | H | cyclohexyl |
| 68 | H | H | Et |

Typical formula 3 compounds are shown in Table III.

Table III: Illustrative Formula 3 Compounds

| Compound Number | $R_3$ | $R_4$ | $R_{10}$ |
|---|---|---|---|
| 69 | OH | $CH_3$ | $-N(Me)_2$ |
| 70 | OH | $CH_3$ | –N(Me)(iPr) |
| 71 | OH | $CH_3$ | –N(cyclohexyl)(Me) |
| 72 | OH | $CH_3$ | $-N(CH2)_5$ |
| 73 | OH | $CH_3$ | $-N(CH2)_2$ |
| 74 | H | $CH_3$ | –N(Me)(cyclopropyl) |
| 75 | H | $CH_3$ | $-N(CH2)_4$ |
| 76 | H | $CH_3$ | –N(Et)(Me) |
| 77 | OH | H | $-N(Me)_2$ |
| 78 | H | H | $-N(Me)_2$ |

The new derivatives of this invention inhibit the growth of a broad spectrum of pathogenic bacteria, especially Gram-positive bacteria and Gram-negative cocci such as Haemophilus influenzae. Tables IV

and V summarize the minimal inhibitory concentrations (MIC's) at which these compounds inhibit certain organisms, as determined by standard agar-dilution assays.

Table IV: Antibiotic Activity of Formula 1 Derivatives[a]

| Organism | Compound Number[b] | | | | | | | | |
|---|---|---|---|---|---|---|---|---|---|
| | 1 | 2 | 3 | 4 | 5 | 6 | 7 | 8 | 9 |
| Staphylococcus aureus X1.1 | 1 | 1 | 1 | 0.25 | 0.5 | 1 | 0.25 | 0.5 | 1 |
| Staphylococcus aureus V41[c] | 64 | –[h] | – | – | – | – | – | 8 | 4 |
| Staphylococcus aureus X400[d] | 64 | – | – | – | – | – | – | 8 | 8 |
| Staphylococcus aureus S13E | 0.125 | 2 | 0.25 | 1 | 1 | 1 | NT | 0.25 | 0.5 |
| Staphylococcus epidermidis 270 | – | – | – | – | – | – | – | NT | NT |
| Staphylococcus epidermidis 222 | 0.25 | 1 | 0.5 | 0.25 | 0.5 | 0.25 | 0.25 | 0.25 | 1 |
| Streptococcus pyogenes C203 | 0.06 | 0.125 | 0.03 | 0.06 | 0.06 | 0.125 | 0.015 | 0.015 | 0.015 |
| Streptococcus pneumoniae Park I | NT[g] | 0.03 | 0.015 | 0.015 | 0.015 | <0.008 | 0.015 | 0.015 | 0.015 |
| Streptococcus sp. group D X66 | 0.125 | 0.25 | 0.125 | 0.125 | 0.125 | 0.125 | 0.06 | 0.06 | 0.25 |
| Streptococcus sp. group D 2041 | 1 | 4 | 4 | 1 | 2 | 4 | 2 | 1 | 2 |
| Haemophilus influenzae C.L.[e] | 0.5 | 1 | 1 | 1 | 1 | 4 | 1 | 1 | 2 |
| Haemophilus influenzae 76[f] | 1 | 4 | 2 | 1 | 2 | 4 | 2 | 1 | 1 |
| Escherichia coli EC14 | 32 | 32 | 32 | 64 | 128 | 64 | 64 | – | – |
| Klebsiella pneumoniae X68 | 64 | 64 | 128 | 128 | 128 | NT | – | – | – |

Table IV: Antibiotic Activity of Formula 1 Derivatives[a] (continued)

| Organism | Compound Number[b] | | | | | | | | |
|---|---|---|---|---|---|---|---|---|---|
| | 10 | 11 | 12 | 13 | 14 | 15 | 16 | 17 | 18 |
| Staphylococcus aureus X1.1 | 4 | 1 | 0.5 | 0.25 | 0.5 | 1 | 0.5 | 1 | 1 |
| Staphylococcus aureus V41[c] | 8 | – | 16 | 8 | 8 | – | 64 | – | 16 |
| Staphylococcus aureus X400[d] | 8 | – | 16 | 8 | 16 | – | 64 | – | 32 |
| Staphylococcus aureus S13E | 2 | 0.5 | 0.25 | 0.25 | 0.25 | 1 | 0.06 | 0.5 | 0.5 |
| Staphylococcus epidermidis 270 | NT | – | – | 32 | 32 | – | – | – | 64 |
| Staphylococcus epidermidis 222 | 4 | 0.25 | 0.5 | 0.125 | 0.25 | 0.25 | 0.25 | 0.5 | 0.5 |
| Streptococcus pyogenes C203 | 0.5 | 0.06 | 0.015 | <0.008 | <0.008 | 0.06 | <0.008 | 0.015 | <0.008 |
| Streptococcus pneumoniae Park I | 0.06 | 0.03 | 0.015 | <0.008 | <0.008 | <0.008 | NT | 0.015 | <0.008 |
| Streptococcus sp. group D X66 | 1 | 0.125 | 0.125 | 0.06 | 0.125 | 0.25 | <0.008 | 0.06 | 0.25 |
| Streptococcus sp. group D 2041 | 8 | 1 | 8 | 1 | 2 | 8 | 1 | 2 | 1 |
| Haemophilus influenzae C.L.[e] | 8 | 1 | 2 | 2 | 2 | 4 | 1 | 2 | 4 |
| Haemophilus influenzae 76[f] | 4 | 1 | 2 | NT | NT | 2 | 1 | 2 | NT |
| Escherichia coli EC14 | – | 128 | 64 | 128 | 128 | 64 | 64 | 128 | – |
| Klebsiella pneumoniae X68 | – | 128 | 32 | – | – | NT | – | – | – |

Table IV: Antibiotic Activity of Formula 1 Derivatives[a] (continued)

| Organism | Compound Number[b] | | | | | | | |
|---|---|---|---|---|---|---|---|---|
| | 19 | 20 | 21 | 22 | 23 | 24 | 25 | 26 |
| Staphylococcus aureus X1.1 | 2 | 2 | 0.5 | 1 | 2 | 0.5 | 1 | 0.5 |
| Staphylococcus aureus V41[c] | – | – | – | – | – | 64 | – | – |
| Staphylococcus aureus X400[d] | – | – | – | – | – | 64 | – | – |
| Staphylococcus aureus S13E | 2 | 2 | 0.25 | 1 | 2 | 0.25 | 8 | 0.25 |
| Staphylococcus epidermidis 270 | – | – | – | – | – | – | – | – |
| Staphylococcus epidermidis 222 | 1 | 1 | 0.5 | 1 | 0.5 | 0.25 | 1 | 0.5 |
| Streptococcus pyogenes C203 | 0.25 | 0.25 | <0.008 | 0.03 | 0.125 | 0.015 | 0.125 | 0.015 |
| Streptococcus pneumoniae Park I | 0.015 | 0.015 | <0.008 | 0.015 | 0.03 | NT | 0.015 | 0.015 |
| Streptococcus sp. group D X66 | 1 | 1 | 0.25 | 0.25 | 0.25 | 0.03 | 0.25 | 0.25 |
| Streptococcus sp. group D 2041 | 8 | 8 | 4 | 4 | 8 | 2 | 8 | 2 |
| Haemophilus influenzae C.L.[e] | 8 | 8 | 2 | 1 | 4 | 1 | 2 | 4 |
| Haemophilus influenzae 76[f] | 8 | 8 | NT | 0.25 | 4 | 2 | 4 | 4 |
| Escherichia coli EC14 | 64 | 64 | 32 | 32 | 64 | 128 | 64 | 128 |
| Klebsiella pneumoniae X68 | – | – | 128 | 64 | NT | – | 128 | – |

Table IV: Antibiotic Activity of Formula 1 Derivatives[a] (continued)

| Organism | Compound Number[b] | | | | | | | |
|---|---|---|---|---|---|---|---|---|
| | 27 | 28 | 29 | 30 | 31 | 38 | 41 | 49 |
| Staphylococcus aureus X1.1 | 1 | 2 | 1 | 1 | 0.5 | 0.5 | 2 | 1 |
| Staphylococcus aureus V41[c] | – | 8 | – | 16 | – | 8 | 4 | 128 |
| Staphylococcus aureus X400[d] | – | 8 | – | 32 | – | 64 | 4 | – |
| Staphylococcus aureus S13E | 1 | 2 | 128 | 2 | 0.5 | 1 | 1 | 0.5 |
| Staphylococcus epidermidis 270 | – | 32 | – | 64 | – | – | 8 | – |
| Staphylococcus epidermidis 222 | 0.5 | 1 | 1 | 1 | 0.25 | 0.5 | 0.5 | 1 |
| Streptococcus pyogenes C203 | 0.5 | 1 | 1 | 0.5 | 0.06 | 0.125 | 0.03 | 0.015 |
| Streptococcus pneumoniae Park I | NT | NT | NT | NT | 0.03 | 0.125 | 0.03 | 0.015 |
| Streptococcus sp. group D X66 | 0.125 | 0.125 | 0.125 | 0.125 | 0.125 | 0.125 | 0.5 | 0.25 |
| Streptococcus sp. group D 2041 | 2 | 2 | 4 | 2 | 8 | 2 | 4 | 4 |
| Haemophilus influenzae C.L.[e] | 2 | 2 | 2 | 4 | 0.5 | 2 | 8 | 4 |
| Haemophilus influenzae 76[f] | 2 | 2 | 4 | 4 | 2 | 4 | 4 | 8 |
| Escherichia coli EC14 | 128 | – | 64 | – | 16 | 64 | – | 128 |
| Klebsiella pneumoniae X68 | 128 | – | 128 | – | 32 | 128 | – | 128 |

[a]MIC's in mcg/mL
[b]Compound numbers from Table I
[c]Penicillin-resistant strain
[d]Methicillin-resistant strain
[e]Ampicillin-sensitive strain
[f]Ampicillin-resistant strain
[g]NT = not tested
[h]– = >128

Table V: Antibiotic Activity of Formula 2 and 3 Derivatives[a]

| Organism | Compound Number[b] | | | | | | | | |
|---|---|---|---|---|---|---|---|---|---|
| | 58 | 59 | 60 | 62 | 63 | 69 | 70 | 71 | 72 |
| Staphylococcus aureus X1.1 | 1 | 0.25 | 1 | 1 | 8 | 4 | 8 | 16 | 16 |
| Staphylococcus aureus V41[c] | –[h] | 8 | – | 64 | – | 64 | – | – | – |
| Staphylococcus aureus X400[d] | – | 8 | – | 128 | – | 64 | – | – | – |
| Staphylococcus aureus S13E | 1 | 0.125 | 2 | 1 | 8 | 2 | 8 | 16 | 16 |
| Staphylococcus epidermidis EPI1 | 128 | – | – | 64 | – | – | – | – | – |
| Staphylococcus epidermidis 222 | 0.5 | 0.125 | 1 | 0.5 | 8 | 2 | 8 | 8 | 8 |
| Streptococcus pyogenes C203 | 0.125 | <0.008 | 0.125 | 0.125 | 0.5 | 0.25 | 0.5 | 1 | 1 |
| Streptococcus pneumoniae Park I | 0.06 | <0.008 | 0.06 | 0.06 | 0.5 | NT[g] | 0.5 | 0.125 | 1 |
| Streptococcus sp. group D X66 | 0.25 | 0.125 | 0.5 | 0.5 | 2 | 0.5 | 2 | 8 | 4 |
| Streptococcus sp. group D 2041 | 4 | 1 | 8 | 4 | 32 | 4 | 32 | 128 | 128 |
| Haemophilus influenzae C.L.[e] | 4 | 1 | 4 | 4 | 8 | 2 | 32 | 32 | 2 |
| Haemophilus influenzae 76[f] | 4 | 2 | 4 | 4 | 8 | 8 | 32 | 32 | 64 |
| Escherichia coli EC14 | 32 | 32 | 64 | – | 32 | 128 | – | – | – |
| Klebsiella pneumoniae X68 | 64 | 128 | 64 | – | 128 | – | – | – | – |

[a]MIC's in mcg/mL
[b]Compound numbers from Tables II–III
[c]Penicillin-resistant strain
[d]Methicillin-resistant strain
[e]Ampicillin-sensitive strain
[f]Ampicillin-resistant strain
[g]NT = not tested
[h]– = >128

In addition to excellent in vitro activity against erythromycin-sensitive bacteria, several members of this new series have shown in vitro activity against erythromycin-resistant strains. These include strains which are inducibly-resistant to erythromycin (S.. aureus V41 and X400 and S. epidermidis EPI1) and even strains which are constitutively-resistant (S. epidermidis 270).

The new derivatives of this invention have also shown in vivo antimicrobial activity against experimental bacterial infections. When two doses of test compound were administered to mice in experimental infections, the activity observed was measured as an $ED_{50}$ value [effective dose in mg/kg to protect 50% of the test animals: see Warren Wick, et al., J. Bacteriol. 81, 233-235 (1961)]. $ED_{50}$ values observed for some of the derivatives are given in Tables VI and VII.

Table VI: Subcutaneous $ED_{50}$ Values for Erythromycylamine Derivatives against Experimental Infections Induced by Gram-positive Bacteria

| Compound Numbers[a] | Infecting Organism Staphylococcus aureus | Streptococcus pyogenes | Streptococcus pneumoniae |
|---|---|---|---|
| 3 | 6.11 | 1.67 | 1.77 |
| 23 | 3.73 | 2.1 | 8.3 |
| 31 | 2.50 | 2.1 | 3.3 |
| 58 | >10, 4.5[b] | NT[c] | NT |
| 60 | 5.0 | NT | NT |

[a]Compound numbers from Tables I and II
[b]Results of two tests
[c]Not tested

Table VII: Oral $ED_{50}$ Values for Erythromycylamine Derivatives against Experimental Infections Induced by Gram-positive Bacteria

| Compound Number[a] | Infecting Organism Staphylococcus aureus | Streptococcus pyogenes | Streptococcus pneumoniae |
|---|---|---|---|
| 1 | 43.8 | 21.0 | 35.4 |
| 2 | 23.4 | 12.5 | 13.4 |
| 3 | 11.8, 12.6[b] | 3.3, 6.1 | 7.2, 6.3 |
| 4 | 14.7 | 12.5 | 12.5 |
| 5 | 25 | 8.8 | 13.6 |
| 6 | 38.6 | 17.7 | 14.3 |
| 7 | >50 | 30.6 | 33.2 |
| 8 | >50, >50 | 23.4, 38.3 | 46.1, >50 |
| 9 | >50 | >50 | >50 |
| 10 | >45 | >50 | >50 |
| 11 | 12.5 | 8.8 | 10.8 |
| 12 | 22.3 | 9.4 | 16.6 |
| 15 | 20.0 | 14.9 | 8.7 |
| 17 | 36.9 | 14.1 | 17.2 |
| 18 | >50 | 26.7 | 33.9 |
| 19 | >50 | 16.6 | 25.0 |
| 20 | >50 | 16.6 | 25.0 |
| 21 | >50 | 42.6 | >50 |
| 22 | >50 | 19.8 | 46.1 |
| 23 | 16.4 | 13.5 | 15.9 |
| 24 | >45.6 | 25.0 | 23.6 |
| 25 | 23.3 | 27.1 | 21.8 |
| 26 | 11.7, 50 | 7.9 | 9.2 |
| 27 | 50 | 27.1 | 30.4 |
| 29 | 36.1 | 21.1 | 28.7 |
| 30 | >50 | >50 | >50 |
| 31 | 35.2 | 16.34 | 18.7 |
| 38 | 41.0 | 20.6 | 50 |
| 41 | >50 | >50 | >50 |
| 49 | >50 | 40.9 | >56 |
| 58 | 9.4, 9.1, 4.0[c] | 6.7 | 5.7 |
| 59 | 7.2 | 4.8 | 5.0 |
| 60 | >50, 15.7, 38.6 | 10.2 | 9.0 |
| 62 | >50, >50, 46 | 15.6 | 15.2 |
| 69 | >43.8 | 21.9 | 43.5 |
| 71 | >50 | >50 | >50 |
| 72 | >44 | 23.6 | 20.6 |

[a]Compound Numbers from Tables I-III
[b]Results of two tests
[c]Results of three tests

Pharmaceutical formulations of the compounds of formulas 1, 2 and 3 or their salts are also provided by this invention. The compounds, preferably as a pharmaceutically acceptable salt, can be formulated for oral or parenteral administration for the therapeutic or prophylactic treatment of bacterial infections. For example, a formulation provided may contain, as an active ingredient, a compound of formula 1,

2, or 3 associated with one or more pharmaceutically acceptable carriers, excipients or diluents and may be used in the form of parenteral solutions, tablets, capsules, elixirs, suspensions, syrups, wafers and the like. The compositions comprising a compound of this invention will contain from about 0.1 to about 90% by weight of the active compound, and more generally from about 10 to about 30%. The compositions may contain common carriers and excipients, such as corn starch or gelatin, lactose, sucrose, microcrystalline cellulose, kaolin, mannitol, dicalcium phosphate, sodium chloride and alginic acid. Disintegrators commonly used in the formulations of this invention include croscarmellose sodium, microcrystalline cellulose, corn starch, sodium starch glycolate and alginic acid. Tablet binders that can be included are acacia, methylcellulose, sodium carboxymethylcellulose, polyvinylpyrrolidone (Povidone), hydroxypropyl methylcellulose, sucrose, starch and ethylcellulose. Lubricants that can be used include magnesium stearate or other metallic stearates, stearic acid, silicone fluid, talc, waxes, oils and colloidal silica. Flavoring agents such as peppermint, oil of wintergreen, cherry flavoring or the like can also be used. It may be desirable to add a coloring agent to make the dosage form more esthetic in appearance or to help identify the product.

For intravenous (IV) use, a water soluble form of the compound can be dissolved in one of the commonly used intravenous fluids and administered by infusion. Such fluids as, for example, physiological saline, Ringer's solution or 5% dextrose solution can be used.

For intramuscular preparations, a sterile formulation of a suitable soluble salt form of the compound, for example the hydrochloride salt, can be dissolved and administered in a pharmaceutical diluent such as Water-for-Injection, physiological saline or 5% glucose. A suitable insoluble form of the compound may be prepared and administered as a suspension in an aqueous base or a pharmaceutically acceptable oil base, e.g. an ester of a long chain fatty acid such as ethyl oleate.

For oral use, solid formulations such as tablets and capsules are particularly useful. Sustained release or enterically coated preparations may also be devised. For pediatric and geriatric applications, suspensions, syrups and chewable tablets are especially suitable.

Alternatively, the unit dosage from of the antibiotic can be a solution of the compound or preferably a salt thereof in a suitable diluent in sterile, hermetically sealed ampoules. The concentration of the antibiotic in the unit dosage may vary, e.g. from about 1 percent to about 50 percent, depending on the compound used and its solubility and the dose desired by the physician.

In a further aspect, this invention provides a method for treating, controlling, or preventing infectious diseases, especially those caused by Gram-positive microorganisms, in animals. This method comprises administering to a warm-blooded animal an effective dose of a compound provided by this invention. An effective dose is generally between about 0.1 and about 100 mg/kg of the compound or its pharmaceutically acceptable salt. A preferred dose is from about 1 to about 30 mg/kg of compound. A typical daily dose for an adult human is from about 100 mg to about 1.0 g.

In practicing this method, the antibiotic compound can be administered as a single daily dose or in multiple doses per day. The treatment regime may require administration over extended periods of time, e.g., for several days or for from two to four weeks. The amount per administered dose or the total amount administered will depend on such factors as the nature and severity of the infection, the age and general health of the patient, the tolerance of the patient to the antibiotic and the microorganism or microorganisms involved in the infection.

A convenient method of practicing the treatment method is to administer the antibiotic orally, using tablets, capsules, suspensions, syrups and the like. The antibiotic may also be administered by other methods, e.g. as a suppository or parenterally via IV infusion.

The following non-limiting examples are provided in order to further illustrate this invention. Unless otherwise indicated, reactions were monitored by thin-layer chromatography (TLC) on silica gel (E. Merck plates), using a $CH_2Cl_2$/MeOH/$NH_4OH$ solvent system in a ratio such as 90:10:2, 90:10:1 or 90:10:0:5 and detecting with $I_2$ or anisaldehyde. Comparable systems, such as replacing $CH_2Cl_2$ with $CHCl_3$ or MeOH with EtOH, may also be used. Erythromycylamine was prepared by the method of Wildsmith (Tetrahedron Letters 1972, 29), and N-alkylerythromycylamines were prepared by the method of Wildsmith, et al. [J. Med. Chem. 16 1059 (1973)].

Example 1

9-Deoxo-9-[isopropylamino]-9,11-(N,O)-cyclic Methylene Adduct of Erythromycin (Compound 58)

N-isopropylerythromycylamine (2.7 g, 3.5 mmoles) was dissolved with warming in $CH_3CN$ (25 mL). Aqueous (37%) formaldehyde (1.8 mL) was added, and the mixture was stirred for 2 hours. TLC showed conversion was complete. The solvent was evaporated under reduced pressure, and the residue was dissolved in $Et_2O$. This solution was washed with saturated NaCl solution, dried ($Na_2SO_4$), filtered and evaporated to give 2.3 g of the title compound as a white foam (83% yield). Field desorption mass spectrometry (FDMS): m/z 788 (M + H).

Example 2

9-Deoxo-9-[(benzyl)amino]-9,11-(N,O)-cyclic Methylene Adduct of Erythromycin (Compound 62)

N-Benzyl-erythromycylamine (1.0 g, 1.2 mmoles) was dissolved with stirring in $CH_3CN$ (10 mL). Aqueous (37%) formaldehyde (0.4 mL) was added, and the mixture was stirred at room temperature for 4.25 hours. The white solid that formed was collected, washed with $CH_3CN$, and dried overnight under vacuum to give 220 mg of the title compound as a white solid (22%) yield); FDMS: m/z 836 (M + H).

Example 3

9-Deoxo-9-(cyclopentylamino)-9,11(N,O)-cyclic Methylene Adduct of Erythromycin (Compound 60)

N-Cyclopentylerythromycylamine (1.3 g, 1.6 mmoles) was dissolved in EtOH (10 mL). Aqueous (37%) formaldehyde (0.5 mL) was added, and the mixture was stirred at room temperature. TLC showed incomplete conversion after 24 and 48 hours, so additional formaldehyde (0.5 mL) was added at each time. TLC after 3 days showed complete conversion. The solvent was evaporated, and the resulting solid was dissolved in $Et_2O$ and washed with water. The organic layer was dried over $Na_2SO_4$, filtered and evaporated to give 1.1 g of the title compound (85% yield) as a white foam; FDMS: m/z 814 (M + H).

Example 4

9-Deoxo-9-[(5-dimethylamino-2-pentyl)amino]-9,11-(N,O)-cyclic, Methylene Adduct of Erythromycin (Compound 63)

N-[5-(Dimethylamino)-2-pentyl]-erythromycyclamine (1.0 g, 1.1 mmoles) was dissolved in $CH_3CN$ (5 mL). Aqueous (37%) formaldehyde (0.5 mL) was added, and the mixture was stirred at room temperature for 3 days. The solvent was evaporated under reduced pressure. The resulting residue was taken up in $CH_2Cl_2$, washed with saturated $NaHCO_3$ solution, dried ($Na_2SO_4$), filtered and evaporated to give a white foam. This foam was further purified by "Chromatatron" chromatography (EtOAc load; step-gradient elution with MeOH/EtOAc in the ratios: 1:9, 17:83 and 1:4) to give 50 mg of the title compound as a white foam (5% yield); FDMS: m/z 887 (M + H).

Example 5

9-Deoxo-9-[(N-methyl-N-isopropyl)amino]erythromycin (Compound 70)

Compound 58 (600 mg, 0.76 mmoles) was taken up in 4 mL each of $CH_3CN$ and pH 5.5, 0.1M potassium phosphate buffer. The pH of the solution was adjusted to 6.0 by addition of 1N HCl. Sodium cyanoborohydride (48 mg, 0.76 mmoles) was added, and the mixture was stirred at room temperature. TLC after 22 hr showed incomplete conversion so more sodium cyanoborohydride (24 mg, 0.38 mmoles) was added and stirring was continued for 18 hours.

At this time 1N NaOH was added until precipitation was complete. The acetonitrile was evaporated, and the residue was partitioned between $CH_2Cl_2$ and saturated sodium bicarbonate solution. The organic layer was dried ($Na_2SO_4$), filtered and evaporated to give 500 mg of crude product.

The crude product was further purified by flash chromatography (silica gel; step-gradient elution with $CH_2Cl_2$ and $CH_2Cl_2$/MeOH in 9:1 and 4:1 ratios) to give 100 mg of the title product (17% yield); FDMS: m/z 790 (M + H).

Example 6

9-Deoxo-9-[(N-cyclohexyl-N-methyl)amino]erythromycin (Compound 71)

The procedure described in Example 5 was used with these exceptions: 1) the starting material was the 9-Deoxo-9-(cyclohexylamino)-9,11(N,O)-cyclic methylene adduct of erythromycin (Compound 61) (2.3 g, 2.8 mmoles); 2) 12 mL each of $CH_3CN$ and buffer and 265 mg (4.2 mmoles) of sodium cyanoborohydride were used; and 3) the reaction was carried out for 1.5 hours at pH 5. The solvent was evaporated, and the residue was partitioned between water and $CH_2Cl_2$. The aqueous layer was saturated with sodium bicarbonate. The organic layer was separated, dried ($Na_2SO_4$), and evaporated to give 2.1 g (90% yield) of the title compound as a white foam; FDMS: m/z 830 (M + H).

Example 7

9-Deoxo-9-(1-piperidinyl)erythromycin (Compound 72)

Erythromycylamine (10.0 g, 13.6 mmoles) was dissolved in $CH_3CN$ (50 mL) and pH 6.5, 0.1M sodium phosphate buffer (50 mL) with warming. The pH of this solution was adjusted to 6.0 by careful addition of 6N HCl. Glutaraldehyde (4.2 mL, 20.4 mmoles) was added, and the mixture was stirred at room temperature for 3 hours. Sodium cyanoborohydride (1.3 g, 20.4 mmoles) was added in four portions, and the mixture was stirred for 1 hour.

The reaction product was worked up as in Example 6 to give a yellow foam. This foam was partitioned between diethyl ether and saturated $NaHCO_3$ solution. The organic layer was dried ($Na_2SO_4$), filtered and evaporated to give 6.3 g of crude product as a white foam.

A portion of this material (1.0 g) was further purified by reverse phase preparative high performance liquid chromatography (HPLC), using a Waters "Prep 500" unit and a gradient solvent system of 0.5% triethylamine (TEA) in $H_2O$ to $CH_3CN$:0.5% TEA in $H_2O$ (3:7), to give 300 mg of the title compound as a white foam; FDMS: m/z 802 (M + H).

Examples 8-9

9-Deoxo-9-(methylamino)erythromycin (Compound 1) and 9-Deoxo-9-(dimethylamino)erythromycin (Compound 69)

The procedure described in Example 6 was used with erythromycylamine (5.0 g, 6.8 mmoles), aqueous (37%) formaldehyde (6.8 mmoles) and sodium cyanoborohydride (640 mg, 10.2 mmoles) in $CH_3CN$ (20 mL) and pH 4.5, 0.5M sodium phosphate buffer (20 mL) at pH 5.0 for 30 minutes.

The material obtained was purified via column chromatography [silica gel; step gradient elution with $CH_2Cl_2$ and MeOH/$CH_2Cl_2$ (1:24 and 2:23)] to give 1.97 g of material containing both products.

The two products were separated from one another by reverse phase HPLC, using a Waters Prep 500 unit and eluting with a gradient of aqueous TEA-phosphoric acid buffer at pH 3.0 to $CH_3CN$/aqueous TEA-phosphoric acid buffer at pH 3.0 (1:3) to give 290 mg of Compound 1 [FDMS: m/z 749 (M + H)] and 280 mg of Compound 61 [FDMS: m/z 763 (M + H)].

General Procedure A

Erythromycylamine (5.0 g, 6.8 mmol) was dissolved in acetonitrile (20 mL) with warming. This solution was removed from the heat and magnetically stirred while 10.2 mmol of the desired aldehyde was added. A pH 4.5, 0.5M, $NaH_2PO_4$ buffer solution (20 mL) was added. The pH of this solution, which was ~7.5, was then adjusted to 5.0 with 6N HCl, and $NaBH_3CN$ (0.64 g, 10.2 mmol) was added. The pH was re-adjusted from ~6 to 5.0 with 6N HCl. The reaction mixture was allowed to stir at room temperature, and reaction progress was followed by TLC.

When the reaction appeared to be complete, the reaction mixture was evaporated to remove $CH_3CN$. The aqueous residue (~15 mL) contained a gummy solid. Saturated sodium bicarbonate solution (~150 mL) was added, and the product was extracted from the aqueous phase with $CH_2Cl_2$ (~250 mL). The organic phase was dried ($Na_2SO_4$), filtered, and evaporated to give a white amorphous solid.

General Procedure B

General procedure A was followed except that 0.86 grams (13.6 mmol) of sodium cyanoborohydride was used.

General Procedure C

Reaction Procedure B was followed, but the pH was not re-adjusted to 5 after adding sodium cyanoborohydride.

General Procedure D

Erythromycylamine was dissolved with warming in $CH_3CN$ (4 mL per gram of erythromycylamine), and the appropriate aldehyde (1.5 equivalents) was added. Sodium phosphate buffer (0.5M, pH 4.5; 4 mL/g of erythromycylamine) was added, and the pH was adjusted to 5.0 by careful addition of 6N HCl. Sodium cyanoborohydride (1.5 mol equiv.) was then added.

The mixture was stirred at room temperature, and the reaction was followed by TLC. When TLC indicated that the reaction was complete, most of the $CH_3CN$ was evaporated under reduced pressure. The resulting aqueous residue was made basic by adding 1.0N NaOH or by saturating with sodium bicarbonate. This material was extracted with $CH_2Cl_2$, and the organic layer was separated, dried over $Na_2SO_4$, filtered and evaporated to give the crude product as a white amorphous solid.

Example 10

9-Deoxo-9-(n-pentylamino)erythromycin (Compound 5)

Reaction Procedure: A
Reaction Time: 2 hours
Aldehyde: pentanal
Isolation Procedure: Product was crystallized from $CH_3CN$.
Yield: 1.142 g (21%)
mp: 163°; FDMS: m/z 804 (M + H).

Example 11

9-Deoxo-9-(n-hexylamino)erythromycin (Compound 6)

Reaction Procedure: B
Reaction Time: 45 minutes
Aldehyde: hexanal
Isolation Procedure: Product was isolated by Waters Prep 500 chromatography (silica gel), using an 8-L gradient of hexane to ethyl acetate containing 1% TEA with an additional 4 L of EtOAc containing 1% TEA. The product was crystallized from $CHCl_3$/hexane.
Yield: 1.328 g (24%)
mp: 98°; FDMS: m/z 819 (M + H).

Example 12

9-Deoxo-9-(-n-heptylamino)erythromycin (Compound 7)

Reaction Procedure: A
Reaction Time: 2 hours
Aldehyde: heptanal
Isolation Procedure: Basic alumina flash column chromatography (activity grade 3), eluting stepwise with $CH_2Cl_2$ (1 L), $CH_2Cl_2/CHCl_3$ (1:1, 1 L) and $CHCl_3$ (2 L). Product crystallized from $CH_3CN$.
Yield: 0.776 g (14%).
mp: 101°; FDMS: m/z 833 (M + H).

Example 13

9-Deoxo-9-(n-octylamino)erythromycin (Compound 8)

Reaction Procedure: C
Reaction Time: 1.5 hour
Aldehyde: octanal
Isolation Procedure: Waters Prep 500 chmromatography (silica gel), using an 8-L gradient of $CH_2Cl_2$ to $MeOH/CH_2Cl_2/NH_4OH$ (7.5:90.5:2), and then basic alumina flash chromatography (activity grade 3), eluting with $CHCl_3$ (2 L) to give a white amorphous solid (foam).
Yield: 0.634 g (11%).
FDMS: m/z 847 (M + H).

Example 14

9-Deoxo-9-(n-decylamino)erythromycin (Compound 9)

Reaction Procedure: B
Reaction Time: 1 hour
Aldehyde: decanal
Isolation Procedure: Alumina flash chromatography as in Example 13, followed by silica-gel flash chromatography (silica 60, finer than 230 mesh) eluting with $CHCl_3$ (250 mL), a 1.5-L gradient of $CHCL_3$ to $MeOH/CHCl_3/NH_4OH$ (8:91.5:0.5), plus an additional liter of the latter solvent to give a white amorphous solid (foam).
Yield: 1.28l g (22%)
FDMS: m/z 875 (M + H).

Example 15

9-Deoxo-9-(n-dodecylamino)erythromycin (Compound 10)

Reaction Procedure: B
Reaction Time: 1.5 hour
Aldehyde: dodecanal
Isolation Procedure: Alumina chromatography as in Example 12 and then silica flash chromatography (silica 60, 230-400 mesh), eluting with $CHCl_3$ (250 mL), a 1.5-L gradient of $CHCl_3$ to MeOH/$CHCl_3$/$NH_4OH$ (6:93.5:0.5), and an additional liter of the latter solvent to give a white foam.
Yield: 2.054 g (34%)
FDMS: m/z 903 (M + H).

Example 16

9-Deoxo-9-[(3-methylbutyl)amino]erythromycin (Compound 11)

Reaction Procedure: B
Reaction Time: 1.5 hour
Aldehyde: isovaleraldehyde
Isolation Procedure: Silica flash chromatography (silica 60, finer than 230 mesh), eluting with $CHCl_3$ (250 mL), a 1.5-L gradient of $CHCl_3$ to MeOH/$CHCl_3$/$NH_4OH$ (6:93.5:0.5), and an additional 2 L of the latter solvent; then, basic alumina chromatography (activity grade 3), eluting with $CHCl_3$ (2 L) to give a white foam.
Yield: 2.073 g (38%).
FDMS: m/z 805 (M + H).

Example 17

9-Deoxo-9-[(2-ethylbutyl)amino]erythromycin (Compound 12)

Reaction Procedure: B
Reaction Time: 1.25 hour
Aldehyde: 2-ethylbutyraldehyde
Isolation Procedure: The reaction product was broken into a fine powder in the presence of hexane and was then filtered. The solid material was dissolved in $CH_2Cl_2$ and placed on a basic alumina flash column (activity grade 3), which was eluted with $CH_2Cl_2$ (1 L) and $CHCl_3$ (2 L). The product crystallized from $CHCl_3$/hexane.
Yield: 2.798 g (50%).
mp: 190°; FDMS: m/z 819 (M + H).

Example 18

9-Deoxo-9-[(trans-dec-4-enyl)amino]erythromycin (Compound 14)

Reaction Procedure: A
Reaction Time: 2 hours
Aldehyde: trans-dec-4-enal
Isolation Procedure: Basic alumina flash chromatography (activity grade 3), eluting stepwise with $CH_2Cl_2$ (1 L), $CH_2Cl_2$:$CHCl_3$ (1 L each of 1:1 and 1:3) and $CHCl_3$ (1 L) to give a white foam. This material was crystallized from $CH_3CN$ and further purified by silica flash chromatography (silica 60, finer than 230 mesh), eluting with $CHCl_3$ (250 mL), a gradient of $CHCl_3$ (1.5 L) to MeOH/$CHCl_3$/$NH_4OH$ (10:89.5:0.5), and an additional liter of the latter solvent to give the title compound as a white foam.
Yield: 1.113 g (19%).
FDMS: m/z 873 (M + H).

Example 19

9-Deoxo-9-[cis-dec-4-enyl)amino]erythromycin (Compound 13)

Reaction Procedure: A
Reaction Time: 2 hours
Aldehyde: cis-dec-4-enal
Isolation Procedure: Basic alumina flash chromatography as in Example 18, following by silica flash chromatography as in Example 18 to give a white foam.

Yield: 1.373 g (23%)
FDMS: m/z 873 (M + H).

Example 20

9-Deoxo-9-[(undec-10-enyl)amino]erythromycin (Compound 28)

Reaction Procedure: A
Reaction Time: 1.5 hour
Aldehyde: undec-10-enal
Isolation Procedure: Basic alumina flash chromatography as in Example 18, eluting with $CH_2Cl_2$ (1 L), a 2-L gradient of $CH_2Cl_2$ to $CHCl_3$, plus an additional 1 L of $CHCl_3$, followed by silica flash chromatography as in Example 18 to give the final product.
Yield: 1.606 g (27%).
FDMS: m/z 886 (M + H).

Example 21

9-Deoxo-9-[(3-cyanopropyl)amino]erythromycin (Compound 29)

Reaction Procedure: A
Reaction Time: 2.5 hours
Aldehyde: 3-cyanopropanal
Isolation Procedure: Basic alumina flash chromatography as in Example 20, but eluting with $CH_2Cl_2$ (250 mL), a 2-L gradient of $CH_2Cl_2$ to $CHCl_3$, plus an additional 1.5 L of $CHCl_3$. The product was crystallized from $CH_3CN$.
Yield: 1.007 g (19%)
mp: 135-140°; FDMS: m/z 801 (M + H).

Example 22

9-Deoxo-9-[(5-hydroxypentyl)amino]erythromycin (Compound 21)

Reaction Procedure: A
Reaction Time: 2 hours
Aldehyde: 5-hydroxypentanal
Isolation Procedure: Basic alumina flash chromatography a s in Example 18 with the addition of 2 L of MeOH/$CHCl_3$ (1.99). The product was further purified by silica flash chromatography as in Example 18 and then was crystallized from $CH_3CN$.
Yield: 0.800 G 914%).
mp: 145°; FDMS: m/z 821 (M + H).

Example 23

9-Deoxo-9-[(3-phenyl-2-propynyl)amino]erythromycin (Compound 18)

Reaction Procedure: A
Reaction Time: 1.75 hour
Aldehyde: phenylpropargyl aldehyde
Isolation Procedure: Basic alumina flash chromatography as in Example 18. The product was crystallized from $CH_3CN$ and then further purified via silica flash chromatography as in Example 18 to give the title compound.
Yield: 0.809 g (14%).
FDMS: m/z 849 (M + H).

Examples 24-25

Endo- and Exo-9-Deoxo-9-[(bicyclo[2.2.1]hept-2-en-5-yl-methyl)amino]erythromycin Compounds 19 and 20)

Reaction Procedure: A
Reaction Time: 1.5 hour
Aldehyde: 5-norbornen-2-carboxaldehyde (endo-, exo-mixture)
Islation Procedure: Basic alumina flash chromatography as in Example 12. The product was crystallized from $CH_3CN$.

Yield: 1.680 g (29%)
mp: 138–142°: FDMS: m/z 841 (M + H).

Example 26

9-Deoxo-9-[[2-(2,6,6-trimethyl-1-cyclohexen-1-yl)ethyl]amino]erythromycin (Compound 30)

Reaction Procedure: A
Reaction Time: 2.5 hours
Aldehyde: 2,6,6-trimethyl-1-cyclohexen-1-acetaldehyde
Isolation Procedure: Basic alumina flash chromatography as in Example 21 except that elution volumes were $CH_2Cl_2$ (250 mL); a 2-L gradient of $CH_2Cl_2$ to $CHCl_3$, and an additional 1 L of $CHCl_3$. The product was crystallized from $CH_3CN$.
Yield: 0.946 g (16%)
mp: 172-175°; FDMS: m/z 885 (M + H).

Example 27

9-Deoxo-9-[(cyclooctylmethyl)amino]erythromycin (Compound 16)

Reaction Procedure: A
Reaction Time: 2.5 hours
Aldehyde: cyclooctanecarboxaldehyde
Isolation Procedure: Same as Example 26; crystallized from $CH_3CN$
Yield: 1.385 g (24%)
mp: 200°; FDMS: m/z 858 (M + H).

Example 28

9-Deoxo-9-[[[2-(ethoxycarbonyl)cyclopropyl]methyl]amino]erythromycin (Compound 27)

Reaction Procedure: A
Reaction Time: 3.5 hours
Aldehyde: ethyl 2-formyl-1-cyclopropanecarboxylate
Isolation Procedure: Basic alumina flash chromatography as in Example 26; then silica-gel flash chromatography as in Example 18.
Yield: 1.726 g (30%)
FDMS: m/z 860 (M + H).

Example 29

9-Deoxo-9-(aziridin-1-yl)erythromycin (Compound 73)

Reaction Procedure: A
Reaction Time: 1.5 hour
Aldehyde: 2-chloroacetaldehyde (50% in $H_2O$)
Isolation Procedure: Basic alumina flash chromatography as in Example 26.
Yield: 1.781 g (34%).
FDMS: m/z 760 (M + H).

Example 30

(9S)-9-Deoxo-9-(ethylamino)erythromycin (Compound 2)

Reaction Procedure: D [5.0 g (6.8 mmoles) of erythromycylamine and 0.45 g (10.2 mmoles) of acetaldehyde]
Reaction Time: 19 hours
Isolation Procedure: Initial workup gave 5.1 g of crude product as a white foam. This crude product was purified by flash chromatography [silica gel; $CH_2Cl_2$:MeOH (9:1 → 4:1)]
Yield: 360 mg (7%)
FDMS: m/z 762 (M + H).

Example 31

9-Deoxo-9-(n-propylamino)erythromycin (Compound 3

Reaction Procedure: D [10.0 g (13.6 mmoles) of erythromycylamine and 1.2 g (20.4 mmoles) of propionaldehyde]
Reaction Time: 3 hours
Isolation Procedure: Initial workup gave 10.5 g of crude product as a white foam; this product was purified by Waters Prep 500 HPLC [$CH_2Cl_2 \rightarrow$ MeOH/$CH_2Cl_2$/$NH_4OH$ (5:94:1)].
Yield: 5.9 g (56%)
FDMS: m/z 776 (M + H).

Example 32

9-Deoxo-9-(n-butylamino)erythromycin (Compound 4)

Reaction Procedure: D [7.0 g (9.5 mmoles) of erythromycylamine and 1.0 g (14.3 mmoles) of butyraldehyde]
Reaction Time: 19.5 hours; TLC showed incomplete conversion, so 1.0 g (14.3 mmoles) of butyraldehyde was added, and the mixture was stirred for an additional 5 hours at room temperature.
Isolation Procedure: Initial workup gave 8.5 g of crude product as a white foam, which was purified by Waters Prep 500 HPLC as in Example 31.
Yield 1.3 g (17%)
FDMS: m/z 790 (M + H).

Example 33

(9S)-9-Deoxo-9-[(3-phenylpropyl)amino]erythromycin (Compound 17)

Reaction Procedure: D [10.0 g (13.6 mmoles) of erythromycylamine and 2.7 g (20.4 mmoles) of hydrocinnamaldehyde]
Reaction Time: 10 minutes
Isolation Procedure: The reaction mixture had separated into two distinct layers. The organic layer was evaporated and taken up in $CH_2Cl_2$. The aqueous layer was saturated with sodium bicarbonate and extracted with the $CH_2Cl_2$ containing the original organic layer. The $CH_2Cl_2$ was dried ($Na_2SO_4$), filtered and evaported to give 12.4 g of crude product. This material was purified by Waters Prep 500 HPLC, eluting with $CH_2Cl_2 \rightarrow$ MeOH/$CH_2Cl_2$/$NH_4OH$ (7.5:87.5:5).
Yield: 5.0 g (43%) as a white foam.
FDMS: m/z 852 (M + H).

Example 34

(9S)-9-Deoxo-9-[(3-methoxypropyl)amino]erythromycin (Compound 23)

Reaction Procedure: D [10.0 g (13.6 mmoles) of erythromycin and 1.8 g (20.4 mmoles) of 3-methoxypropionaldehyde]
Reaction Time: 1 hour
Isolation Procedure: Initial workup gave 9.1 of crude product which was purified by Waters Prep 500 HPLC [$CH_2Cl_2 \rightarrow$ MeOH/$CH_2Cl_2$/$NH_4OH$ (7.5:90:2)].
Yield: 2.4 g (22%)
FDMS: m/z 806 (M + H).

Example 35

9-Deoxo-9-[(1-cyclohex-4-enylmethyl)amino]erythromycin (Compound 15)

Reaction Procedure: D [10.0 g (13.6 mmoles) of erythromycylamine and 2.2 g (20.4 mmoles) of 1,2,3,6-tetrahydrobenzaldehyde]
Reaction Time: 1 hour
Isolation Procedure: Initial workup gave 12.6 g of crude product as a white foam which was purified by Waters Prep 500 HPLC [$CH_2Cl_2 \rightarrow$ MeOH/$CH_2Cl_2$ (5:95)].
Yield: 1.6 g (14%)
FDMS: m/z 829 (M + H).

Example 36

9-Deoxo-9-[[3-(methylthio)propyl]amino]erythromycin (Compound 26)

Reaction Procedure: D [10.0 g (10.6 mmoles) of erythromycylamine and 2.1 g (20.4 mmoles) of 3-(methylthio)propionaldehyde]
Reaction Time: 5 hours
Isolation Procedure: Initial workup gave 12.2 g of crude product as a white foam; this was purified by Waters Prep 500 HPLC [$CH_2Cl_2 \rightarrow MeOH/CH_2Cl_2$ (1:9)].
Yield: 1.5 g (13%)
FDMS: m/z 823 (M + H).

Example 37

9-Deoxo-9-[(3-hydroxy-2,2-dimethylpropyl)amino]erythromycin (Compound 22)

Reaction Procedure: D [5.0 g (6.8 mmoles) of erythromycylamine and 1.0 g (10.2 mmoles) of 3-hydroxy-2,2-dimethylpropionaldehyde]
Reaction Time: 5 hours [after 3 hours, additional sodium cyanoborohydride (10.2 mmoles) was added; 1 hour later, additional aldehyde (10.2 mmoles) was added; TLC after 5 hours showed no further conversion than had occurred at 1 hour].
Isolation Procedure: Initial workup gave a white foam which crystallized from acetonitrile.
Yield: 864 mg (16%)
FDMS: m/z 821 (M + H).

Example 38

9-Deoxo-9-[(7-methoxy-3,7-dimethyloctyl)amino]erythromycin (Compound 24)

Reaction Procedure: D [5.0 g (6.8 mmoles) of erythromycylamine and 1.9 g (10.2 mmoles) of 7-methoxy-3,7-dimethyloctanaldehyde]
Reaction Time: 1 hour
Isolation Procedure: Initial workup gave 6.6 g of crude product which was purified by silica gravity column chromatography [$CH_2Cl_2 \rightarrow MeOH/CH_2Cl_2$ (1:24)]
Yield: 1.0 g (16%)
FDMS: m/z 904 (M + H).

Example 39

9-Deoxo-9-[[2-(2-methoxyethoxy)ethyl]amino]erythromycin (Compound 25)

(9S)-9,11-Dideoxy-9,11-[imino[2-(2-methoxyethoxy)ethylidene]oxy]erythromycin (1.0 g, 1.2 mmoles), prepared as described in U.S. patent 4,048,306, was dissolved in $CH_3CN$ (5 mL) and pH 4.5, 0.5m potassium phosphate buffer (5 mL). The pH of the resulting solution was adjusted to 5.0 by adding 6N HCl. Sodium cyanoborohydride (302 mg, 4.8 mmoles) was added, and the mixture was stirred at room temperature for 90 hours.

The reaction mixture was worked up as described in general procedure D to give 1.5 g of crude product. This crude product was purified by silica-gel flash chromatography, eluting stepwise with $CH_2Cl_2$, $MeOH/CH_2Cl_2$ (1:24) and $MeOH/CH_2Cl_2$ (2:23).
Yield: 450 mg (45%)
FDMS: m/z 837 (M + H).

Example 40

Preparation of Compound 3 by Hydrogenation

Erythromycylamine (150 g, 0.204 mole) and propionaldehyde (18 g, 0.310 mole) were dissolved in a mixture of tetrahydrofuran (750 mL) and methanol (1200 mL). This solution was hydrogenated over 5% palladium on carbon (150 g) at 120°C for 16 hr at 500 psi. The solvent was then removed under vacuum to give 133 g of crude product. This material was purified by reverse-phase silica-gel column chromatography (15 μ, C8), eluting with aqueous 0.25% acetic acid containing 0 to 5% $CH_3CN$.

Example 41

9-Deoxo-9-[(2-Methoxyethyl)amino]erythromycin (Compound 31)

Methoxyacetaldehyde dimethylacetal (0.79 mL, 6.2 mmoles) in 1 N hydrochloric acid (6 mL) was stirred for 4 hrs.

Erythromycylamine (3.0 g, 4.1 mmoles) was dissolved with warming in acetonitrile (12 mL), and sodium phosphate buffer (12 mL; 0.5 M, pH 6.5) was added. The acidic methoxyacetaldehyde solution was added dropwise. Hydrochloric acid (6 N) was carefully added until the pH of the reaction mixture was 5.0. After the mixture was stirred for 10 minutes at room temperature, sodium cyanoborohydride (390 mg, 6.2 mmoles) was added. One hour after sodium cyanoborohydride addition, the mixture was worked up as in Procedure D to give 2.6 g white solid.

Flash chromatography [silica gel; $CH_2Cl_2$:$CH_3OH$ (24:1) elution] of the solid gave 1.1 g (34% yield) of the title compound. FDMS m/z 794 (M + H).

Tables VIII-X summarize relevant proton nuclear magnetic resonance (NMR) data for exemplified compounds.

**Table VIII: Proton NMR of Formula 1 Compounds**

| Compound Number | Example Number | C-9-Substituent | Representative Chemical Shift Values δ (ppm from internal TMS) ($CDCl_3$ solvent) | | |
|---|---|---|---|---|---|
| | | | 9-H | $-NHCH_2$ | Other H's |
| 1 | 8 | -NHMe | 2.05 | - | $NHCH_3$ 2.10 |
| 2 | 30 | -NHEt | 2.21 | 2.75/~2.45 | |
| 3 | 31 | -NH(nPr) | 2.18 | 2.70/ 2.46 | |
| 4 | 32 | -NHBu | 2.18 | 2.72/~2.45 | |
| 5 | 10 | $-NH(CH_2)_4Me$ | 2.18 | 2.72/~2.46 | |
| 6 | 11 | $-NH(CH_2)_5Me$ | 2.18 | 2.72/~2.46 | |
| 7 | 12 | $-NH(CH_2)_6Me$ | 2.18 | 2.72/~2.46 | |
| 8 | 13 | $-NH(CH_2)_7Me$ | 2.18 | 2.72/~2.46 | |
| 9 | 14 | $-NH(CH_2)_9Me$ | 2.18 | 2.73/~2.46 | |
| 10 | 15 | $-NH(CH_2)_{11}Me$ | 2.18 | 2.72/~2.46 | |
| 11 | 16 | $-NH(CH_2)_2CH(Me)_2$ | 2.18 | 2.74/~2.46 | |
| 12 | 17 | $-NHCH_2CH(Et)_2$ | 2.15 | 2.66/~2.45 | |
| 13 | 19 | -NH(cis-dec-4-enyl) | 2.18 | 2.74/~2.46 | olefinic 5.38 |
| 14 | 18 | -NH(trans-dec-4-enyl) | 2.18 | 2.72/~2.45 | olefinic 5.38 |
| 15 | 35 | $-NHCH_2$(cyclohex-3-enyl) | 2.10 | 2.60/2.45 | olefinic 5.68 |
| 16 | 27 | $-NHCH_2$(cyclooctyl) | 2.16 | ~2.6/~2.45 | |
| 17 | 33 | $-NH(CH_2)_3Ph$ | 2.18 | 2.78/~2.45 | aromatic 7.24/7.18 |
| 18 | 23 | $-NHCH_2C{\equiv}CPh$ | ~2.2 | 3.77/ 3.54 | aromatic 7.3/7.4 |

## Table VIII, cont'd.

| Compound Number | Example Number | C-9-Substituent | Representative Chemical Shift Values δ (ppm from internal TMS) ($CDCl_3$ solvent) 9-H | -NHC$\underline{H_2}$ | Other H's |
|---|---|---|---|---|---|
| 19,20 | 24,25 | $-NHCH_2$(5-norbornen-2-yl) | 2.12 | ~2.8/2.5 | olefinic 6.12/5.94 |
| 21 | 22 | $-NH(CH_2)_5OH$ | 2.18 | 2.76/~2.46 | |
| 22 | 37 | $-NHCH_2C(Me)_2CH_2OH$ | 2.16 | 2.90 | $C(CH_3)_2$ 0.92/0.84 |
| 23 | 34 | $-NH(CH_2)_3OMe$ | 2.18 | ~2.80/2.52 | OMe 3.34 |
| 24 | 38 | -NH(3,7-diMe-7-MeO-octyl) | ~2.2 | ~2.5 | OMe 3.32 |
| 25 | 39 | $-NH(CH_2)_2O(CH_2)_2OMe$ | 2.18 | 2.50 | OMe 3.40 |
| 26 | 36 | $-NH(CH_2)_3SMe$ | 2.08 | 2.64/~2.5 | SMe 2.10 |
| 27 | 28 | $-NHCH_2$(2-COOEt-cycloprop-1-yl) | ~2.29 | 2.62/~2.46 | O<br>‖<br>C-O-C$\underline{H_2}$ 4.12 |
| 28 | 20 | -NH(undec-10-en-1-yl) | 2.19 | 2.72/~2.48 | olefinic 5.82/4.96 |
| 29 | 21 | $-NH(CH_2)_3CN$ | 2.21 | 2.84/2.62 | |
| 30 | 26 | $-NH(CH_2)_2$(2,6,6-triMe-cyclohex-1-en-1-yl) | 2.24 | 2.77/~2.46 | |
| 31 | 41 | $-NH(CH_2)OMe$ | 2.18 | 2.59/2.82 | OMe 3.28 |
| 38 | - | $-NH(CH_2)_2CH_3$ | - | 2.73/2.48 | |
| 41 | - | $-NH(CH_2)_9CH_3$ | - | 2.77/~2.5 | |
| 49 | - | $-NH(CH_2)_2O(CH_2)_2OMe$ | - | ~2.9/2.68 | 3.44-3.70 ($CH_2O$),<br>3.40 ($CH_3$) |

Table IX: Proton NMR of Formula 2 Compounds

| Compound Number | Example Number | C-9-Substituent | Chemical Shift of $N-CH_2-O$ (δ value) |
|---|---|---|---|
| 58 | 1 | $-N(CH_2-O-11)(iPr)$ | 4.62 |
| 60 | 3 | $-N(CH_2-O-11)$(cyclopentyl) | 4.64 |
| 62 | 2 | $-N(CH_2-O-11)$(benzyl) | 4.36 |
| 63 | 4 | $-N(CH_2-O-11)$(5-diMeamino-2-pentyl) | 4.59 |

Table X: Proton NMR of Formula 3 Compounds

| Compound Number | Example Number | C-9-Substituent | Chemical Shift (δ value) | |
|---|---|---|---|---|
| | | | $N-CH_3$ | $N-CH_2$ |
| 69 | 9 | $-N(Me)_2$ | 2.43 | |
| 70 | 5 | $-N(Me)(iPr)$ | 2.42 | |
| 71 | 6 | $-N$(cyclohexyl)(Me) | 2.40 | |
| 72 | 7 | $-N(CH_2)_5$ | – | 2.80/2.64 |
| 73 | 29 | $-N(CH_2)_2$ | – | 1.81/1.73 |

**Claims for the Contracting States: BE, CH, DE, FR, GB, IT, LI, LU, NL, SE**

1. A compound of formula (1):

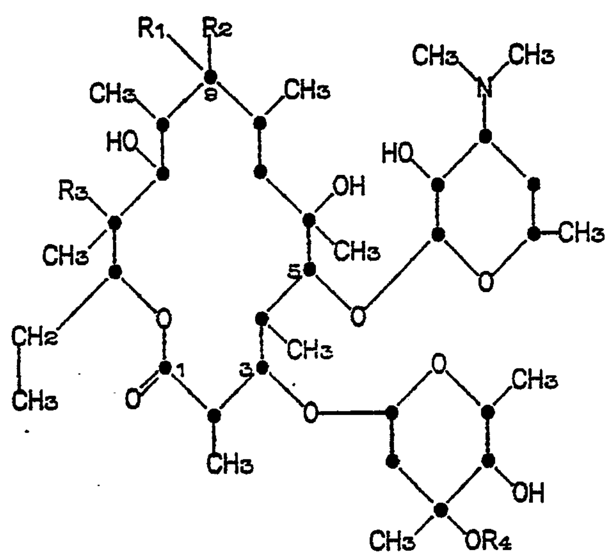

(1)

wherein $R_1$ and $R_2$ are different and are hydrogen or $-NHCH_2R_5$;
$R_3$ is hydrogen or hydroxyl;
$R_4$ is hydrogen or methyl; and
$R_5$ is hydrogen or a $C_1$-$C_{14}$-alkyl or $-(CH_2)_lX(CH_2)_mY$ group, either of which group may have from one to three substituents selected from halo, hydroxyl, $C_1$-$C_4$-alkyl, $C_1$-$C_4$-alkoxy, $C_1$-$C_4$-alkylthio, cyano, $C_1$-$C_4$-alkoxycarbonyl, mono- or di($C_1$-$C_4$-alkyl)amino, $-N(CH_2)_s$, $R_6$-substituted-phenyl, or an $R_6$-substituted-monocyclic heterocyclic group having from 3 to 7 ring atoms;
X is oxygen or sulfur;
Y is $-X(CH_2)_nCH_3$, $-N(CH_2)_s$ or $-N[(CH_2)_nCH_3]_2$;
l is 1 or 2;
m is an integer from 1 to 3;
n is an integer from 0 to 3;
s is an integer from 2 to 7; and

$R_6$ is hydrogen, halo, $C_1$-$C_4$-alkyl or $C_1$-$C_4$-alkoxy; provided that, when the substituent on the $R_5$ group is selected from hydroxyl, cyano, alkoxycarbonyl, mono- or dialkylamino or $-N(CH_2)_s$, it cannot be located on the second or third carbon atom from the nitrogen of the $-NHCH_2R_5$ group unless the second carbon atom is quaternary;
or a salt thereof.

2. A compound of claim 1 wherein $R_5$ is hydrogen, $C_1$-$C_{14}$-alkyl or substituted $C_1$-$C_{14}$-alkyl.

3. A compound of claim 2 wherein $R_5$ is $C_1$-$C_5$-alkyl or substituted $C_1$-$C_5$-alkyl.

4. A compound of claim 2 wherein $R_5$ is $C_6$-$C_{14}$-alkyl or substituted $C_6$-$C_{14}$-alkyl.

5. A compound of claim 1 wherein $R_5$ is a $-(CH_2)_lX(CH_2)_mY$ group or a substituted $-(CH_2)_lX(CH_2)_mY$ group.

6. A compound of claim 5 wherein X is oxygen and Y is $-O(CH_2)_nCH_3$.

7. A compound of formula (2):

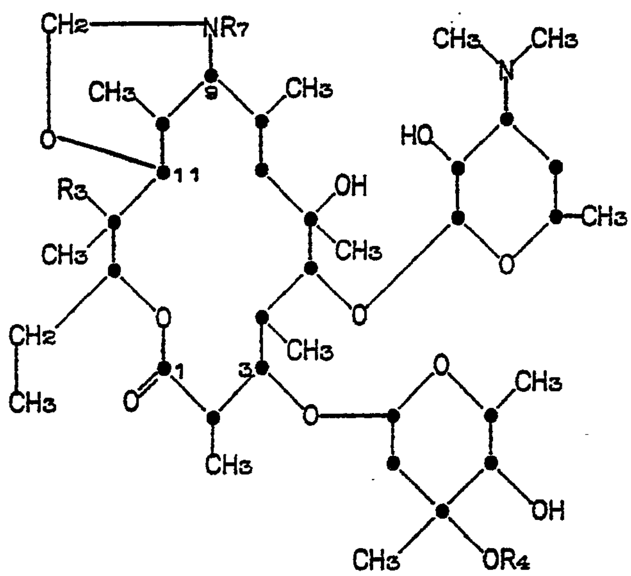

(2)

wherein $R_3$ is hydrogen or hydroxyl;
$R_4$ is hydrogen or methyl;
$R_7$ is $CH_2R_5$, $C_3$-$C_8$-cycloalkyl, $-CHR_8(CH_2)_pR_9$, $-(CH_2)_qR_{10}$ or $-CH_2(CH=CH)_rAr$;
$R_5$ is hydrogen or a $C_1$-$C_{14}$-alkyl or $-(CH_2)_lX(CH_2)_mY$ group, either of which group may have from one to three substituents selected from halo, hydroxyl, $C_1$-$C_4$-alkyl, $C_1$-$C_4$-alkoxy, $C_1$-$C_4$-alkylthio, cyano, $C_1$-$C_4$-alkoxycarbonyl, mono- or di($C_1$-$C_4$-alkyl)amino, $-N(CH_2)_s$, $R_6$-substituted-phenyl, or an $R_6$-substituted-monocyclic heterocyclic group having from 3 to 7 ring atoms;
$R_6$ is hydrogen, halo, $C_1$-$C_4$-alkyl or $C_1$-$C_4$-alkoxy;
$R_8$ is $C_1$-$C_4$-alkyl, phenyl or benzyl;
$R_9$ is hydrogen, halo, hydroxyl, $C_1$-$C_4$-alkoxy, mono- or di($C_1$-$C_4$-alkyl)amino, $-N(CH_2)_s$ or phenyl
$R_{10}$ is hydroxy, cyano, $C_1$-$C_4$-alkoxycarbonyl, mono- or di($C_1$-$C_4$-alkyl)amino or $-N(CH_2)_s$;
Ar is phenyl; phenyl having one or more halo, $C_1$-$C_4$-alkyl, $C_1$-$C_4$-alkoxy or hydroxy substituents; or an $R_6$-substituted monocyclic aromatic heterocyclic group having from 5 to 7 ring atoms;
X is oxygen or sulfur;
Y is $-X(CH_2)_nCH_3$, $-N(CH_2)_s$ or $-N[(CH_2)_nCH_3]_2$;
l is 1 or 2;
m is an integer from 1 to 3;
n is an integer from 0 to 3;
p is an integer from 1 to 5;
q is 2 or 3;
r is 0 or 1; and
s is an integer from 2 to 7;
or a salt thereof.

8. A compound of formula (3):

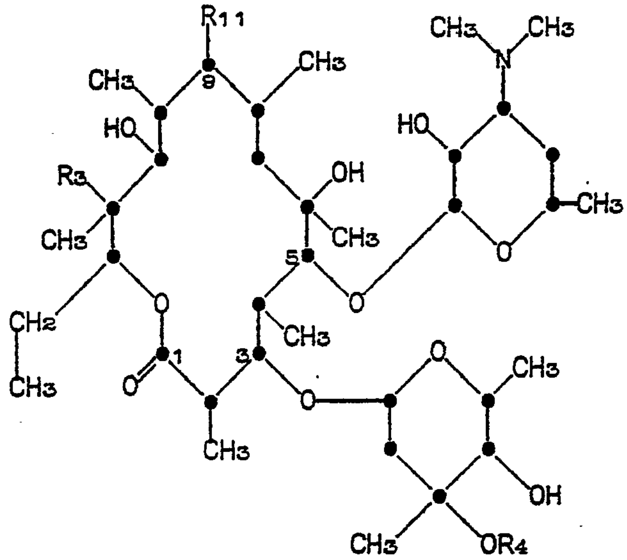

(3)

wherein $R_3$ is hydrogen or hydroxyl;
$R_4$ is hydrogen or methyl;
$R_{11}$ is $-N(CH_3)R_7$ or $-N(CH_2)_s$;
$R_7$ is $CH_2R_5$, $C_3$-$C_8$-cycloalkyl, $-CHR_8(CH_2)_pR_9$, $-(CH_2)_qR_{10}$ or $-CH_2(CH{=}CH)_rAr$;
$R_5$ is hydrogen or a $C_1$-$C_{14}$-alkyl or $-(CH_2)_lX(CH_2)_mY$ group, either of which group have from one to three substituents selected from halo, hydroxyl, $C_1$-$C_4$-alkyl, $C_1$-$C_4$-alkoxy, $C_1$-$C_4$-alkylthio, cyano, $C_1$-$C_4$-alkoxycarbonyl, mono- or di($C_1$-$C_4$-alkyl)amino, $-N(CH_2)_s$, $R_6$-substituted-phenyl, or an $R_6$-substituted-monocyclic heterocyclic group having from 3 to 7 ring atoms;
$R_6$ is hydrogen, halo, $C_1$-$C_4$-alkyl or $C_1$-$C_4$-alkoxy;
$R_8$ is $C_1$-$C_4$-alkyl, phenyl, or benzyl;
$R_9$ is hydrogen, halo, hydroxyl, $C_1$-$C_4$-alkoxy, mono- or di($C_1$-$C_4$-alkyl)amino, $-N(CH_2)_s$ or phenyl
$R_{10}$ is hydroxyl, cyano, $C_1$-$C_4$-alkoxycarbonyl, mono- or di($C_1$-$C_4$-alkyl)amino or $-N(CH_2)_s$;
Ar is phenyl; phenyl having one or more halo, $C_1$-$C_4$-alkyl, $C_1$-$C_4$-alkoxy or hydroxyl substituents; or an $R_6$-substituted monocyclic aromatic heterocyclic group having from 5 to 7 ring atoms;
X is oxygen or sulfur;
Y is $-X(CH_2)_nCH_3$, $-N(CH_2)_s$ or $-N[(CH_2)_nCH_3]_2$;
l is 1 or 2;
m is an integer from 1 to 3;
n is an integer from 0 to 3;
p is an integer from 1 to 5;
q is 2 or 3;
r is 0 or 1; and
s is an integer from 2 to 7;
or a salt thereof.

9. A pharmaceutical formulation, which comprises as an active ingredient, a compound of Formula (1), (2), or (3), or a pharmaceutically-acceptable salt thereof, as defined in claim 1, 7 or 8, respectively, associated with one or more pharmaceutically-acceptable carriers, diluents or excipients therefor.

10. A compound of Formula (1), (2), or (3), or a pharmaceutically-acceptable salt thereof, as defined in claim 1, 7 or 8, respectively, for use in the treatment, prevention or control of infectious diseases.

11. 9-Deoxo-9-(n-propylamino)erythromycin.

**Claims for the Contracting State : GR**

1. A process for preparing a compound of formula (1):

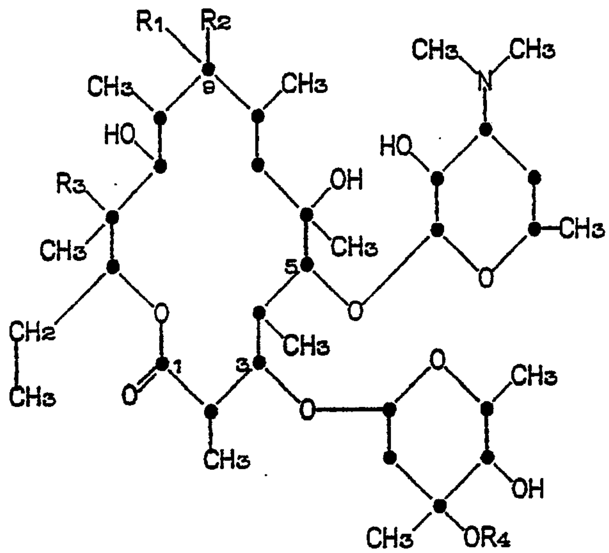

(1)

wherein $R_1$ and $R_2$ are different and are hydrogen or $-NHCH_2R_5$;
$R_3$ is hydrogen or hydroxyl;
$R_4$ is hydrogen or methyl; and
$R_5$ is hydrogen or a $C_1$-$C_{14}$-alkyl or $-(CH_2)_lX(CH_2)_mY$ group, either of which group may have from one to three substituents selected from halo, hydroxyl, $C_1$-$C_4$-alkyl, $C_1$-$C_4$-alkoxy, $C_1$-$C_4$-alkylthio, cyano, $C_1$-$C_4$-alkoxycarbonyl, mono- or di($C_1$-$C_4$-alkyl)amino, $-N(CH_2)_s$, $R_6$-substituted-phenyl, or an $R_6$-substituted-monocyclic heterocyclic group having from 3 to 7 ring atoms;
X is oxygen or sulfur;
Y is $-X(CH_2)_nCH_3$, $-N(CH_2)_s$ or $-N[(CH_2)_nCH_3]_2$;
l is 1 or 2;
m is an integer from 1 to 3;
n is an integer from 0 to 3;
s is an integer from 2 to 7; and
$R_6$ is hydrogen, halo, $C_1$-$C_4$-alkyl or $C_1$-$C_4$-alkoxy; provided that, when the substituent on the $R_5$ group is selected from hydroxyl, cyano, alkoxycarbonyl, mono- or dialkylamino or $-N(CH_2)_s$, it cannot be located on the second or third carbon atom from the nitrogen of the $-NHCH_2R_5$ group unless the second carbon atom is quaternary;
or a salt thereof, which comprises reducing, by catalytic hydrogenation or chemical reduction at a pH of 4 to 6, a compound of formula (1a):

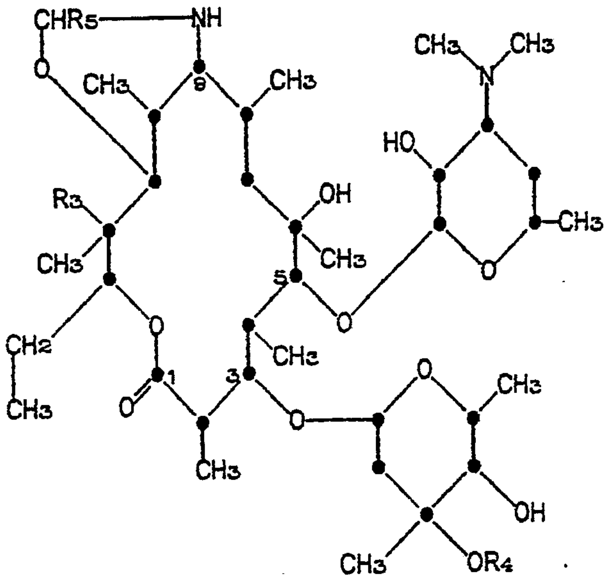

1a

and, if desired, salifying the product.

2. A process according to claim 1 in which the catalytic hydrogenation is carried out at a temperature of from 80°C to 150°C.

3. A process according to either of claims 1 and 2 for preparing a compound as claimed in claim 1 wherein $R_5$ is hydrogen, $C_1$-$C_{14}$-alkyl or substituted $C_1$-$C_{14}$-alkyl.

4. A process for preparing a compound as claimed in claim 3 wherein $R_5$ is $C_1$-$C_5$-alkyl or substituted $C_1$-$C_5$-alkyl.

5. A process for preparing a compound as claimed in claim 3 wherein $R_5$ is $C_6$-$C_{14}$-alkyl or substituted $C_6$-$C_{14}$-alkyl.

6. A process according to either of claims 1 and 2 for preparing a compound as claimed in claim 1 wherein $R_5$ is a $-(CH_2)_lX(CH_2)_mY$ group or a substituted $-(CH_2)_lX(CH_2)_mY$ group.

7. A process for preparing a compound as claimed in claim 6 wherein X is oxygen and Y is $-O(CH_2)_nCH_3$.

8. A process for preparing a compound of formula (2):

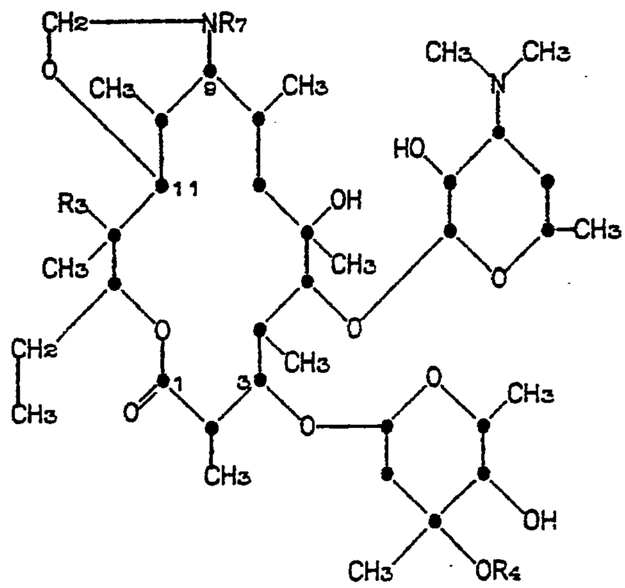

(2)

wherein $R_3$ is hydrogen or hydroxyl;
$R_4$ is hydrogen or methyl;
$R_7$ is $CH_2R_5$, $C_3$-$C_8$-cycloalkyl, $-CHR_8(CH_2)_pR_9$, $-(CH_2)_qR_{10}$ or $-CH_2(CH=CH)_rAr$;
$R_5$ is hydrogen or a $C_1$-$C_{14}$-alkyl or $-(CH_2)_lX(CH_2)_mY$ group, either of which group may have from one to three substituents selected from halo, hydroxyl, $C_1$-$C_4$-alkyl, $C_1$-$C_4$-alkoxy, $C_1$-$C_4$-alkylthio, cyano, $C_1$-$C_4$-alkoxycarbonyl, mono- or di($C_1$-$C_4$-alkyl)amino, $-N(CH_2)_s$, $R_6$-substituted-phenyl, or an $R_6$-substituted-monocyclic heterocyclic group having from 3 to 7 ring atoms;
$R_6$ is hydrogen, halo, $C_1$-$C_4$-alkyl or $C_1$-$C_4$-alkoxy;
$R_8$ is $C_1$-$C_4$-alkyl, phenyl or benzyl;
$R_9$ is hydrogen, halo, hydroxyl, $C_1$-$C_4$-alkoxy, mono- or di($C_1$-$C_4$-alkyl)amino, $-N(CH_2)_s$ or phenyl
$R_{10}$ is hydroxy, cyano, $C_1$-$C_4$-alkoxycarbonyl, mono- or di($C_1$-$C_4$-alkyl)amino or $-N(CH_2)_s$;
Ar is phenyl; phenyl having one or more halo, $C_1$-$C_4$-alkyl, $C_1$-$C_4$-alkoxy or hydroxy substituents; or an $R_6$-substituted monocyclic aromatic heterocyclic group having from 5 to 7 ring atoms;
X is oxygen or sulfur;
Y is $-X(CH_2)_nCH_3$, $-N(CH_2)_s$ or $-N[(CH_2)_nCH_3]_2$;
l is 1 or 2;
m is an integer from 1 to 3;
n is an integer from 0 to 3;
p is an integer from 1 to 5;
q is 2 or 3;
r is 0 or 1, and
s is an integer from 2 to 7;
or a salt thereof, which comprises:
reacting a compound of formula (2a):

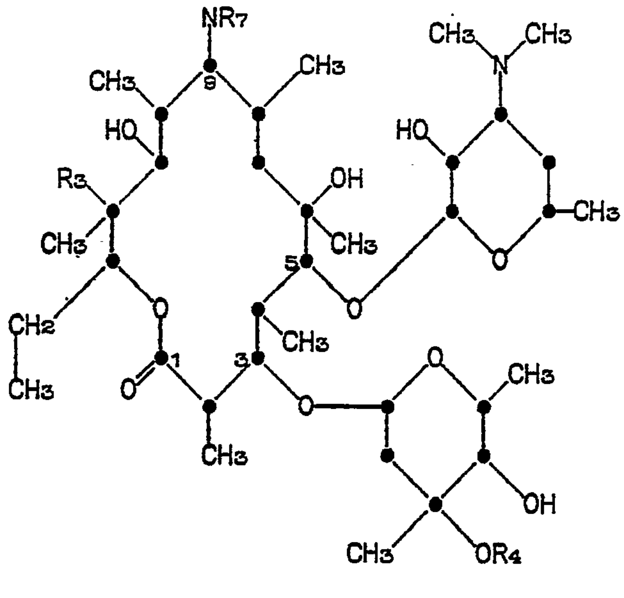

(2a)

in which $R_3$, $R_4$ and $R_7$ are as defined above, with formaldehyde.

9. A process for preparing a compound of formula (3):

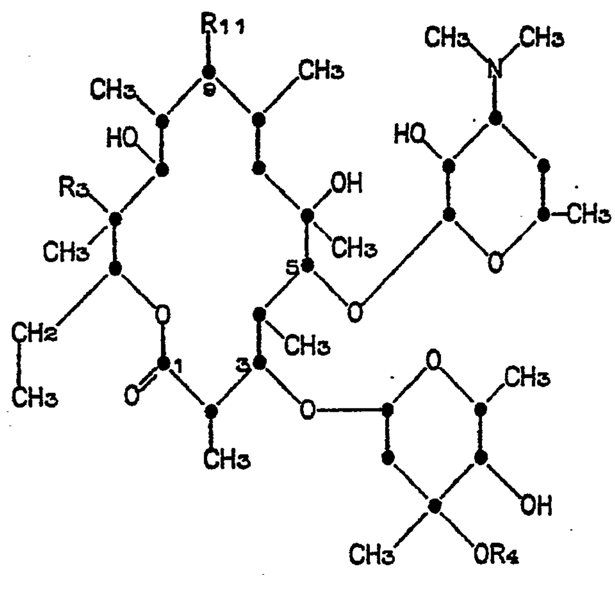

(3)

wherein $R_3$ is hydrogen or hydroxyl;
$R_4$ is hydrogen or methyl;
$R_{11}$ is $-N(CH_3)R_7$ or $-N(CH_2)_s$;
$R_7$ is $CH_2R_5$, $C_3$-$C_8$-cycloalkyl, $-CHR_8(CH_2)_pR_9$, $-(CH_2)_qR_{10}$ or $-CH_2(CH=CH)_rAr$;
$R_5$ is hydrogen or a $C_1$-$C_{14}$-alkyl or $-(CH_2)_lX(CH_2)_mY$ group, either of which group may have from one to three substituents selected from halo, hydroxyl, $C_1$-$C_4$-alkyl, $C_1$-$C_4$-alkoxy, $C_1$-$C_4$-alkylthio, cyano, $C_1$-$C_4$-alkoxycarbonyl, mono- or di($C_1$-$C_4$-alkyl)amino, $-N(CH_2)_s$, $R_6$-substituted-phenyl, or an $R_6$-substituted-monocyclic heterocyclic group having from 3 to 7 ring atoms;
$R_6$ is hydrogen, halo, $C_1$-$C_4$-alkyl or $C_1$-$C_4$-alkoxy;
$R_8$ is $C_1$-$C_4$-alkyl, phenyl or benzyl;
$R_9$ is hydrogen, halo, hydroxyl, $C_1$-$C_4$-alkoxy, mono- or di($C_1$-$C_4$-alkyl)amino, $-N(CH_2)_s$ or phenyl

$R_{10}$ is hydroxyl, cyano, $C_1$-$C_4$-alkoxycarbonyl, mono- or di($C_1$-$C_4$-alkyl)amino or $-N(CH_2)_s$;
Ar is phenyl; phenyl having one or more halo, $C_1$-$C_4$-alkyl, $C_1$-$C_4$-alkoxy or hydroxyl substituents; or an $R_6$-substituted monocyclic aromatic heterocyclic group having from 5 to 7 ring atoms;
X is oxygen or sulfur;
Y is $-X(CH_2)_nCH_3$, $-N(CH_2)_s$ or $-N[(CH_2)_nCH_3]_2$;
l is 1 or 2;
m is an integer from 1 to 3;
n is an integer from 0 to 3;
p is an integer from 1 to 5;
q is 2 or 3;
r is 0 or 1; and
s is an integer from 2 to 7;
or a salt thereof, which comprises:

(a) reducing, by catalytic hydrogenation or chemical reduction at a pH of 4 to 6, a compound of Formula (2) as defined in claim 7 to produce a compound of Formula (3) in which $R_{11}$ is a $-N(CH_3)R_7$ and, if desired, salifying the product; or

(b) reacting an erythromycylamine with a dialdehyde of the formula $OHC-(CH_2)_b-CHO$ where b is an integer from 1 to 5, followed by reducing the product, by catalytic hydrogenation at a pH of 4 to 6; and if desired, salifying the product.

10. A process according to claim 9 in which the catalyctic hydrogenation steps are carried out at a temperature of from 80°C to 150°C.

11. A process for preparing a compound of formula (1) as defined in claim 1, or a salt thereof, which comprises reacting an erythromycylamine with an aliphatic aldehyde and reducing, in situ, by catalyctic hydrogenation or chemical reduction at a pH of 4 to 6, and if desired salifying the product.

12. A process according to claim 11 for preparing a compound as claimed in claim 1 wherein $R_5$ is $C_1$-$C_5$-alkyl or substituted $C_1$-$C_5$-alkyl.

13. A process according to claim 11 for preparing a compound as claimed in claim 1 wherein $R_5$ is $C_6$-$C_{14}$-alkyl or substituted $C_6$-$C_{14}$-alkyl.

14. A process according to claim 11 for preparing a compound as claimed in claim 1 wherein $R_5$ is $-(CH_2)_lO(CH_2)_mO(CH_2)_nCH_3$.

15. A process for preparing a compound of formula (3) as defined in claim 9, or a salt thereof, which comprises reacting a compound of formula (2a) as defined in claim 8 with formaldehyde and reducing, in situ, by catalytic hydrogenation or chemical reduction at a pH of 4 to 6, to produce a compound of formula (3) in which $R_{11}$ is a $-N(CH_3)R_7$, and if desired salifying the product.

16. A compound of formula (1a), as defined in claim 1, or a salt thereof.

17. A compound of formula (2a), as defined in claim 8, or a salt thereof.

18. A compound of formula (1), as defined in claim 1, or a salt thereof.

19. A compound of formula (2), as defined in claim 8, or a salt thereof.

**Claims for the Contracting States: AT, ES**

1. A compound of formula (1):

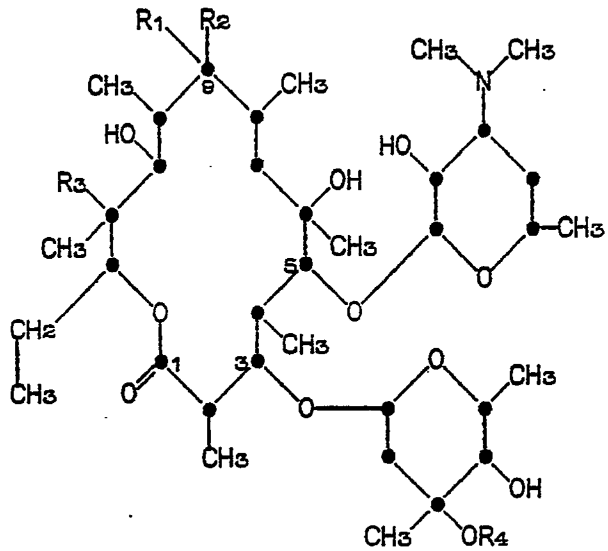

(1)

wherein $R_1$ and $R_2$ are different and are hydrogen or $-NHCH_2R_5$;
$R_3$ is hydrogen or hydroxyl;
$R_4$ is hydrogen or methyl; and
$R_5$ is hydrogen or a $C_1$-$C_{14}$-alkyl or $-(CH_2)_lX(CH_2)_mY$ group, either of which group may have from one to three substituents selected from halo, hydroxyl, $C_1$-$C_4$-alkyl, $C_1$-$C_4$-alkoxy, $C_1$-$C_4$-alkylthio, cyano, $C_1$-$C_4$-alkoxycarbonyl, mono- or di($C_1$-$C_4$-alkyl)amino, $-N(CH_2)_s$, $R_6$-substituted-phenyl, or an $R_6$-substituted-monocyclic heterocyclic group having from 3 to 7 ring atoms;
X is oxygen or sulfur;
Y is $-X(CH_2)_nCH_3$, $-N(CH_2)_s$ or $-N[(CH_2)_nCH_3]_2$;
l is 1 or 2;
m is an integer from 1 to 3;
n is an integer from 0 to 3;
s is an integer from 2 to 7; and
$R_6$ is hydrogen, halo, $C_1$-$C_4$-alkyl or $C_1$-$C_4$-alkoxy; provided that, when the substituent on the $R_5$ group is selected from hydroxyl, cyano, alkoxycarbonyl, mono- or dialkylamino or $-N(CH_2)_s$, it cannot be located on the second or third carbon atom from the nitrogen of the $-NHCH_2R_5$ group unless the second carbon atom is quaternary;
or a salt thereof, which comprises reducing, by catalytic hydrogenation or chemical reduction at a pH of 4 to 6, a compound of formula (1a):

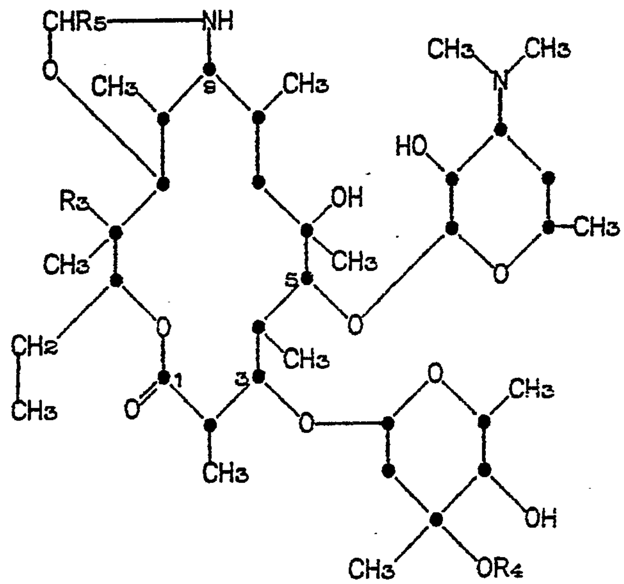

1a

and, if desired, salifying the product.

2. A process according to claim 1 in which the catalytic hydrogenation is carried out at a temperature of from 80°C to 150°C.

3. A process according to either of claims 1 and 2 for preparing a compound as claimed in claim 1 wherein $R_5$ is hydrogen, $C_1$-$C_{14}$-alkyl or substituted $C_1$-$C_{14}$-alkyl.

4. A process for preparing a compound as claimed in claim 3 wherein $R_5$ is $C_1$-$C_5$-alkyl or substituted $C_1$-$C_5$-alkyl.

5. A process for preparing a compound as claimed in claim 3 wherein $R_5$ is $C_6$-$C_{14}$-alkyl or substituted $C_6$-$C_{14}$-alkyl.

6. A process according to either of claims 1 and 2 for preparing a compound as claimed in claim 1 wherein $R_5$ is a $-(CH_2)_lX(CH_2)_mY$ group or a substituted $-(CH_2)_lX(CH_2)_mY$ group.

7. A process for preparing a compound as claimed in claim 6 wherein X is oxygen and Y is $-O(CH_2)_nCH_3$.

8. A process for preparing a compound of formula (2):

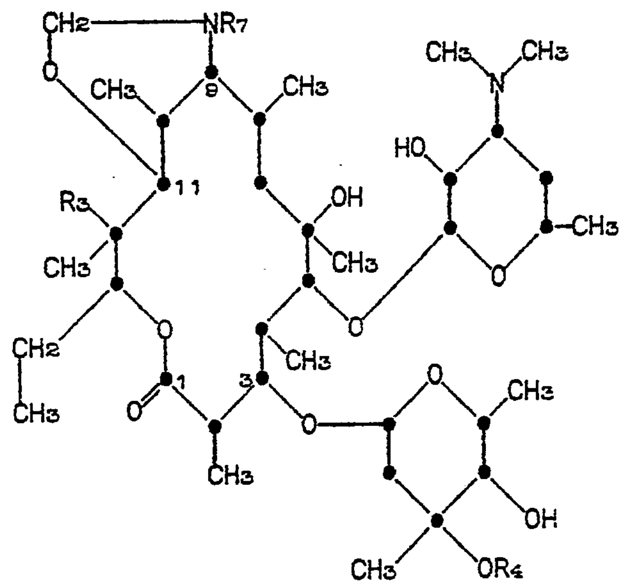

(2)

wherein $R_3$ is hydrogen or hydroxyl;
$R_4$ is hydrogen or methyl;
$R_7$ is $CH_2R_5$, $C_3$-$C_8$-cycloalkyl, $-CHR_8(CH_2)_pR_9$, $-(CH_2)_qR_{10}$ or $-CH_2(CH{=}CH)_rAr$;
$R_5$ is hydrogen or a $C_1$-$C_{14}$-alkyl or $-(CH_2)_lX(CH_2)_mY$ group, either of which group may have from one to three substituents selected from halo, hydroxyl, $C_1$-$C_4$-alkyl, $C_1$-$C_4$-alkoxy, $C_1$-$C_4$-alkylthio, cyano, $C_1$-$C_4$-alkoxycarbonyl, mono- or di($C_1$-$C_4$-alkyl)amino, $-N(CH_2)_s$, $R_6$-substituted-phenyl, or an $R_6$-substituted-monocyclic heterocyclic group having from 3 to 7 ring atoms;
$R_6$ is hydrogen, halo, $C_1$-$C_4$-alkyl or $C_1$-$C_4$-alkoxy;
$R_8$ is $C_1$-$C_4$-alkyl, phenyl or benzyl;
$R_9$ is hydrogen, halo, hydroxyl, $C_1$-$C_4$-alkoxy, mono- or di($C_1$-$C_4$-alkyl)amino, $-N(CH_2)_s$ or phenyl
$R_{10}$ is hydroxy, cyano, $C_1$-$C_4$-alkoxycarbonyl, mono- or di($C_1$-$C_4$-alkyl)amino or $-N(CH_2)_s$;
Ar is phenyl; phenyl having one or more halo, $C_1$-$C_4$-alkyl, $C_1$-$C_4$-alkoxy or hydroxy substituents; or an $R_6$-substituted monocyclic aromatic heterocyclic group having from 5 to 7 ring atoms;
X is oxygen or sulfur;
Y is $-X(CH_2)_nCH_3$, $-N(CH_2)_s$ or $-N[(CH_2)_nCH_3]_2$;
l is 1 or 2;
m is an integer from 1 to 3;
n is an integer from 0 to 3;
p is an integer from 1 to 5;
q is 2 or 3;
r is 0 or 1, and
s is an integer from 2 to 7;
or a salt thereof, which comprises:
reacting a compound of formula (2a):

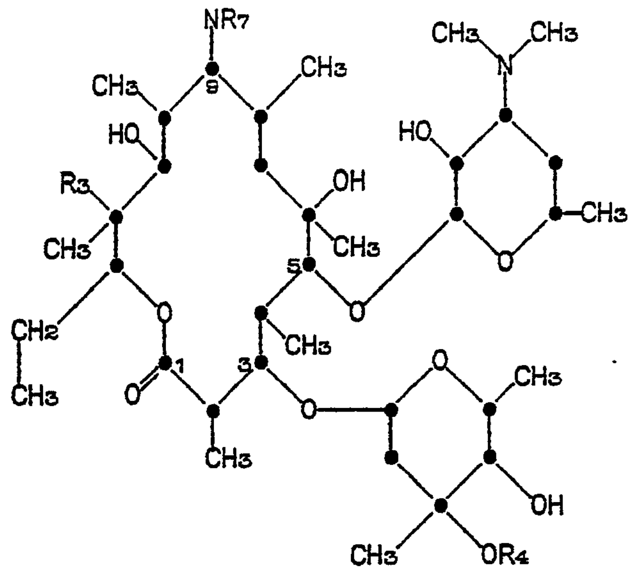

(2a)

in which $R_3$, $R_4$ and $R_7$ are as defined above, with formaldehyde.

9. A process for preparing a compound of formula (3):

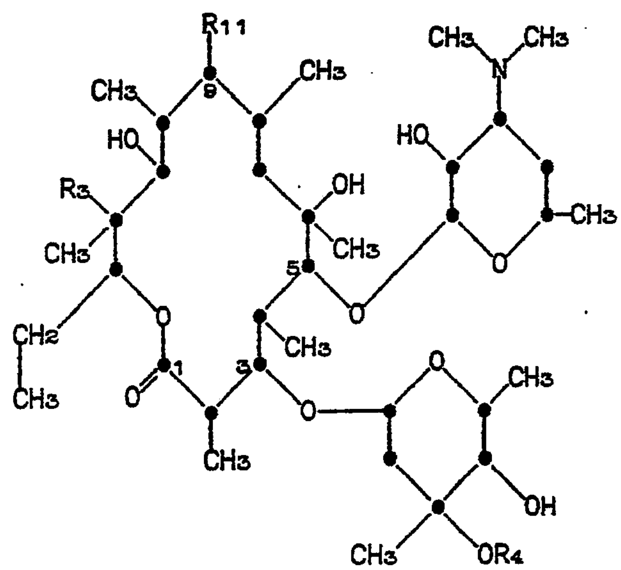

(3)

wherein $R_3$ is hydrogen or hydroxyl;
$R_4$ is hydrogen or methyl;
$R_{11}$ is $-N(CH_3)R_7$ or $-N(CH_2)_s$;
$R_7$ is $CH_2R_5$, $C_3$-$C_8$-cycloalkyl, $-CHR_8(CH_2)_pR_9$, $-(CH_2)_qR_{10}$ or $-CH_2(CH=CH)_rAr$;
$R_5$ is hydrogen or a $C_1$-$C_{14}$-alkyl or $-(CH_2)_lX(CH_2)_mY$ group, either of which group may have from one to three substituents selected from halo, hydroxyl, $C_1$-$C_4$-alkyl, $C_1$-$C_4$-alkoxy, $C_1$-$C_4$-alkylthio, cyano, $C_1$-$C_4$-alkoxycarbonyl, mono- or di($C_1$-$C_4$-alkyl)amino, $-N(CH_2)_s$, $R_6$-substituted-phenyl, or an $R_6$-substituted-monocyclic heterocyclic group having from 3 to 7 ring atoms;
$R_6$ is hydrogen, halo, $C_1$-$C_4$-alkyl or $C_1$-$C_4$-alkoxy;
$R_8$ is $C_1$-$C_4$-alkyl, phenyl or benzyl;

$R_9$ is hydrogen, halo, hydroxyl, $C_1$-$C_4$-alkoxy, mono- or di($C_1$-$C_4$-alkyl)amino, -$N(CH_2)_s$ or phenyl
$R_{10}$ is hydroxyl, cyano, $C_1$-$C_4$-alkoxycarbonyl, mono- or di($C_1$-$C_4$-alkyl)amino or -$N(CH_2)_s$;
Ar is phenyl; phenyl having one or more halo, $C_1$-$C_4$-alkyl, $C_1$-$C_4$-alkoxy or hydroxyl substituents; or an $R_6$-substituted monocyclic aromatic heterocyclic group having from 5 to 7 ring atoms;
X is oxygen or sulfur;
Y is -$X(CH_2)_nCH_3$, -$N(CH_2)_s$ or -$N[(CH_2)_nCH_3]_2$;
l is 1 or 2;
m is an integer from 1 to 3;
n is an integer from 0 to 3;
p is an integer from 1 to 5;
q is 2 or 3;
r is 0 or 1; and
s is an integer from 2 to 7;
or a salt thereof which comprises:
(a) reducing, by catalytic hydrogenation or chemical reduction at a pH of 4 to 6, a compound of formula (2) as defined in claim 7 to produce a compound of formula (3) in which $R_{11}$ is a -$N(CH_3)R_7$ and, if desired, salifying the product; or
(b) reacting an erythromycylamine with a dialdehyde of the formula $OHC-(CH_2)_b-CHO$ where b is an integer from 1 to 5, followed by reducing the product, by catalytic hydrogenation at a pH of 4 to 6; and if desired, salifying the product.

10. A process according to claim 9 in which the catalyctic hydrogenation steps are carried out at a temperature of from 80°C to 150°C.

11. A process for preparing a compound of formula (1) as defined in claim 1, or a salt thereof, which comprises reacting an erythromycylamine with an aliphatic aldehyde and reducing, in situ, by catalyctic hydrogenation or chemical reduction at a pH of 4 to 6, and if desired salifying the product.

12. A process according to claim 11 for preparing a compound as claimed in claim 1 wherein $R_5$ is $C_1$-$C_5$-alkyl or substituted $C_1$-$C_5$-alkyl.

13. A process according to claim 11 for preparing a compound as claimed in claim 1 wherein $R_5$ is $C_6$-$C_{14}$-alkyl or substituted $C_1$-$C_{14}$-alkyl.

14. A process according to claim 11 for preparing a compound as claimed in claim 1 wherein $R_5$ is -$(CH_2)_lO(CH_2)_mO(CH_2)_nCH_3$.

15. A process for preparing a compound of formula (3) as defined in claim 9, or a salt thereof, which comprises reacting a compound of formula (2a) as defined in claim 8 with formaldehyde and reducing, in situ, by catalytic hydrogenation or chemical reduction at a pH of 4 to 6, to produce a compound of formula (3) in which $R_{11}$ is a -$N(CH_3)R_7$, and if desired salifying the product.

16. Use of a compound as defined in claim 1 for the manufacture of a medicament for the treatment of infections caused by Gram-positive bacteria.

**Patentansprüche für die Vertragsstaaten: BE, CH, DE, FR, GB, IT, LI, LU, NL, SE**

1. Verbindung der Formel (1)

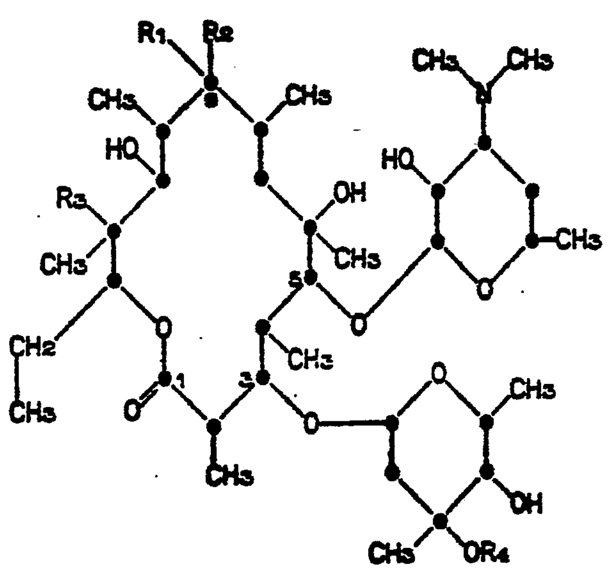

(1)

worin
$R_1$ und $R_2$ unterschiedlich sind und Wasserstoff oder $-NHCH_2R_5$ bedeuten,
$R_3$ Wasserstoff oder Hydroxyl ist,
$R_4$ Wasserstoff oder Methyl darstellt, und
$R_5$ Wasserstoff oder eine $C_1$–$C_{14}$-Alkylgruppe oder eine Gruppe $-(CH_2)_lX(CH_2)_mY$ bedeutet, wobei jede dieser Gruppen ein bis drei Substituenten enthalten kann, die ausgewählt sind aus Halogen, Hydroxyl, $C_1$–$C_4$-Alkyl, $C_1$–$C_4$-Alkoxy, $C_1$–$C_4$-Alkylthio, Cyano, $C_1$–$C_4$-Alkoxycarbonyl, Mono($C_1$–$C_4$-alkyl)amino, Di($C_1$–$C_4$-alkyl)amino, $-N(CH_2)_s$, durch $R_6$ substituiertem Phenyl oder einer durch $R_6$ substituierten monocyclischen heterocyclischen Gruppe mit 3 bis 7 Ringatomen,
X Sauerstoff oder Schwefel ist,
Y für $-X(CH_2)_nCH_3$, $-N(CH_2)_s$ oder $N[(CH_2)_nCH_3]_2$ steht,
l für 1 oder 2 steht,
m eine Zahl von 1 bis 3 bedeutet,
n eine Zahl von 0 bis 3 ist,
S eine Zahl von 2 bis 7 darstellt, und
$R_6$ Wasserstoff, Halogen, $C_1$–$C_4$-Alkyl oder $C_1$–$C_4$-Alkoxy bedeutet, mit der Maßgabe, daß der Substituent an der Gruppe $R_5$, falls dieser aus Hydroxyl, Cyano, Alkoxycarbonyl, Monoalkylamino, Dialkylamino oder $-N(CH_2)_s$ ausgewählt ist, nicht am zweiten oder dritten Kohlenstoffatom vom Stickstoffatom der Gruppe $-NHCH_2R_5$ angeordnet sein kann, es sei denn, das zweite Kohlenstoffatom ist quaternär, oder ein Salz hiervon.

2. Verbindung nach Anspruch 1, worin $R_5$ Wasserstoff, $C_1$–$C_{14}$-Alkyl oder substituiertes $C_1$–$C_{14}$-Alkyl ist.

3. Verbindung nach Anspruch 2, worin $R_5$ für $C_1$–$C_5$-Alkyl oder substituiertes $C_1$–$C_5$-Alkyl steht.

4. Verbindung nach Anspruch 2, worin $R_5$ für $C_6$–$C_{14}$-Alkyl oder substituiertes $C_6$–$C_{14}$-Alkyl steht.

5. Verbindung nach Anspruch 1, worin $R_5$ eine Gruppe
$-(CH_2)_lX(CH_2)_mY$ oder eine substituierte Gruppe
$-(CH_2)_lX(CH_2)_mY$ ist.

6. Verbindung nach Anspruch 5, worin X Sauerstoff ist und Y für $-O(CH_2)_nCH_3$ steht.

7. Verbindung der Formel (2)

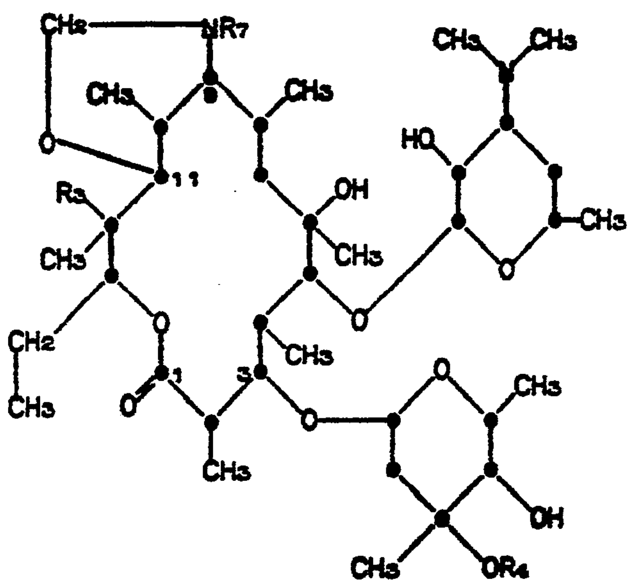

(2)

worin
$R_3$ Wasserstoff oder Hydroxyl ist,
$R_4$ Wasserstoff oder Methyl bedeutet,
$R_7$ für $CH_2R_5$, $C_3$–$C_8$-Cycloalkyl, $-CHR_8(CH_2)_pR_9$, $-(CH_2)_qR_{10}$ oder $-CH_2(CH{=}CH)_rAr$ steht,
$R_5$ Wasserstoff oder eine $C_1$–$C_{14}$-Alkylgruppe oder eine Gruppe $-(CH_2)_lX(CH_2)_mY$ bedeutet, wobei jede dieser Gruppen ein bis drei Substituenten enthalten kann, die ausgewählt sind aus Halogen, Hydroxyl, $C_1$–$C_4$-Alkyl, $C_1$–$C_4$-Alkoxy, $C_1$–$C_4$-Alkylthio, Cyano, $C_1$–$C_4$-Alkoxycarbonyl, Mono($C_1$–$C_4$-alkyl)amino, Di($C_1$–$C_4$-alkyl)amino, $-N(CH_2)_s$, durch $R_6$ substituiertes Phenyl oder einer durch $R_6$ substituierten monocyclischen heterocyclischen Gruppe mit 3 bis 7 Ringatomen,
$R_6$ Wasserstoff, Halogen, $C_1$–$C_4$-Alkyl oder $C_1$–$C_4$-Alkoxy bedeutet,
$R_8$ für $C_1$–$C_4$-Alkyl, Phenyl oder Benzyl steht,

$R_9$ Wasserstoff, Halogen, Hydroxyl, $C_1$–$C_4$-Alkoxy, Mono($C_1$–$C_4$-alkyl)amino, Di($C_1$–$C_4$-alkyl)amino, $-N(CH_2)_s$ oder Phenyl ist,
$R_{10}$ Hydroxy, Cyano, $C_1$–$C_4$-Alkoxycarbonyl, Mono($C_1$–$C_4$-alkyl) amino, Di($C_1$–$C_4$-alkyl)amino oder $-N(CH_2)_S$ bedeutet,
Ar Phenyl, einfach oder mehrfach durch Halogen, $C_1$–$C_4$-Alkyl, $C_1$–$C_4$-Alkoxy oder Hydroxy substituiertes Phenyl oder eine $R_6$-substituierte monocyclische aromatische heterocyclische Gruppe mit 5 bis 7 Ringatomen bedeutet,
X Sauerstoff oder Schwefel ist,
Y für $-X(CH_2)_nCH_3$, $-N(CH_2)_s$ oder $-N[(CH_2)_nCH_3]_2$ steht,
l für 1 oder 2 steht,
m eine Zahl von 1 bis 3 ist,
n eine Zahl von 0 bis 3 bedeutet,
p eine Zahl von 1 bis 5 ist,
q für 2 oder 3 steht,
r für 0 oder 1 steht, und
s eine Zahl von 2 bis 7 ist,
oder ein Salz hiervon.

8. Verbindung der Formel (3)

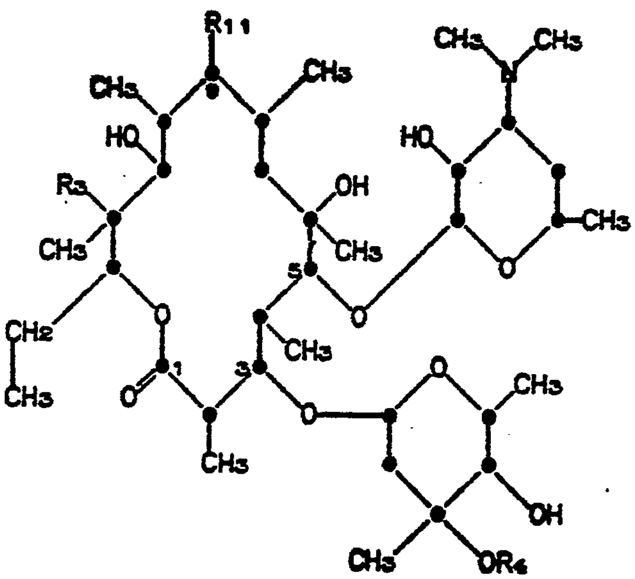

(3)

worin
$R_3$ Wasserstoff oder Hydroxyl ist,
$R_4$ Wasserstoff oder Methyl bedeutet,
$R_{11}$ für $-N(CH_3)R_7$ oder $-N(CH_2)_s$ steht,
$R_7$ für $CH_2R_5$, $C_3$–$C_8$-Cycloakyl, $-CHR_8(CH_2)_pR_9$, $-(CH_2)_qR_{10}$ oder $-CH_2(CH{=}CH)_rAr$ steht,
$R_5$ Wasserstoff oder eine $C_1$–$C_{14}$-Alkylgruppe oder eine Gruppe $-(CH_2)_lX(CH_2)_mY$ bedeutet, wobei jede dieser Gruppen ein bis drei Substituenten enthalten kann, die ausgewählt sind aus Halogen, Hydroxyl, $C_1$–$C_4$-Alkyl, $C_1$–$C_4$-Alkoxy, $C_1$–$C_4$-Alkylthio, Cyano, $C_1$–$C_4$-Alkoxycarbonyl, Mono($C_1$–$C_4$-alkyl)amino, Di($C_1$–$C_4$-alkyl)amino, $-N(CH_2)_s$, durch $R_6$ substituiertem Phenyl oder einer durch $R_6$ substituierten monocyclischen heterocyclischen Gruppe mit 3 bis 7 Ringatomen,
$R_6$ Wasserstoff, Halogen, $C_1$–$C_4$-Alkyl oder $C_1$–$C_4$-Alkoxy bedeutet,
$R_8$ für $C_1$–$C_4$-Alkyl, Phenyl oder Benzyl steht,
$R_9$ Wasserstoff, Halogen, Hydroxyl, $C_1$–$C_4$-Alkoxy, Mono($C_1$-$C_4$-alkyl)amino, Di($C_1$–$C_4$-alkyl)amino, $-N(CH_2)_s$ oder Phenyl ist,
$R_{10}$ Hydroxy, Cyano, $C_1$–$C_4$-Alkoxycarbonyl, Mono($C_1$–$C_4$-alkyl)amino, Di($C_1$–$C_4$-alkyl)amino oder $-N(CH_2)_s$ bedeutet,
Ar Phenyl, einfach oder mehrfach durch Halogen, $C_1$–$C_4$-Alkyl, $C_1$–$C_4$-Alkoxy oder Hydroxy substituiertes Phenyl oder eine $R_6$-substituierte monocyclische aromatische heterocyclische Gruppe mit 5 bis 7 Ringatomen bedeutet,
X Sauerstoff oder Schwefel ist,
Y für $-X(CH_2)_nCH_3$, $-N(CH_2)_s$ oder $-N[(CH_2)_nCH_3]_2$ steht,
l für 1 oder 2 steht,

m eine Zahl von 1 bis 3 ist,
n eine Zahl von 0 bis 3 bedeutet,
p eine Zahl von 1 bis 5 ist,
q für 2 oder 3 steht,
r für 0 oder 1 steht, und
s eine Zahl von 2 bis 7 ist,
oder ein Salz hiervon.

9. Pharmazeutische Formulierung, dadurch gekennzeichnet, daß sie eine Verbindung der Formel (1), (2) oder (3) oder ein pharmazeutisch annehmbares Salz hiervon gemäß Definition der Ansprüche 1, 7 oder 8 als Wirkstoff in Kombination mit einem oder mehreren pharmazeutisch annehmbaren Trägern, Verdünnungsmitteln oder Hilfsstoffen hierfür enthält.

10. Verbindung der Formel (1), (2) oder (3) oder ein pharmazeutisch annehmbares Salz hiervon gemäß Definition nach Anspruch 1, 7 oder 8 zur Verwendung bei der Behandlung, Verhinderung oder Bekämpfung von Infektionskrankheiten.

11. 9-Deoxo-9-(n-propylamino)erythromycin.

**Patentansprüche für den Vertragsstaat: GR**

1. Verfahren zur Herstellung einer Verbindung der Formel (1)

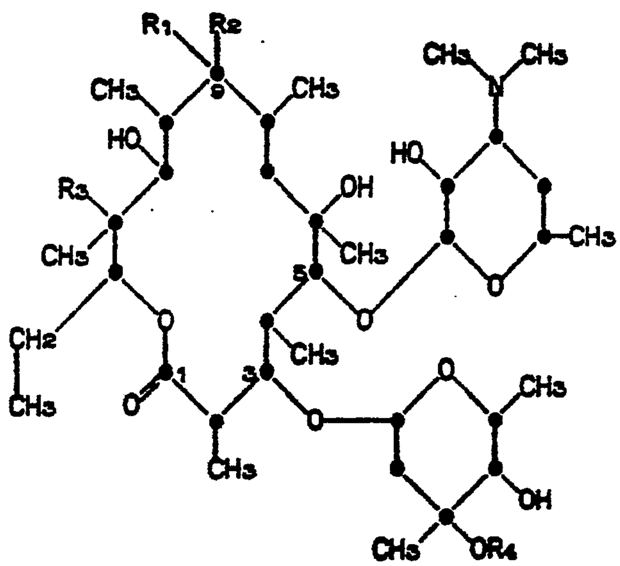

(1)

worin
$R_1$ und $R_2$ unterschiedlich sind und Wasserstoff oder $-NHCH_2R_5$ bedeuten,
$R_3$ Wasserstoff oder Hydroxyl ist,
$R_4$ Wasserstoff oder Methyl darstellt, und
$R_5$ Wasserstoff oder eine $C_1$–$C_{14}$-Alkylgruppe oder eine Gruppe $-(CH_2)_lX(CH_2)_mY$ bedeutet, wobei jede dieser Gruppen ein bis drei Substituenten enthalten kann, die ausgewählt sind aus Halogen, Hydroxyl, $C_1$–$C_4$-Alkyl, $C_1$–$C_4$-Alkoxy, $C_1$–$C_4$-Alkylthio, Cyano, $C_1$–$C_4$-Alkoxycarbonyl, Mono($C_1$–$C_4$-alkyl)amino, Di($C_1$–$C_4$-alkyl)amino $-N(CH_2)_s$, durch $R_6$ substituiertem Phenyl oder einer durch $R_6$ substituierten monocyclischen heterocyclischen Gruppe mit 3 bis 7 Ringatomen,
X Sauerstoff oder Schwefel ist,
Y für $-X(CH_2)_nCH_3$, $-N(CH_2)_s$ oder $-N[(CH_2)_nCH_3]_2$ steht,
l für 1 oder 2 steht,
m eine Zahl von 1 bis 3 bedeutet,
n eine Zahl von 0 bis 3 ist,
s eine Zahl von 2 bis 7 darstellt, und
$R_6$ Wasserstoff, Halogen, $C_1$–$C_4$-Alkyl oder $C_1$–$C_4$-Alkoxy bedeutet,
mit der Maßgabe, daß der Substituent an der Gruppe $R_5$, falls dieser aus Hydroxyl, Cyano, Alkoxycarbonyl, Monoalkylamino, Dialkylamino oder $-N(CH_2)_s$ ausgewählt ist, nicht am zweiten oder dritten Kohlenstoffatom vom Stickstoffatom der Gruppe $-NHCH_2R_5$ angeordnet sein kann, es sei denn, das zweite Kohlenstoffatom ist quaternär, oder eines Salzes hiervon,
dadurch gekennzeichnet, daß eine Verbindung der Formel (1a)

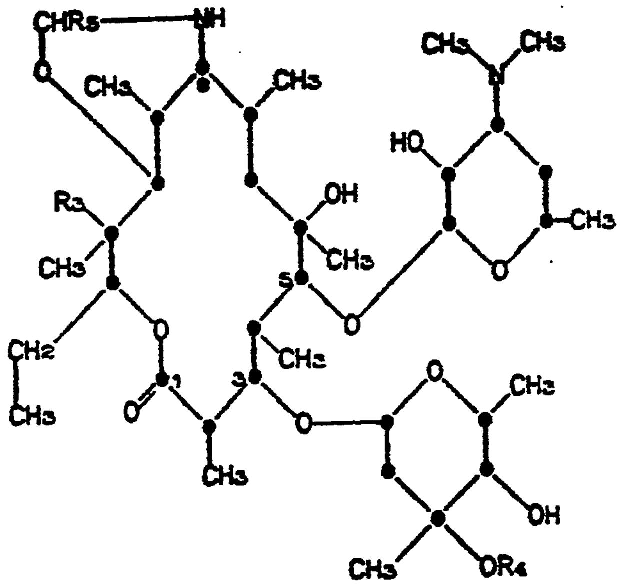

(1a)

durch katalytische Hydrierung oder durch chemische Reduktion bei einem pH-Wert von 4 bis 6 reduziert wird und das erhaltene Produkt gewünschtenfalls in ein Salz überführt wird.

2. Verfahren nach Anspruch 1, worin die katalytische Hydrierung bei einer Temperatur von 80°C bis 150°C durchgeführt wird.

3. Verfahren nach Anspruch 1 oder 2 zur Herstellung einer Verbindung nach Anspruch 1, worin $R_5$ Wasserstoff, $C_1$–$C_{14}$-Alkyl oder substituiertes $C_1$–$C_4$-Alkyl ist.

4. Verfahren zur Herstellung einer Verbindung nach Anspruch 3, worin $R_5$ für $C_1$–$C_5$-Alkyl oder substituiertes $C_1$–$C_5$-Alkyl steht.

5. Verfahren zur Herstellung einer Verbindung nach Anspruch 3, worin $R_5$ für $C_6$–$C_{14}$-Alkyl oder substituiertes $C_6$–$C_{14}$-Alkyl steht.

6. Verfahren nach einem der Ansprüche 1 und 2 zur Herstellung einer Verbindung nach Anspruch 1, worin $R_5$ eine Gruppe
$-(CH_2)_lX(CR_2)_mY$ oder eine substituierte Gruppe
$-(CH_2)_lX(CH_2)_mY$ ist ist.

7. Verfahren zur Herstellung einer Verbindung nach Anspruch 6, worin X Sauerstoff ist und Y für $-O(CH_2)_nCH_3$ steht.

8. Verfahren zur Herstellung einer Verbindung der Formel (2)

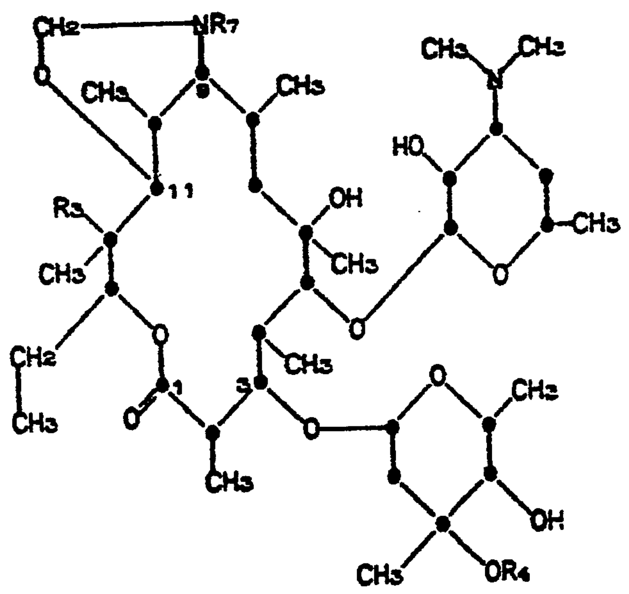

(2)

worin
$R_3$ Wasserstoff oder Hydroxyl ist,

$R_4$ Wasserstoff oder Methyl bedeutet,
$R_7$ für $CH_2R_5$, $C_3$–$C_8$-Cycloalkyl, $-CHR_8(CH_2)_pR_9$, $-(CH_2)_qR_{10}$ oder $-CH_2(CH=CH)_rAr$ steht,
$R_5$ Wasserstoff oder eine $C_1$–$C_{14}$-Alkylgruppe oder eine Gruppe $-(CH_2)_lX(CH_9)_mY$ bedeutet, wobei jede dieser Gruppen ein bis drei Substituenten enthalten kann, die ausgewählt sind aus Halogen, Hydroxyl, $C_1$–$C_4$-Alkyl, $C_1$–$C_4$-Alkoxy, $C_1$–$C_4$-Alkylthio, Cyano, $C_1$–$C_4$-Alkoxycarbonyl, Mono($C_1$–$C_4$-alkyl)amino, Di($C_1$–$C_4$-alkyl)amino, $-N(CH_2)_s$, durch $R_6$ substituiertem Phenyl oder einer durch $R_6$ substituierten monocyclischen heterocyclischen Gruppe mit 3 bis 7 Ringatomen,
$R_6$ Wasserstoff, Halogen, $C_1$–$C_4$-Alkyl oder $C_1$–$C_4$-Alkoxy bedeutet,
$R_8$ für $C_1$–$C_4$-Alkyl, Phenyl oder Benzyl steht,
$R_9$ Wasserstoff, Halogen, Hydroxyl, $C_1$–$C_4$-Alkoxy, Mono($C_1$–$C_4$-alkyl)amino, Di($C_1$–$C_4$-alkyl)amino, $-N(CH_2)_s$ oder Phenyl ist,
$R_{10}$ Hydroxy, Cyano, $C_1$–$C_4$-Alkoxycarbonyl, Mono($C_1$–$C_4$-alkyl)amino, Di($C_1$–$C_4$-alkyl)amino oder $-N(CH_2)_s$ bedeutet,
Ar Phenyl, einfach oder mehrfach durch Halogen, $C_1$–$C_4$-Alkyl, $C_1$–$C_4$-Alkoxy oder Hydroxy substituiertes Phenyl oder eine $R_6$-substituierte monocyclische aromatische heterocyclische Gruppe mit 5 bis 7 Ringatomen bedeutet,
X Sauerstoff oder Schwefel ist,
Y für $-X(CH_2)_nCH_3$, $-N(CH_2)_s$ oder $-N[(CH_2)_nCH_3]_2$ steht,
l für 1 oder 2 steht,
m eine Zahl von 1 bis 3 ist,
n eine Zahl von 0 bis 3 bedeutet,
p eine Zahl von 1 bis 5 ist,
q für 2 oder 3 steht,
r für 0 oder 1 steht,und
s eine Zahl von 2 bis 7 ist,
oder eines Salzes hiervon, dadurch gekennzeichnet, daß eine Verbindung der Formel (2a)

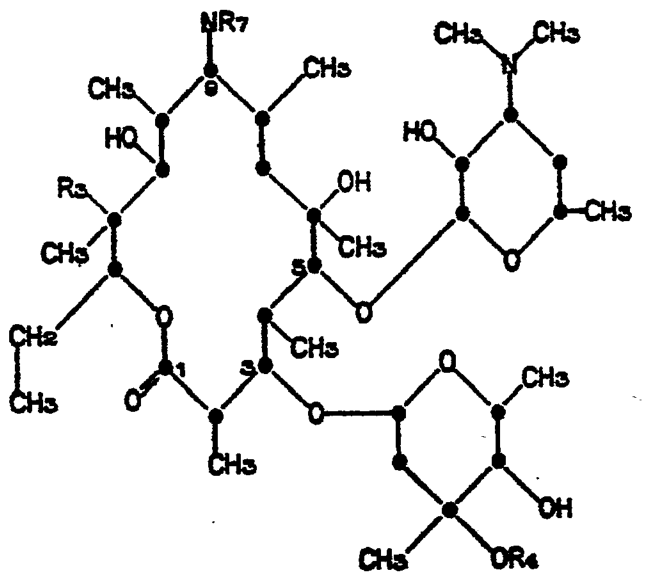

(2a)

worin $R_3$, $R_4$ und $R_7$ wie oben definiert sind, mit Formaldehyd umgesetzt wird.

9. Verfahren zur Herstellung einer Verbindung der Formel (3)

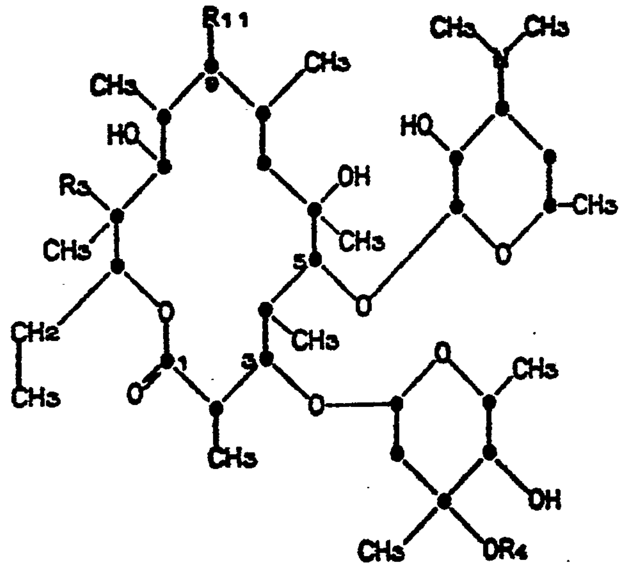

(3)

worin

$R_3$ Wasserstoff oder Hydroxyl ist,

$R_4$ Wasserstoff oder Methyl bedeutet,

$R_{11}$ für $-N(CH_3)R_7$ oder $-N(CH_2)_s$ steht,

$R_7$ für $CH_2R_5$, $C_3$–$C_8$-Cycloalkyl, $-CHR_8(CH_2)_pR_9$, $-(CH_2)_qR_{10}$ oder $-CH_2(CH{=}CH)_rAr$ steht,

$R_5$ Wasserstoff oder eine $C_1$–$C_{14}$-Alkylgruppe oder eine Gruppe $-(CH_2)_lX(CH_2)_mY$ bedeutet, wobei jede dieser Gruppen ein bis drei Substituenten enthalten kann, die ausgewählt sind aus Halogen, Hydroxyl, $C_1$–$C_4$-Alkyl, $C_1$–$C_4$-Alkoxy, $C_1$–$C_4$-Alkylthio, Cyano, $C_1$–$C_4$-Alkoxycarbonyl, Mono($C_1$–$C_4$-alkyl)amino, Di($C_1$–$C_4$-alkyl)amino, $-N(CH_2)_s$, durch $R_6$ substituiertem Phenyl oder einer durch $R_6$ substituierten monocyclischen heterocyclischen Gruppe mit 3 bis 7 Ringatomen,

$R_6$ Wasserstoff, Halogen, $C_1$–$C_4$-Alkyl oder $C_1$–$C_4$-Alkoxy bedeutet,

$R_8$ für $C_1$–$C_4$-Alkyl, Phenyl oder Benzyl steht,

$R_9$ Wasserstoff, Halogen, Hydroxyl, $C_1$–$C_4$-Alkoxy, Mono($C_1$–$C_4$-alkyl)amino, Di($C_1$–$C_4$-alkyl)amino, $-N(CH_2)_s$ oder Phenyl ist,

$R_{10}$ Hydroxy, Cyano, $C_1$–$C_4$-Alkoxycarbonyl, Mono($C_1$–$C_4$-alkyl)amino, Di($C_1$–$C_4$-alkyl)amino oder $-N(CH_2)_s$ bedeutet,

Ar Phenyl, einfach oder mehrfach durch Halogen, $C_1$–$C_4$-Alkyl, $C_1$–$C_4$-Alkoxy oder Hydroxy substituiertes Phenyl oder eine $R_6$-substituierte monocyclische aromatische heterocyclische Gruppe mit 5 bis 7 Ringatomen bedeutet,

X Sauerstoff oder Schwefel ist,

Y für $-X(CH_2)_nCH_3$, $-N(CH_2)_s$ oder $-N[(CH_2)_nCH_3]_2$ steht,

l für 1 oder 2 steht,

m eine Zahl von 1 bis 3 ist,

n eine Zahl von 0 bis 3 bedeutet,

p eine Zahl von 1 bis 5 ist,

q für 2 oder 3 steht,

r für 0 oder 1 steht, und

s eine Zahl von 2 bis 7 ist,

oder eines Salzes hiervon, dadurch gekennzeichnet, daß

(a) eine Verbindung der Formel (2) wie in Anspruch 7 definiert durch katalytische Hydrierung oder durch chemische Reduktion bei einem pH-Wert von 4 bis 6 zu einer Verbindung der Formel (3) reduziert wird, worin $R_{11}$ für $-N(CH_3)R_7$ steht, und das erhaltene Produkt gewünschtenfalls in ein Salz überführt wird, oder

(b) ein Erythromycylamin mit einem Dialdehyd der Formel $OHC-(CH_2)_b-CHO$, worin b eine ganze Zahl von 1 bis 5 ist, umgesetzt wird, das erhaltene Produkt durch katalytische Hydrierung oder durch chemische Reduktion bei einem pH-Wert von 4 bis 6 reduziert wird und das erhaltene Produkt gewünschtenfalls in ein Salz überführt wird.

10. Verfahren nach Anspruch 9, worin die Stufen der katalytischen Hydrierung bei einer Temperatur von 80°C bis 150°C durchgeführt werden.

11. Verfahren zur Herstellung einer Verbindung der Formel (1) gemäß Definition von Anspruch 1 oder eines Salzes hiervon, dadurch gekennzeichnet, daß ein Erythromycylamin mit einem aliphatischen Aldehyd umgesetzt und in situ durch katalytische Hydrierung oder chemische Reduktion bei einem pH-Wert von 4 bis 6 reduziert wird, und das erhaltene Produkt gewünschtenfalls in ein Salz überführt wird.

12. Verfahren nach Anspruch 11 zur Herstellung einer Verbindung gemäß Anspruch 1, worin $R_5$ für $C_1$–$C_5$-Alkyl oder substituiertes $C_1$–$C_5$-Alkyl steht.

13. Verfahren nach Anspruch 11 zur Herstellung einer Verbindung gemäß Anspruch 1, worin $R_5$ für $C_6$–$C_{14}$-Alkyl oder substituiertes $C_6$–$C_{14}$-Alkyl steht.

14. Verfahren nach Anspruch 11 zur Herstellung einer Verbindung gemäß Anspruch 1, worin $R_5$ für $-(CH_2)_lO(CH_2)_mO(CH_2)_nCH_3$ steht.

15. Verfahren zur Herstellung einer Verbindung der Formel (3) gemäß Definition von Anspruch 9 oder eines Salzes hiervon, dadurch gekennzeichnet, daß eine Verbindung der Formel (2a) gemäß Definition von Anspruch 8 mit Formaldehyd umgesetzt und in situ durch katalytische Hydrierung oder chemische Reduktion bei einem pH-Wert von 4 bis 6 unter Bildung einer Verbindung der Formel (3), worin $R_{11}$ für $-N(CH_3)R_7$ steht, reduziert wird und das erhaltene Produkt gewünschtenfalls in ein Salz überführt wird.

16. Verbindung der Formel (1a) gemäß Definition von Anspruch 1 oder ein Salz hiervon.

17. Verbindung der Formel (2a) gemäß Definition von Anspruch 8 oder ein Salz hiervon.

18. Verbindung der Formel (1) gemäß Definition von Anspruch 1 oder ein Salz hiervon.

19. Verbindung der Formel (2) gemäß Definition von Anspruch 8 oder ein Salz hiervon.

**Patentansprüche für die Vertragsstaaten: AT, ES**

1. Verfahren zur Herstellung einer Verbindung der Formel (1)

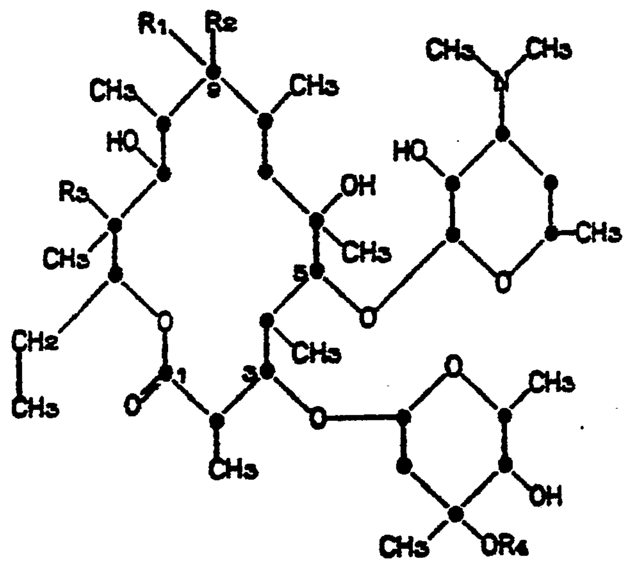

(1)

worin
$R_1$ und $R_2$ unterschiedlich sind und Wasserstoff oder $-NHCH_2R_5$ bedeuten,
$R_3$ Wasserstoff oder Hydroxyl ist,
$R_4$ Wasserstoff oder Methyl darstellt, und
$R_5$ Wasserstoff oder eine $C_1$–$C_{14}$-Alkylgruppe oder eine Gruppe $-(CH_2)_lX(CH_2)_mY$ bedeutet, wobei jede dieser Gruppen ein bis drei Substituenten enthalten kann, die ausgewählt sind aus Halogen, Hydroxyl, $C_1$–$C_4$-Alkyl, $C_1$–$C_4$-Alkoxy, $C_1$–$C_4$-Alkylthio, Cyano, $C_1$–$C_4$-Alkoxycarbonyl, Mono($C_1$–$C_4$-alkyl)amino, Di($C_1$–$C_4$-alkyl)amino, $-N(CH_2)_s$, durch $R_6$ substituiertem Phenyl oder einer durch $R_6$ substituierten monocyclischen heterocyclischen Gruppe mit 3 bis 7 Ringatomen,
X Sauerstoff oder Schwefel ist,
Y für $-X(CH_2)_nCH_3$, $-N(CH_2)_s$ oder $-N[(CH_2)_nCH3]_2$ steht,
l für 1 oder 2 steht,
m eine Zahl von 1 bis 3 bedeutet,
n eine Zahl von 0 bis 3 ist,
s eine Zahl von 2 bis 7 darstellt, und
$R_6$ Wasserstoff, Halogen, $C_1$–$C_4$-Alkyl oder $C_1$–$C_4$-Alkoxy bedeutet,
mit der Maßgabe, daß der Substituent an der Gruppe $R_5$, falls dieser aus Hydroxyl, Cyano, Alkoxycarbonyl, Monoalkylamino, Dialkylamino oder $-N(CH_2)_s$ ausgewählt ist, nicht am zweiten oder dritten Kohlenstoffatom vom Stickstoffatom der Gruppe $-NHCH_2R_5$ angeordnet sein kann, es sei denn, das zweite Kohlenstoffatom ist quaternär, oder eines Salzes hiervon, dadurch gekennzeichnet, daß eine Verbindung der Formel (1a)

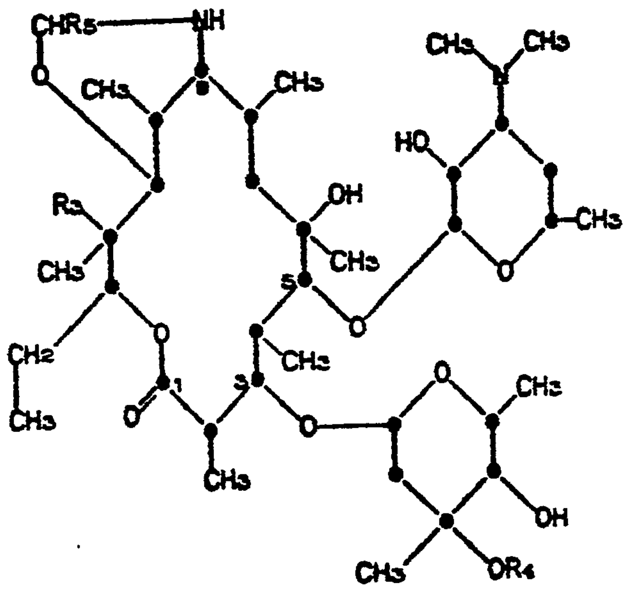

(1a)

durch katalytische Hydrierung oder durch chemische Reduktion bei einem pH-Wert von 4 bis 6 reduziert wird und das erhaltene Produkt gewünschtenfalls in ein Salz überführt wird.

2. Verfahren nach Anspruch 1, worin die katalytische Hydrierung bei einer Temperatur von 80°C bis 150°C durchgeführt wird.

3. Verfahren nach Anspruch 1 oder 2 zur Herstellung einer Verbindung nach Anspruch 1, worin $R_5$ Wasserstoff, $C_1$–$C_{14}$-Alkyl oder substituiertes $C_1$–$C_4$-Alkyl ist.

4. Verfahren zur Herstellung einer Verbindung nach Anspruch 3, worin $R_5$ für $C_1$–$C_5$-Alkyl oder substituiertes $C_1$-$C_5$-Alkyl steht.

5. Verfahren zur Herstellung einer Verbindung nach Anspruch 3, worin $R_5$ für $C_6$–$C_{14}$-Alkyl oder substituiertes $C_6$–$C_{14}$-Alkyl steht.

6. Verfahren nach einem der Ansprüche 1 und 2 zur Herstellung einer Verbindung nach Anspruch 1, worin $R_5$ eine Gruppe
$-(CH_2)_lX(CH_2)_mY$ oder eine substituierte Gruppe
$-(CR_2)_lX(CH_2)_mY$ ist.

7. Verfahren zur Herstellung einer Verbindung nach nspruch 6, worin X Sauerstoff ist und Y für $-O(CH_2)_nCH_3$ steht.

8. Verfahren zur Herstellung einer Verbindung der Formel (2)

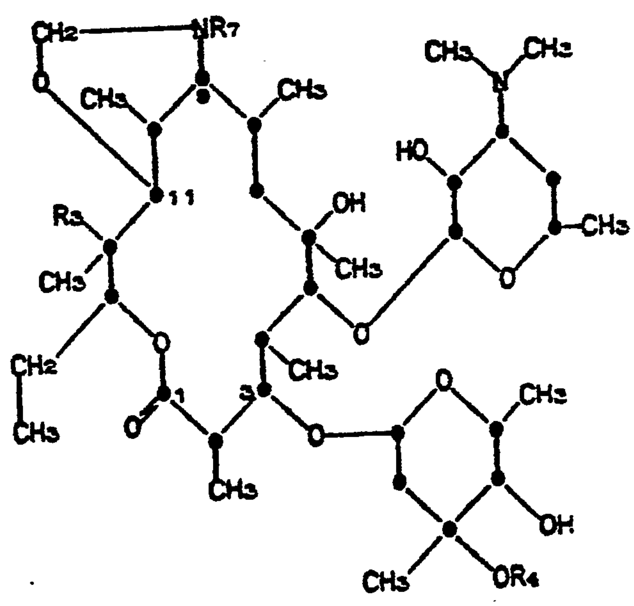

(2)

worin
$R_3$ Wasserstoff oder Hydroxyl ist,
$R_4$ Wasserstoff oder Methyl bedeutet,
$R_7$ für $CH_2R_5$, $C_3$–$C_8$-Cycloalkyl, $-CHR_8(CH_2)_pR_9$,$-(CH_2)_qR_{10}$ oder $-CH_2(CH=CH)_rAr$ steht,
$R_5$ Wasserstoff oder eine $C_1$–$C_{14}$-Alkylgruppe oder eine Gruppe $-(CH_2)_lX(CH_2)_mY$ bedeutet, wobei jede dieser Gruppen ein bis drei Substituenten enthalten kann, die ausgewählt sind aus Halogen, Hydroxyl, $C_1$–$C_4$-Alkyl, $C_1$–$C_4$-Alkoxy, $C_1$–$C_4$-Alkylthio, Cyano, $C_1$–$C_4$-Alkoxycarbonyl, Mono($C_1$–$C_4$-alkyl)amino, Di($C_1$–$C_4$-alkyl)amino, $-N(CH_2)_s$, durch $R_6$ substituiertem Phenyl oder einer durch $R_6$ substituierten monocyclischen heterocyclischen Gruppe mit 3 bis 7 Ringatomen,
$R_6$ Wasserstoff, Halogen, $C_1$–$C_4$-Alkyl oder $C_1$–$C_4$-Alkoxy bedeutet,
$R_8$ für $C_1$–$C_4$-Alkyl, Phenyl oder Benzyl steht,
$R_9$ Wasserstoff, Halogen, Hydroxyl, $C_1$–$C_4$-Alkoxy, Mono($C_1$–$C_4$-alkyl)amino, Di($C_1$–$C_4$-alkyl)amino, $-N(CH_2)_s$ oder Phenyl ist,
$R_{10}$ Hydroxy, Cyano, $C_1$–$C_4$-Alkoxycarbonyl, Mono($C_1$–$C_4$-alkyl)amino, Di($C_1$–$C_4$-alkyl)amino oder $-N(CH_2)_s$ bedeutet
Ar Phenyl, einfach oder mehrfach durch Halogen, $C_1$–$C_4$-Alkyl, $C_1$–$C_4$-Alkoxy oder Hydroxy substituiertes Phenyl oder eine $R_6$-substituierte monocyclische aromatische heterocyclische Gruppe mit 5 bis 7 Ringatomen bedeutet,
X Sauerstoff oder Schwefel ist,
Y für $-X(CH_2)_nCH_3$, $-N(CH_2)_s$ oder $-N[(CH_2)_nCH_3]_2$ steht,
l für 1 oder 2 steht,
m eine Zahl von 1 bis 3 ist,
n eine Zahl von 0 bis 3 bedeutet,
p eine Zahl von 1 bis 5 ist,
q für 2 oder 3 steht,
r für 0 oder 1 steht, und
s eine Zahl von 2 bis 7 ist,
oder eines Salzes hiervon, dadurch gekennzeichnet, daß eine Verbindung der Formel (2a)

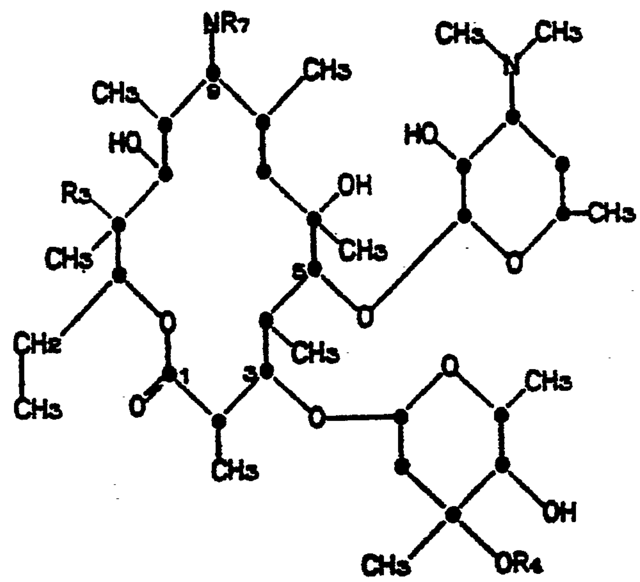

(2a)

worin $R_3$, $R_4$ und $R_7$ wie oben definiert sind, mit Formaldehyd umgesetzt wird.

9. Verfahren zur Herstellung einer Verbindung der Formel (3)

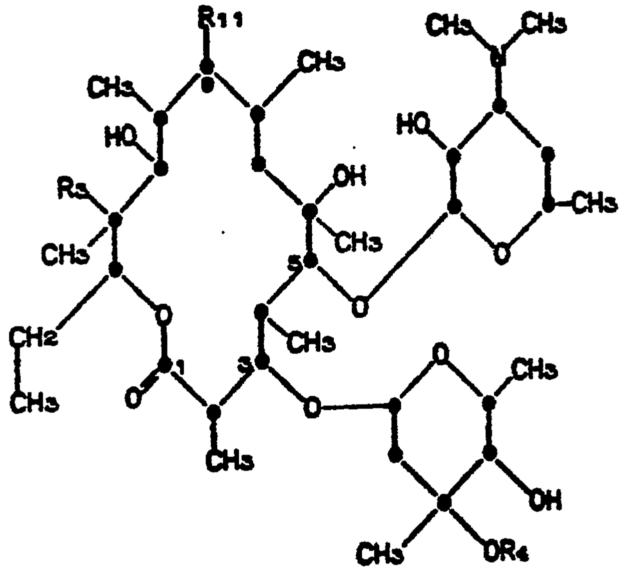

(3)

worin
$R_3$ Wasserstoff oder Hydroxyl ist,
$R_4$ Wasserstoff oder Methyl bedeutet,
$R_{11}$ für $-N(CH_3)R_7$ oder $-N(CH_2)_s$ steht,
$R_7$ für $CH_2R_5$, $C_3-C_8$-Cycloalkyl, $.CHR_8(CH_2)_pR_9$, $-(CH_2)_qR_{10}$ oder $-CH_2(CH=CH)_rAr$ steht,
$R_5$ Wasserstoff oder eine $C_1-C_{14}$-Alkylgruppe oder eine Gruppe $-(CH_2)_lX(CH_2)_mY$ bedeutet, wobei jede dieser Gruppen ein bis drei Substituenten enthalten kann, die ausgewählt sind aus Halogen, Hydroxyl, $C_1-C_4$-Alkyl, $C_1-C_4$-Alkoxy, $C_1-C_4$-Alkylthio, Cyano, $C_1-C_4$-Alkoxycarbonyl, Mono($C_1-C_4$-alkyl)amino, Di($C_1-C_4$-alkyl)amino, $-N(CH_2)_s$, durch $R_6$ substituiertem Phenyl oder einer durch $R_6$ substituierten monocyclischen heterocyclischen Gruppe mit 3 bis 7 Ringatomen,
$R_6$ Wasserstoff, Halogen, $C_1-C_4$-Alkyl oder $C_1-C_4$-Alkoxy bedeutet,
$R_8$ für $C_1-C_4$-Alkyl, Phenyl oder Benzyl steht,
$R_9$ Wasserstoff, Halogen, Hydroxyl, $C_1-C_4$-Alkoxy, Mono($C_1-C_4$-alkyl)amino, Di($C_1-C_4$-alkyl)amino, $-N(CH_2)_s$ oder Phenyl ist,
$R_{10}$ Hydroxy, Cyano, $C_1-C_4$-Alkoxycarbonyl, Mono($C_1-C_4$-alkyl)amino, Di($C_1-C_4$-alkyl)amino oder $-N(CH_2)_s$ bedeutet,
Ar Phenyl, einfach oder mehrfach durch Halogen, $C_1-C_4$-Alkyl, $C_1-C_4$-Alkoxy oder Hydroxy substituiertes Phenyl oder eine $R_6$-substituierte monocyclische aromatische heterocyclische Gruppe mit 5 bis 7 Ringatomen bedeutet,
X Sauerstoff oder Schwefel ist,
Y für $-X(CH_2)_nCH_3$, $-N(CH_2)_s$ oder $-N[(CH_2)_nCH_3]_2$ steht,
l für 1 oder 2 steht,
m eine Zahl von 1 bis 3 ist,
n eine Zahl von 0 bis 3 bedeutet,
p eine Zahl von 1 bis 5 ist,
q für 2 oder 3 steht,
r für 0 oder 1 steht, und
s eine Zahl von 2 bis 7 ist,
oder eines Salzes hiervon, dadurch gekennzeichnet, daß
(a) eine Verbindung der Formel (2) wie in Anspruch 7 definiert durch katalytische Hydrierung oder durch chemische Reduktion bei einem pH-Wert von 4 bis 6 zu einer Verbindung der Formel (3) reduziert wird, worin $R_{11}$ für $-N(CH_3)R_7$ steht, und das erhaltene Produkt gewünschtenfalls in ein Salz überführt wird, oder
(b) ein Erythromycylamin mit einem Dialdehyd der Formel $OHC-(CH_2)_b-CHO$, worin b eine ganze Zahl von 1 bis 5 ist, umgesetzt wird, das erhaltene Produkt durch katalytische Hydrierung oder durch chemische Reduktion bei einem pH-Wert von 4 bis 6 reduziert wird und das erhaltene Produkt gewünschtenfalls in ein Salz überführt wird.

10. Verfahren nach Anspruch 9, worin die Stufen der katalytischen Hydrierung bei einer Temperatur von 80°C bis 150°C durchgeführt werden.

11. Verfahren zur Herstellung einer Verbindung der Formel (1) gemäß Definition von Anspruch 1 oder eines Salzes hiervon, dadurch gekennzeichnet, daß ein Erythromycylamin mit einem aliphatischen Aldehyd umgesetzt und in situ durch katalytische Hydrierung oder chemische Reduktion bei einem pH-Wert von 4 bis 6 reduziert wird, und das erhaltene Produkt gewünschtenfalls in ein Salz überführt wird.

12. Verfahren nach Anspruch 11 zur Herstellung einer Verbindung gemäß Anspruch 1, worin $R_5$ für $C_1$–$C_5$-Alkyl oder substituiertes $C_1$–$C_5$-Alkyl steht.

13. Verfahren nach Anspruch 11 zur Herstellung einer Verbindung gemäß Anspruch 1, worin $R_5$ für $C_6$–$C_{14}$-Alkyl oder substituiertes $C_6$–$C_{14}$-Alkyl steht.

14. Verfahren nach Anspruch 11 zur Herstellung einer Verbindung gemäß Anspruch 1, worin $R_5$ für $-(CH_2)_lO(CH_2)_mO(CH_2)_nCH_3$ steht.

15. Verfahren zur Herstellung einer Verbindung der Formel (3) gemäß Definition von Anspruch 9 oder eines Salzes hiervon, dadurch gekennzeichnet, daß eine Verbindung der Formel (2a) gemäß Definition von Anspruch 8 mit Formaldehyd umgesetzt und in situ durch katalytische Hydrierung oder chemische Reduktion bei einem pH-Wert von 4 bis 6 unter Bildung einer Verbindung der Formel (3), worin $R_{11}$ für $-N(CH_3)R_7$ steht, reduziert wird und das erhaltene Produkt gewünschtenfalls in ein Salz überführt wird.

16. Verwendung einer Verbindung gemäß Definition von Anspruch 1 für die Herstellung eines Medikaments zur Behandlung von durch gram-positive Bakterien hervorgerufene Infektionen.

**Revendications pour les Etats Contractants: BE, CH, DE, FR, GB, IT, LI, LU, NL, SE**

1. Composé de formule (1):

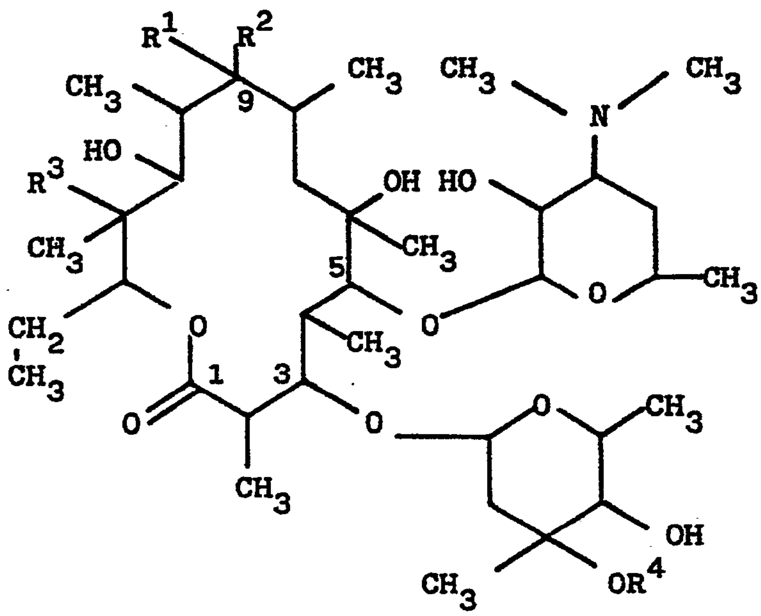

1

dans laquelle $R^1$ et $R^2$ sont différents et représentent chacun un atome d'hydrogène ou $-NHCH_2R^5$;
$R^3$ représente un atome d'hydrogène ou un groupe hydroxyle;
$R^4$ représente un atome d'hydrogène ou un groupe méthyle;et
$R^5$ représente un atome d'hydrogène, un groupe alkyle en $C_1$–$C_{14}$ ou un groupe $-(CH_2)_lX(CH_2)_mY$, chacun de ces groupes pouvant comporter un à trois substituants choisis parmi un atome d'halogène, un groupe hydroxyle, un groupe alkyle en $C_1$–$C_4$, un groupe alcoxy en $C_1$–$C_4$, un groupe alkyl(en $C_1$–$C_4$)thio, un groupe cyano, un groupe alcoxy(en $C_1$–$C_4$)carbonyle, un groupe mono- ou di(alkyl en $C_1$–$C_4$)amino, un groupe $-N(CH_2)_s$, un groupe phényle-$R^6$-substitué ou un groupe monocyclique hétérocyclique $R^6$-substitué contenant 3 à 7 atomes cycliques;
X représente un atome d'oxygène ou un atome de soufre;
Y représente $-X(CH_2)_nCH_3$, $-N(CH_2)_s$ ou $-N[(CH_2)_nCH_3]_2$;
l représente 1 ou 2;
m représente un nombre entier de 1 à 3;
n représente un nombre entier de 0 à 3;
s représente un nombre entier de 2 à 7; et
$R^6$ représente un atome d'hydrogène, un atome d'halogène, un groupe alkyle en $C_1$–$C_4$ ou un groupe alcoxy en $C_1$–$C_4$, avec cette réserve que, lorsque le substituant se trouvant sur le groupe

R⁵ est choisi parmi un groupe hydroxyle, un groupe cyano, un groupe alcoxycarbonyle, un groupe mono- ou dialkylamino ou $-N(CH_2)_s$, il ne peut être situé ni sur le deuxième, ni sur le troisième atome de carbone partant de l'azote du groupe $-NHCH_2R^5$, à moins que le deuxième atome de carbone ne soit quaternaire; ou un de ses sels.

2. Composé selon la revendication 1, dans lequel $R^5$ représente un atome d'hydrogène, un groupe alkyle en $C_1$–$C_{14}$ ou un groupe alkyle en $C_1$–$C_{14}$ substitué.

3. Composé selon la revendication 2, dans lequel $R^5$ représente un groupe alkyle en $C_1$–$C_5$ ou un groupe alkyle en $C_1$–$C_5$ substitué.

4. Composé selon la revendication 2, dans lequel $R^5$ représente un groupe alkyle en $C_6$–$C_{14}$ ou un groupe alkyle en $C_6$–$C_{14}$ substitué.

5. Composé selon la revendication 1, dans lequel $R^5$ représente un groupe $-(CH_2)_lX(CH_2)_mY$ ou un groupe $-(CH_2)_lX(CH_2)_mY$ substitué.

6. Composé selon la revendication 5, dans lequel X représente un atome d'oxygène et Y représente un groupe $-O(CH_2)nCH_3$.

7. Composé de formule (2):

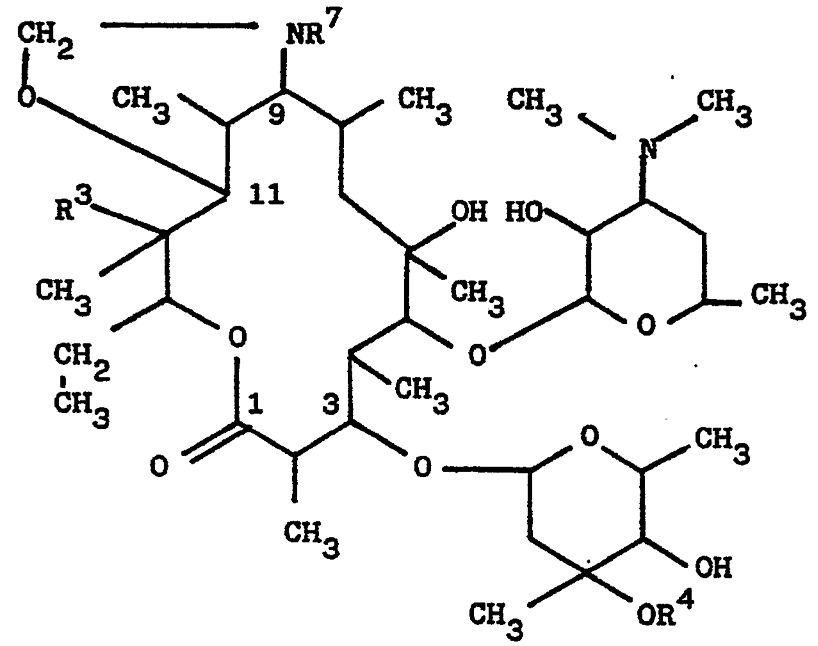

2

dans laquelle

$R^3$ représente un atome d'hydrogène ou un groupe hydroxyle;

$R^4$ représente un atome d'hydrogène ou un groupe méthyle;

$R^7$ représente un groupe $CH_2R^5$, un groupe cycloalkyle en $C_3$–$C_8$, un groupe $-CHR^8(CH_2)_pR^9$, un groupe $-(CH_2)_qR^{10}$ ou un groupe $-CH_2(CH=CH)_rAr$;

$R^5$ représente un atome d'hydrogène, un groupe alkyle en $C_1$–$C_{14}$ ou un groupe $-(CH_2)_lX(CH_2)_mY$, l'un ou l'autre de ces groupes pouvant comporter un à trois substituants choisis parmi un atome d'halogène, un groupe hydroxyle, un groupe alkyle en $C_1$–$C_4$, un groupe alcoxy en $C_1$–$C_4$, un groupe alkyl(en $C_1$–$C_4$)thio, un groupe cyano, un groupe alcoxy(en $C_1$–$C_4$)carbonyle, un groupe mono- ou di(alkyl en $C_1$–$C_4$)amino, un groupe $-N(CH_2)_s$, un groupe phényle-$R^6$ substitué ou un groupe monocyclique hétérocyclique $R^6$-substitué contenant 3 à 7 atomes cycliques;

$R^6$ représente un atome d'hydrogène, un atome d'halogène, un groupe alkyle en $C_1$–$C_4$ ou un groupe alcoxy en $C_1$–$C_4$;

$R^8$ représente un groupe alkyle en $C_1$–$C_4$, un groupe phényle ou un groupe benzyle;

$R^9$ représente un atome d'hydrogène, un atome d'halogène, un groupe hydroxyle, un groupe alcoxy en $C_1$–$C_4$, un groupe mono- ou di(alkyl en $C_1$–$C_4$)amino, un groupe $-N(CH_2)_s$ ou un groupe phényle;

$R^{10}$ représente un groupe hydroxyle, un groupe cyano, un groupe alcoxy(en $C_1$–$C_4$)carbonyle, un groupe mono- ou di(alkyl en $C_1$–$C_4$)amino ou un groupe $-N(CH_2)_s$;

Ar représente un groupe phényle; un groupe phényle comportant un ou plusieurs substituants halogène, alkyle en $C_1$–$C_4$, alcoxy en $C_1$–$C_4$ ou hydroxyle; ou un groupe monocyclique aromatique hétérocyclique $R^6$-substitué contenant 5 à 7 atomes cycliques;
X représente un atome d'oxygène ou un atome de soufre;
Y représente $-X(CH_2)_nCH_3$, un groupe $-N(CH_2)_s$ ou un groupe $-N[(CH_2)_nCH_3]_2$;
l représente 1 ou 2;
m représente un nombre entier de 1 à 3;
n représente un nombre entier de 0 à 3;
p représente un nombre entier de 1 à 5;
q représente 2 ou 3;
r représente o ou 1; et
s représente un nombre entier de 2 à 7;
ou un de ses sels.

8. Composé de formule (3):

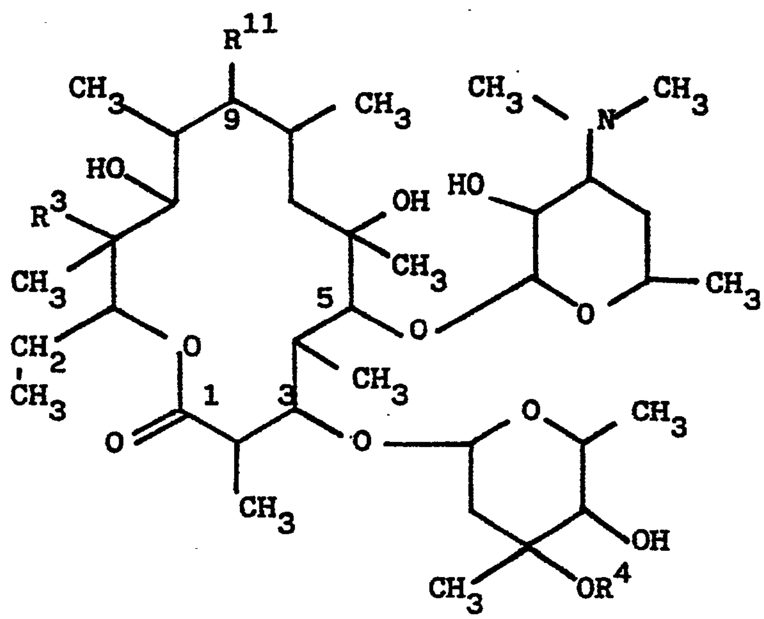

3

dans laquelle
$R^3$ représente un atome d'hydrogène ou un groupe hydroxyle;
$R^4$ représente un atome d'hydrogène ou un groupe méthyle;
$R^{11}$ représente un groupe $-N(CH_3)R^7$ ou un groupe $-N(CH_2)_s$;
$R^7$ représente un groupe $CH_2R^5$, un groupe cycloalkyle en $C_3$–$C_8$, un groupe $-CHR^8(CH_2)_pR^9$, un groupe $-(CH_2)_qR^{10}$ ou un groupe $-CH_2(CH{=}CH)_rAr$;
$R^5$ représente un atome d'hydrogène, un groupe alkyle en $C_1$–$C_{14}$ ou un groupe $-(CH_2)_lX(CH_2)_mY$, l'un ou l'autre de ces groupes pouvant comporter un à trois substituants choisis parmi un atome d'halogène, un groupe hydroxyle, un groupe alkyle en $C_1$–$C_4$, un groupe alcoxy en $C_1$–$C_4$, un groupe alkyl(en $C_1$–$C_4$)thio, un groupe cyano, un groupe alcoxy(en $C_1$–$C_4$)carbonyle, un groupe mono- ou di(alkyl en $C_1$–$C_4$)amino, un groupe $-N(CH_2)_s$, un groupe phényle-$R^6$-substitué ou un groupe monocyclique hétérocyclique $R^6$-substitué contenant 3 à 7 atomes cycliques;
$R^6$ représente un atome d'hydrogène, un atome d'halogène, un groupe alkyle en $C_1$–$C_4$ ou un groupe alcoxy en $C_1$–$C_4$;
$R^8$ représente un groupe alkyle en $C_1$–$C_4$, un groupe phényle ou un groupe benzyle;
$R^9$ représente un atome d'hydrogène, un atome d'halogène, un groupe hydroxyle, un groupe alcoxy en $C_1$–$C_4$, un groupe mono- ou di(alkyl en $C_1$–$C_4$)amino, un groupe $-N(CH_2)_s$ ou un groupe phényle;
$R^{10}$ représente un groupe hydroxyle, un groupe cyano, un groupe alcoxy(en $C_1$–$C_4$)carbonyle, un groupe mono- ou di(alkyl en $C_1$–$C_4$)amino ou un groupe $-N(CH_2)_s$;
Ar représente un groupe phényle; un groupe phényle comportant un ou plusieurs substituants halogène, alkyle en $C_1$–$C_4$, alcoxy en $C_1$–$C_4$ ou hydroxyle; ou un groupe monocyclique aromatique hétérocyclique $R^6$-substitué contenant 5 à 7 atomes cycliques;

X représente un atome d'oxygène ou un atome de soufre;
Y représente un groupe $-X(CH_2)_nCH_3$, un groupe $-N(CH_2)_s$ ou un groupe $-N[(CH_2)_nCH_3]_2$;
l représente 1 ou 2;
m représente un nombre entier de 1 à 3;
n représente un nombre entier de 0 à 3;
p représente un nombre entier de 1 à 5;
q représente 2 ou 3;
r représente 0 ou 1; et
s est un nombre entier de 2 à 7;
ou un de ses sels.

9. Formulation pharmaceutique comprenant, comme ingrédient actif, un composé de formule (1), (2) ou (3) ou un de ses sels pharmaceutiquement acceptables selon la revendication 1, 7 ou 8 respectivement, en association avec un ou plusieurs supports, diluants ou excipients pharmaceutiquement acceptables à cet effet.

10. Composé de formule (1), (2) ou (3), ou un des sels pharmaceutiquement acceptables comme défini dans les revendications 1, 7 ou 8 respectivement, en vue de l'utiliser pour le traitement, la prévention ou le contrôle des maladies infectieuses.

11. La 9-déoxo-9-(n-propylamino)érythromycine.

**Revendications pour l'Etat Contractant: GR**

1. Procédé de préparation d'un composé de formule (1):

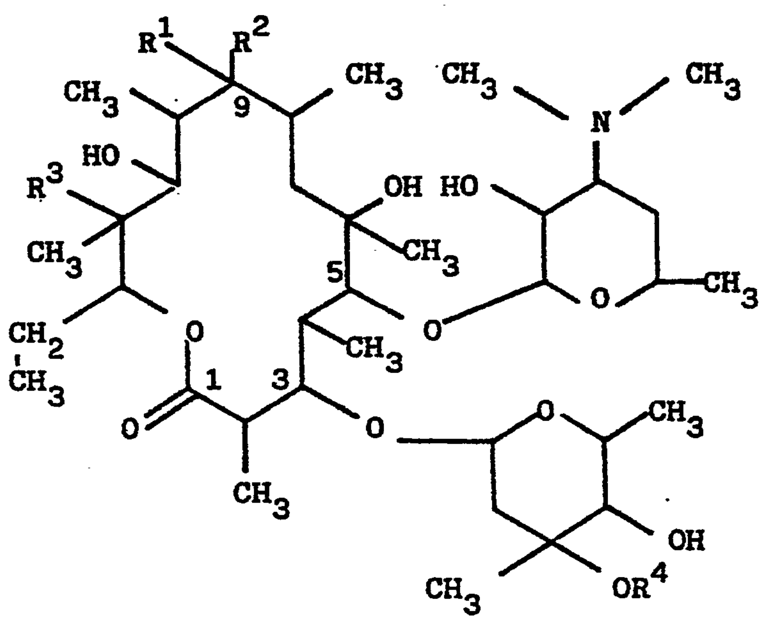

1

dans laquelle
$R^1$ et $R^2$ sont différents et représentent chacun un atome d'hydrogène ou un groupe $-NHCH_2R^5$;
$R^3$ représente un atome d'hydrogène ou un groupe hydroxyle;
$R^4$ représente un atome d'hydrogène ou un groupe méthyle; et
$R^5$ représente un atome d'hydrogène, un groupe alkyle en $C_1$–$C_{14}$ ou un groupe $-(CH_2)_lX(CH_2)_mY$, l'un ou l'autre de ces groupes pouvant comporter un à trois substituants choisis parmi un atome d'halogène, un groupe hydroxyle, un groupe alkyle en $C_1$–$C_4$, un groupe alcoxy en $C_1$–$C_4$, un groupe alkyl(en $C_1$–$C_4$)thio, un groupe cyano, un groupe alcoxy(en $C_1$–$C_4$)carbonyle, un groupe mono- ou di(alkyl en $C_1$–$C_4$)amino, un groupe $-N(CH_2)_s$, un groupe phényle-$R^6$-substitué ou un groupe monocyclique hétérocyclique $R^6$-substitué contenant 3 à 7 atomes cycliques;
X représente un atome d'oxygène ou un atome de soufre;
Y représente un groupe $-X(CH_2)_nCH_3$, un groupe $-N(CH_2)_s$ ou un groupe $-N[(CH_2)_nCH_3]_2$;
l représente 1 ou 2;
m représente un nombre entier de 1 à 3;
n représente un nombre entier de 0 à 3;
s représente un nombre entier de 2 à 7; et

$R^6$ représente un atome d'hydrogène, un atome d'halogène, un groupe alkyle en $C_1$–$C_4$ ou un groupe alcoxy en $C_1$–$C_4$, avec cette réserve que, lorsque le substituant se trouvant sur le groupe $R^5$ est choisi parmi un groupe hydroxyle, un groupe cyano, un groupe alcoxycarbonyle, un groupe mono- ou dialkylamino ou un groupe $-N(CH_2)_s$, il ne peut être situé ni sur le deuxième, ni sur le troisième atome de carbone partant de l'azote du groupe $-NHCH_2R^5$, à moins que le deuxième atome de carbone ne soit quaternaire; ou d'un de ses sels, caractérisé en ce qu'il comprend les étapes qui consistent à réduire, par hydrogénation catalytique ou réduction chimique à un pH de 4 à 6, un composé de formule (1a):

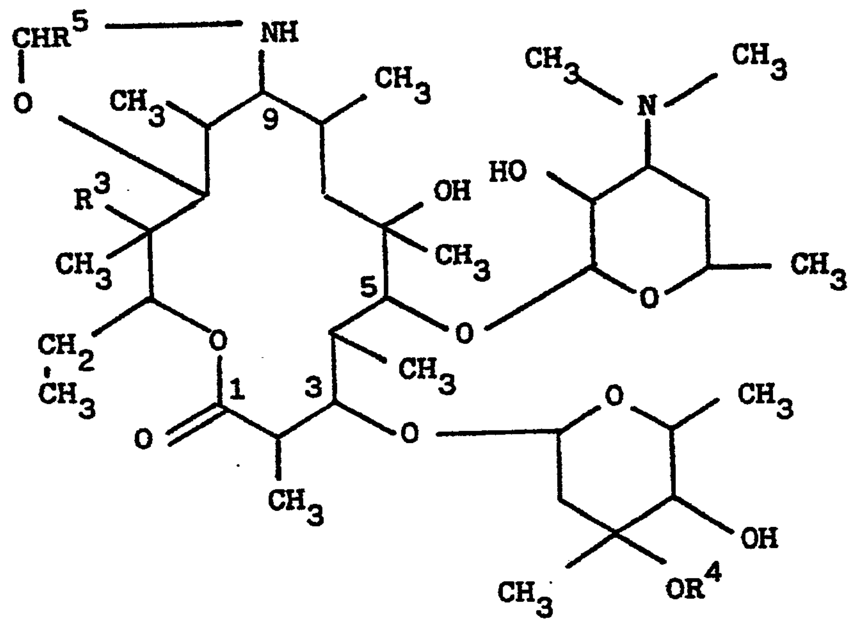

1a

et, éventuellement salifier le produit.

2. Procédé selon la revendication 1, dans lequel l'hydrogénation catalytique est effectuée à une température de 80°C à 150°C.

3. Procédé selon l'une quelconque des revendications 1 et 2 pour la préparation d'un composé selon la revendication 1, où $R^5$ représente un atome d'hydrogène, un groupe alkyle en $C_1$–$C_{14}$ ou un groupe alkyle en $C_1$–$C_{14}$ substitué.

4. Procédé de préparation d'un composé selon la revendication 3, où $R^5$ représente un groupe alkyle en $C_1$–$C_5$ ou un groupe alkyle en $C_1$–$C_5$ substitué.

5. Procédé de préparation d'un composé selon la revendication 3, où $R^5$ représente un groupe alkyle en $C_6$–$C_{14}$ ou un groupe alkyle en $C_6$–$C_{14}$ substitué.

6. Procédé selon l'une quelconque des revendications 1 et 2 pour la préparation d'un composé selon la revendication 1, où $R^5$ représente un groupe $-(CH_2)_lX(CH_2)_mY$ ou un groupe $-(CH_2)_lX(CH_2)_mY$ substitué.

7. Procédé de préparation d'un composé selon la revendication 6, où X représente un atome d'oxygène et Y représente un groupe $-O(CH_2)_nCH_3$.

8. Procédé de préparation d'un composé de formule (2):

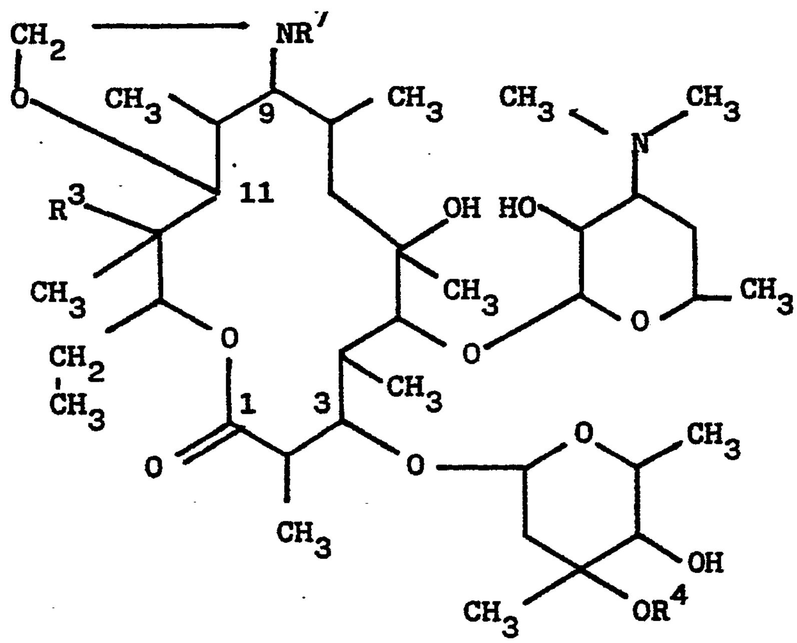

2

dans laquelle

$R^3$ représente un atome d'hydrogène ou un groupe hydroxyle;

$R^4$ représente un atome d'hydrogène ou un groupe méthyle;

$R^7$ représente un groupe $CH_2R^5$, un groupe cycloalkyle en $C_3$–$C_8$, un groupe $-CHR^8(CH_2)_pR^9$, un groupe $-(CH_2)_qR^{10}$ ou un groupe $-CH_2(CH{=}CH)_rAr$;

$R^5$ représente un atome d'hydrogène, un groupe alkyle en $C_1$-$C_{14}$ ou un groupe $-(CH_2)_lX(CH_2)_mY$ l'un ou l'autre de ces groupes pouvant comporter un à trois substituants choisis parmi un atome d'halogène, un groupe hydroxyle, un groupe alkyle en $C_1$–$C_4$, un groupe alcoxy en $C_1$–$C_4$, un groupe alkyl(en $C_1$–$C_4$)thio, un groupe cyano, un groupe alcoxy(en $C_1$–$C_4$)carbonyle, un groupe mono- ou di(alkyl en $C_1$–$C_4$)amino, un groupe $-N(CH_2)_s$, un groupe phényle-$R^6$-substitué ou un groupe monocyclique hétérocyclique $R^6$-substitué contenant 3 à 7 atomes cycliques;

$R^6$ représente un atome d'hydrogène, un atome d'halogène, un groupe alkyle en $C_1$–$C_4$ ou un groupe alcoxy en $C_1$–$C_4$;

$R^8$ représente un groupe alkyle en $C_1$–$C_4$, un groupe phényle ou un groupe benzyle;

$R^9$ représente un atome d'hydrogène, un atome d'halogène, un groupe hydroxyle, un groupe alcoxy en $C_1$–$C_4$, un groupe mono- ou di(alkyl en $C_1$–$C_4$)amino, un groupe $-N(CH_2)_s$ ou un groupe phényle;

$R^{10}$ représente un groupe hydroxyle, un groupe cyano, un groupe alcoxy(en $C_1$–$C_4$)carbonyle, un groupe mono- ou di(alkyl en $C_1$–$C_4$)amino ou un groupe $-N(CH_2)_s$;

Ar représente un groupe phényle, un groupe phényle comportant un ou plusieurs substituants halogène, alkyle en $C_1$–$C_4$, alcoxy en $C_1$–$C_4$ ou hydroxyle; ou un groupe monocyclique aromatique hétérocyclique $R^6$-substitué contenant 5 à 7 atomes cycliques;

X représente un atome d'oxygène ou un atome de soufre;

Y représente un groupe $-X(CH_2)_nCH_3$, un groupe $-N(CH_2)_s$ ou un groupe $-N[(CH_2)_nCH_3]_2$;

l représente 1 ou 2;

m représente un nombre entier de 1 à 3;

n représente un nombre entier de 0 à 3;

p représente un nombre entier de 1 à 5;

q représente 2 ou 3;

r représente 0 ou 1; et

s représente un nombre entier de 2 à 7;

ou d'un de ses sels, caractérisé en ce qu'il consiste à faire réagir un composé de formule (2a):

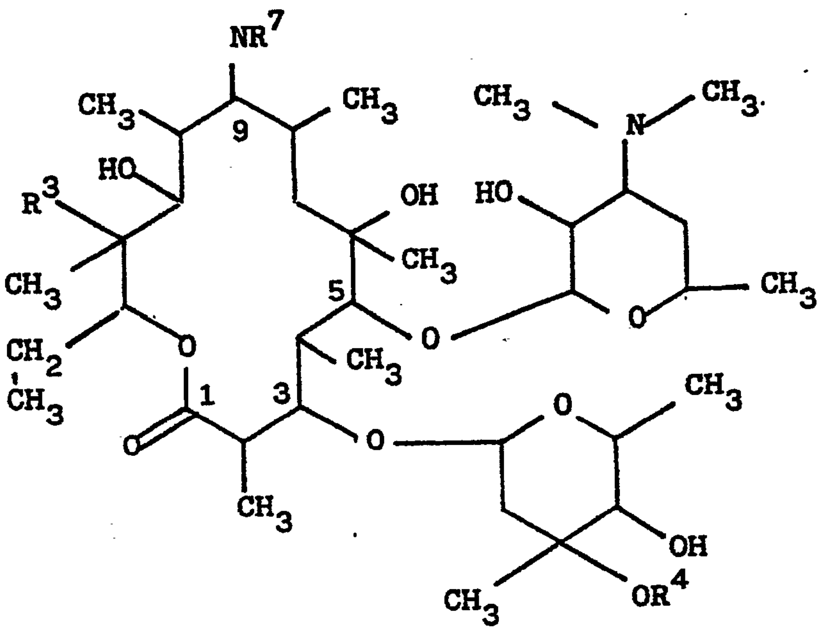

2a

dans laquelle $R^3$, $R^4$ et $R^7$ ont les significations définies ci-dessus, avec du formaldéhyde.

9. Procédé de préparation d'un composé de formule (3):

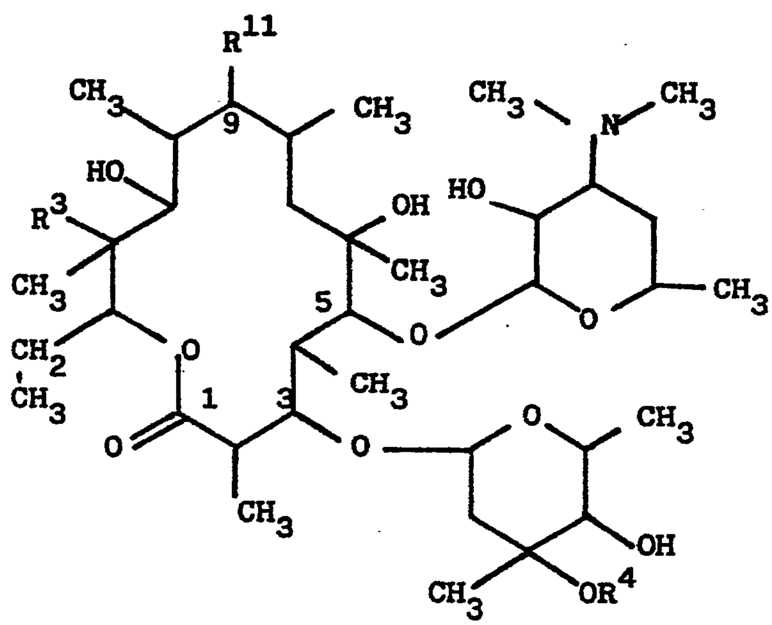

3

dans laquelle

$R^3$ représente un atome d'hydrogène ou un groupe hydroxyle;

$R^4$ représente un atome d'hydrogène ou un groupe méthyle;

$R^{11}$ représente un groupe $-N(CH_3)R^7$ ou un groupe $-N(CH_2)_s$;

$R^7$ représente un groupe $CH_2R^5$, un groupe cycloalkyle en $C_3$–$C_8$, un groupe $-CHR^8(CH_2)_pR^9$, un groupe $-(CH_2)_qR^{10}$ ou un groupe $-CH_2(CH=CH)_rAr$;

$R^5$ représente un atome d'hydrogène, un groupe alkyle en $C_1$–$C_{14}$ ou un groupe $-(CH_2)_lX(CH_2)_mY$,

l'un ou l'autre de ces groupes pouvant comporter un à trois substituants choisis parmi un atome d'halogène, un groupe hydroxyle, un groupe alkyle en $C_1$–$C_4$, un groupe alcoxy en $C_1$–$C_4$, un groupe alkyl(en $C_1$–$C_4$)thio, un groupe cyano, un groupe alcoxy(en $C_1$–$C_4$)carbonyle, un groupe mono- ou di(alkyl en $C_1$–$C_4$)amino, un groupe $-N(CH_2)_s$, un groupe phényle-$R^6$-substitué ou un groupe monocyclique hétérocyclique $R^6$-substitué contenant 3 à 7 atomes cycliques;

$R^6$ représente un atome d'hydrogène, un atome d'halogène, un groupe alkyle en $C_1$–$C_4$ ou un groupe alcoxy en $C_1$–$C_4$;

$R^8$ représente un groupe alkyle en $C_1$–$C_4$, un groupe phényle ou un groupe benzyle;

$R^9$ représente un atome d'hydrogène, un atome d'halogène, un groupe hydroxyle, un groupe alcoxy en $C_1$–$C_4$, un groupe mono- ou di(alkyl en $C_1$–$C_4$)amino, un groupe $-N(CH_2)_s$ ou un groupe phényle;

$R^{10}$ représente un groupe hydroxyle, un groupe cyano, un groupe alcoxy(en $C_1$–$C_4$)carbonyle, un groupe mono- ou di(alkyl en $C_1$–$C_4$)amino ou un groupe $-N(CH_2)_s$;

Ar représente un groupe phényle, un groupe phényle contenant un ou plusieurs substituants halogène, alkyle en $C_1$–$C_4$, alcoxy en $C_1$–$C_4$ ou hydroxyle; ou un groupe monocyclique aromatique hétérocyclique $R^6$-substitué contenant 5 à 7 atomes cycliques;

X représente un atome d'oxygène ou un atome de soufre;

Y représente un groupe $-X(CH_2)_nCH_3$,un groupe $-N(CH_2)_s$ ou un groupe $-N[(CH_2)_nCH_3]_2$;

l représente 1 ou 2;

m représente un nombre entier de 1 à 3;

n représente un nombre entier de 0 à 3;

p représente un nombre entier de 1 à 5;

q représente 2 ou 3;

r représente 0 ou 1; et

s représente un nombre entier de 2 à 7;

ou d'un de ses sels, caractérisé en ce qu'il comprend les étapes qui consistent à:

(a) réduire, par hydrogénation catalytique ou réduction chimique à un pH de 4 à 6, un composé de formule (2) comme défini dans la revendication 7 en vue de former un composé de formule (3) où $R^{11}$ représente un groupe $-N(CH_3)R^7$ et, si on le désire, salifier le produit; ou

(b) faire réagir une érythromycylamine avec un dialdéhyde de formule $OHC-(CH_2)_b-CHO$ où b est un nombre entier de 1 à 5, puis réduire le produit par hydrogénation catalytique ou réduction chimique à un pH de 4 à 6;

et, éventuellement salifier le produit.

10. Procédé selon la revendication 9, dans lequel les étapes d'hydrogénation catalytique sont effectuées à une température de 80 à 150°C.

11. Procédé de préparation d'un composé de formule (1) selon la revendication 1, ou d'un de ses sels, caractérisé en ce qu'il consiste à faire réagir une érythromycylamine avec un aldéhyde aliphatique et réduire, in situ, par hydrogénation catalytique ou une réduction chimique à un pH de 4 à 6, et éventuellement salifier le produit.

12. Procédé selon la revendication 11 pour la préparation d'un composé selon la revendication 1, où $R^5$ représente un groupe alkyle en $C_1$–$C_5$ ou un groupe alkyle en $C_1$–$C_5$ substitué.

13. Procédé selon la revendication 11 pour la préparation d'un composé selon la revendication 1, où $R^5$ représente un groupe alkyle en $C_6$–$C_{14}$ ou un groupe alkyle en $C_6$–$C_{14}$ substitué.

14. Procédé selon la revendication 11 pour la préparation d'un composé selon la revendication 1, où $R^5$ représente un groupe $-(CH_2)_lO(CH_2)_mO(CH_2)_nCH_3$.

15. Procédé de préparation d'un composé de formule (3) selon la revendication 9 ou d'un de ses sels, caractérisé en ce qu'il consiste à faire réagir un composé de formule (2a) selon la revendication 8, avec du formaldéhyde et réduire, in situ, par hydrogénation catalytique ou réduction chimique à un pH de 4 à 6, pour obtenir un composé de formule (3) dans laquelle $R^{11}$ représente un groupe $-N(CH_3)R^7$ et éventuellement salifier le produit.

16. Composé de formule (1a) selon la revendication 1, ou un de ses sels.

17. Composé de formule (2a) selon la revendication 8, ou un de ses sels.

18. Composé de formule (1) selon la revendication 1, ou un de ses sels.

19. Composé de formule (2) selon la revendication 8, ou un de ses sels.

**Revendications pour les Etats Contractants: AT, ES**

1. Procédé de préparation d'un composé de formule (1);

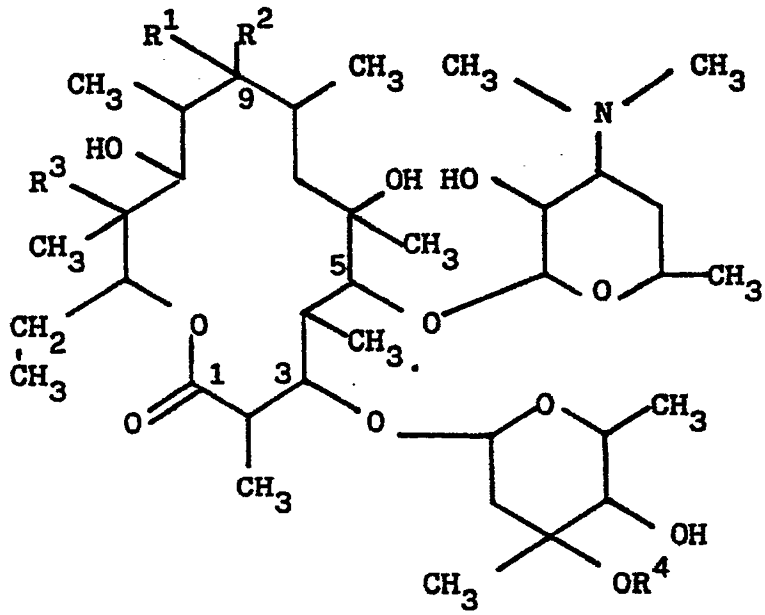

1

dans laquelle

$R^1$ et $R^2$ sont différents et représentent chacun un atome d'hydrogène ou un groupe $-NHCH_2R^5$;

$R^3$ représente un atome d'hydrogène ou un groupe hydroxyle;

$R^4$ représente un atome d'hydrogène ou un groupe méthyle; et

$R^5$ représente un atome d'hydrogène, un groupe alkyle en $C_1$–$C_{14}$ ou un groupe $-(CH_2)_lX(CH_2)_mY$, l'un ou l'autre de ces groupes pouvant comporter un à trois substituants choisis parmi un atome d'halogène, un groupe hydroxyle, un groupe alkyle en $C_1$–$C_4$, un groupe alcoxy en $C_1$–$C_4$, un groupe alkyl(en $C_1$–$C_4$)thio, un groupe cyano, un groupe alcoxy(en $C_1$–$C_4$)carbonyle, un groupe mono- ou di(alkyl en $C_1$–$C_4$)amino, un groupe $-N(CH_2)_s$, un groupe phényle-$R^6$-substitué ou un groupe monocyclique hétérocyclique $R^6$-substitué contenant 3 à 7 atomes cycliques;

X représente un atome d'oxygène ou un atome de soufre;

Y représente un groupe $-X(CH_2)_nCH_3$, un groupe $-N(CH_2)_s$ ou un groupe $-N[(CH_2)_nCH_3]_2$;

l représente 1 ou 2;

m représente un nombre entier de 1 à 3;

n représente un nombre entier de 0 à 3;

s représente un nombre entier de 2 à 7; et

$R^6$ représente un atome d'hydrogène, un atome d'halogène, un groupe alkyle en $C_1$–$C_4$ ou un groupe alcoxy en $C_1$–$C_4$, avec cette réserve que, lorsque le substituant se trouvant sur le groupe $R^5$ est choisi parmi un groupe hydroxyle, un groupe cyano, un groupe alcoxycarbonyle, un groupe mono- ou dialkylamino ou un groupe $-N(CH_2)_s$, il ne peut être situé ni sur le deuxième, ni sur le troisième atome de carbone partant de l'azote du groupe $-NHCH_2R^5$, à moins que le deuxième atome de carbone ne soit quaternaire;

ou d'un de ses sels, caractérisé en ce qu'il consiste à réduire, par hydrogénation catalytique ou réduction chimique, à un pH de 4 à 6, un composé de formule (1a):

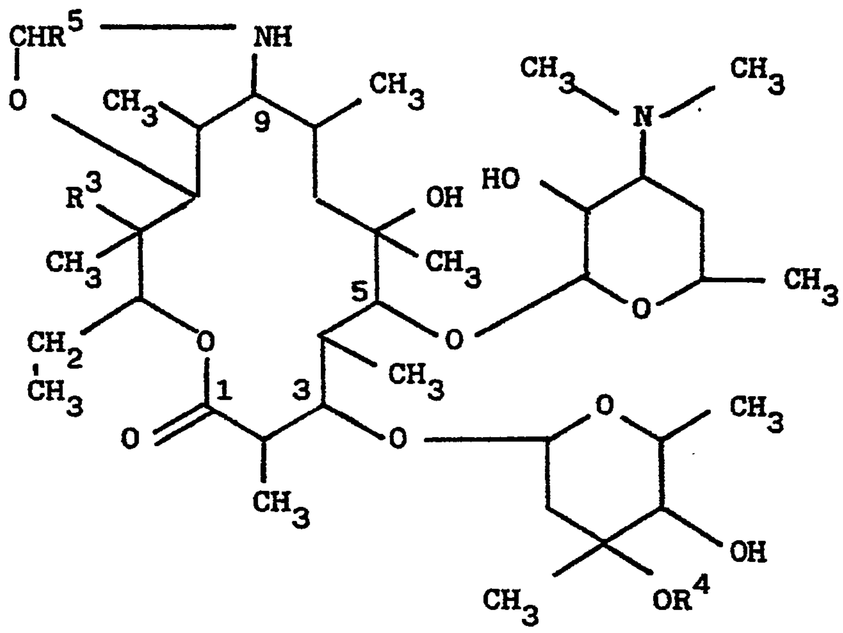

1a

et, éventuellement salifier le produit.

2. Procédé selon la revendication 1, dans lequel l'hydrogénation catalytique est effectuée à une température de 80°C à 150°C.

3. Procédé selon l'une quelconque des revendications 1 et 2 pour la préparation d'un composé selon la revendication 1, où $R^5$ représente un atome d'hydrogène, un groupe alkyle en $C_1$–$C_{14}$ ou un groupe alkyle en $C_1$–$C_{14}$ substitué.

4. Procédé de préparation d'un composé selon la revendication 3, où $R^5$ représente un groupe alkyle en $C_1$–$C_5$ ou un groupe alkyle en $C_1$–$C_5$ substitué.

5. Procédé de préparation d'un composé selon la revendication 3, où $R^5$ représente un groupe alkyle en $C_6$–$C_{14}$ ou un groupe alkyle en $C_6$–$C_{14}$ substitué.

6. Procédé selon l'une quelconque des revendications 1 et 2 pour la préparation d'un composé selon la revendication 1, où $R^5$ représente un groupe $-(CH_2)_lX(CH_2)_mY$ ou un groupe $-(CH_2)_lX(CH_2)_mY$ substitué.

7. Procédé pour la préparation d'un composé selon la revendication 6, où X représente un atome d'oxygène et Y représente un groupe $-O(CH_2)_nCH_3$.

8. Procédé de préparation d'un composé de formule (2):

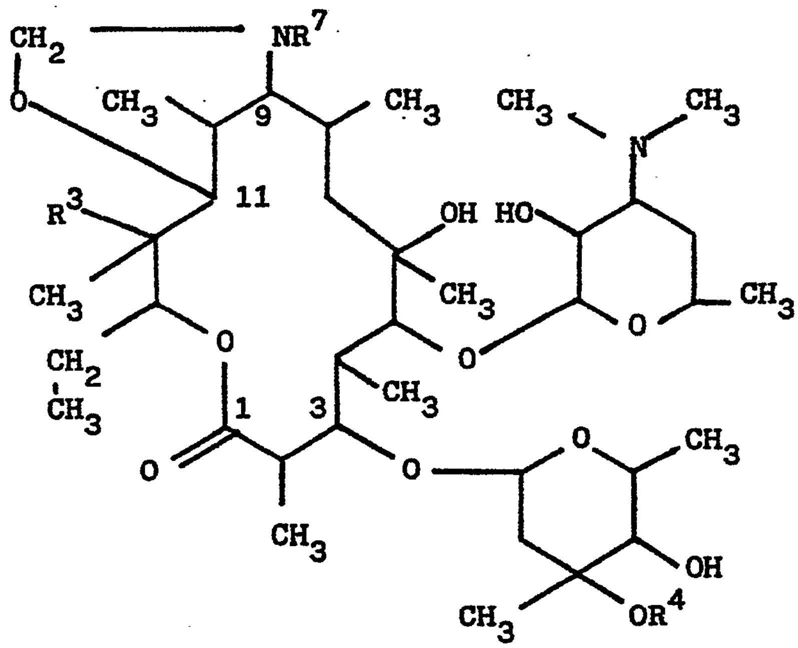

2

dans laquelle

$R^3$ représente un atome d'hydrogène ou un groupe hydroxyle;

$R^4$ représente un atome d'hydrogène ou un groupe méthyle;

$R^7$ représente un groupe $CH_2R^5$, un groupe cycloalkyle en $C_3$–$C_8$, un groupe $-CHR^8(CH_2)_pR^9$, un groupe $-(CH_2)_qR^{10}$ ou un groupe $-CH_2(CH=CH)_rAr$;

$R^5$ représente un atome d'hydrogène, un groupe alkyle en $C_1$–$C_{14}$ ou un groupe $-(CH_2)_lX(CH_2)_mY$, l'un ou l'autre de ces groupes pouvant comporter un à trois substituants choisis parmi un atome d'halogène, un groupe hydroxyle, un groupe alkyle en $C_1$–$C_4$, un groupe alcoxy en $C_1$–$C_4$, un groupe alkyl(en $C_1$–$C_4$)thio, un groupe cyano, un groupe alcoxy(en $C_1$–$C_4$)carbonyle, un groupe mono- ou di(alkyl en $C_1$–$C_4$)amino, un groupe $-N(CH_2)_s$, un groupe phényle-$R^6$-substitué ou un groupe monocyclique hétérocyclique $R^6$-substitué contenant 3 à 7 atomes cycliques;

$R^6$ représente un atome d'hydrogène, un atome d'halogène, un groupe alkyle en $C_1$–$C_4$ ou un groupe alcoxy en $C_1$–$C_4$;

$R^8$ représente un groupe alkyle en $C_1$–$C_4$, un groupe phényle ou un groupe benzyle;

$R^9$ représente un atome d'hydrogène, un atome d'halogène, un groupe hydroxyle, un groupe alcoxy en $C_1$–$C_4$, un groupe mono- ou di(alkyl en $C_1$–$C_4$)amino, un groupe $-N(CH_2)_s$ ou un groupe phényle;

$R^{10}$ représente un groupe hydroxyle, un groupe cyano, un groupe alcoxy(en $C_1$–$C_4$)carbonyle, un groupe mono- ou di(alkyl en $C_1$–$C_4$)amino ou un groupe $-N(CH_2)_s$;

Ar représente un groupe phényle; un groupe phényle comportant un ou plusieurs substituants halogène, alkyle en $C_1$–$C_4$, alcoxy en $C_1$–$C_4$ ou hydroxyle; ou encore un groupe monocyclique aromatique hétérocyclique $R^6$-substitué comportant 5 à 7 atomes cycliques;

X représente un atome d'oxygène ou un atome de soufre;

Y représente un groupe $-X(CH_2)_nCH_3$, un groupe $-N(CH_2)_s$ ou un groupe $-N[(CH_2)_nCH_3]_2$

l représente 1 ou 2;

m représente un nombre entier de 1 à 3;

n représente un nombre entier de 0 à 3;

p représente un nombre entier de 1 à 5;

q représente 2 ou 3;

r représente 0 ou 1; et

s représente un nombre entier de 2 à 7;

ou d'un de ses sels, caractérisé en ce qu'il consiste à:

faire réagir un composé de formule (2a):

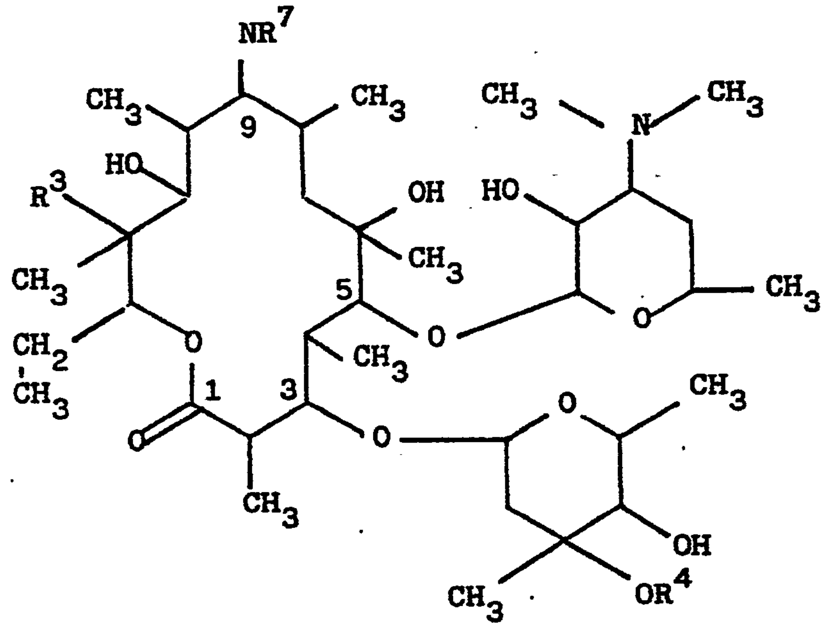

2a

dans laquelle $R^3$, $R^4$ et $R^7$ ont les significations définies ci-dessus, avec du formaldéhyde.

9. Procédé de préparation d'un composé de formule (3):

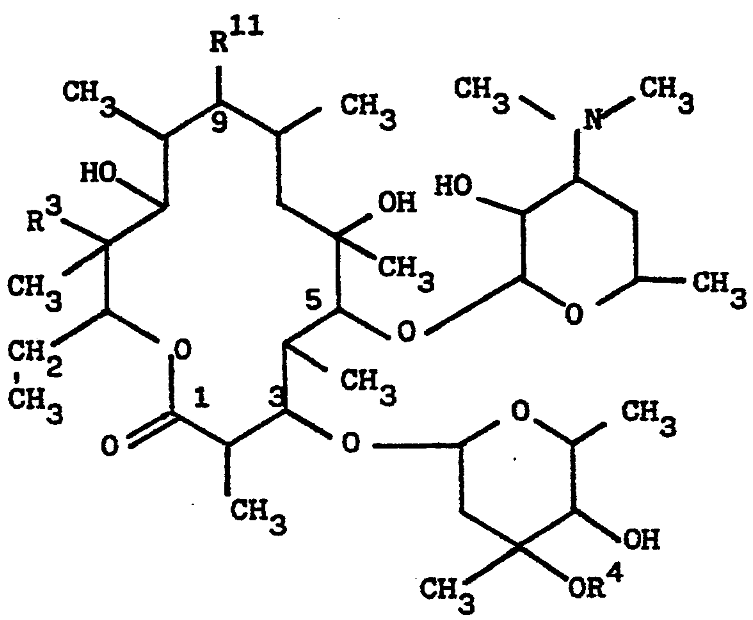

3

dans laquelle
$R^3$ représente un atome d'hydrogène ou un groupe hydroxyle;
$R^4$ représente un atome d'hydrogène ou un groupe méthyle;
$R^{11}$ représente $-N(CH_3)R^7$ ou $-N(CH_2)_s$;
$R^7$ représente un groupe $CH_2R^5$, un groupe cycloalkyle en $C_3$–$C_8$, un groupe $-CHR^8(CH_2)_pR^9$, un groupe $-(CH_2)_qR^{10}$ ou un groupe $-CH_2(CH{=}CH)_rAr$;

$R^5$ représente un atome d'hydrogène, un groupe alkyle en $C_1$–$C_{14}$ ou un groupe $-(CH_2)_lX(CH_2)_mY$, l'un ou l'autre de ces groupes pouvant comporter un à trois substituants choisis parmi un atome d'halogène, un groupe hydroxyle, un groupe alkyle en $C_1$–$C_4$, un groupe alcoxy en $C_1$–$C_4$, un groupe alkyl(en $C_1$–$C_4$)thio, un groupe cyano, un groupe alcoxy(en $C_1$–$C_4$)carbonyle, un groupe mono- ou di(alkyl en $C_1$–$C_4$)amino, un groupe $-N(CH_2)_s$, un groupe phényle-$R^6$-substitué ou un groupe monocyclique hétérocyclique $R^6$-substitué contenant 3 à 7 atomes cycliques;
$R^6$ représente un atome d'hydrogène, un atome d'halogène, un groupe alkyle en $C_1$–$C_4$ ou un groupe alcoxy en $C_1$–$C_4$;
$R^8$ représente un groupe alkyle en $C_1$–$C_4$, un groupe phényle ou un groupe benzyle;
$R^9$ représente un atome d'hydrogène, un atome d'halogène, un groupe hydroxyle, un groupe alcoxy en $C_1$–$C_4$, un groupe mono- ou di(alkyl en $C_1$–$C_4$)amino, un groupe $-N(CH_2)_s$ ou un groupe phényle;
$R^{10}$ représente un groupe hydroxyle, un groupe cyano, un groupe alcoxy(en $C_1$–$C_4$)carbonyle un groupe mono- ou di(alkyl en $C_1$–$C_4$)amino ou un groupe $-N(CH_2)_s$;
Ar represnte un groupe phényle, un groupe phényle comportant un ou plusieurs substituants halogène, alkyle en $C_1$–$C_4$, alcoxy en $C_1$–$C_4$ ou hydroxyle, ou encore un groupe monocyclique aromatique hétérocyclique $R^6$-substitué comportant 5 à 7 atomes cycliques;
X représente un atome d'oxygène ou un atome de soufre;
Y représente un groupe $-X(CH_2)_nCH_3$, un groupe $-N(CH_2)_s$ ou un groupe $-N[(CH_2)_n)CH_3]_2$;
l représente 1 ou 2;
m représente un nombre entier de 1 à 3;
n représente un nombre entier de 0 à 3;
p représente un nombre entier de 1 à 5;
q représente 2 ou 3;
r représente 0 ou 1; et
s représente un nombre entier de 2 à 7;
ou d'un de ses sels, caractérisé en ce qu'il comprend les étapes qui consistent à

(a) réduire par hydrogénation catalytique ou réduction chimique à un pH de 4 à 6, un composé de formule (2) comme défini dans la revendication 7, pour obtenir un composé de formule (3) dans laquelle $R^{11}$ représente un groupe $-N(CH_3)R^7$ et, si on le désire, salifier le produit; ou

(b) faire réagir une érythromycylamine avec un dialdéhyde de formule $OHC-(CH_2)_b-CHO$ où b représente un nombre entier de 1 à 5, puis réduire le produit, par hydrogénation catalytique ou réduction chimique, à un pH de 4 à 6;

et, si on le désire, salifier le produit.

10. Procédé selon la revendication 9, dans lequel les étapes d'hydrogénation catalytique sont effectuées à une température de 80°C à 150°C.

11. Procédé de préparation d'un composé de formule (1) selon la revendication 1, ou d'un de ses sels, caractérisé en ce qu'il consiste à faire réagir une érythromycylamine avec un aldéhyde aliphatique et réduire, in situ, par hydrogénation catalytique ou réduction chimique, à un pH de 4 à 6 et, si on le désire, salifier le produit.

12. Procédé selon la revendication 11, pour la préparation d'un composé selon la revendication 1, où $R^5$ représente un groupe alkyle en $C_1$–$C_5$ ou un groupe alkyle en $C_1$–$C_5$ substitué.

13. Procédé selon la revendication 11, pour la préparation d'un composé selon la revendication 1, où $R^5$ représente un groupe alkyle en $C_6$–$C_{14}$ ou un groupe alkyle en $C_6$–$C_{14}$ substitué.

14. Procédé selon la revendication 11, pour la préparation d'un composé selon la revendication 1, où $R^5$ représente un groupe $-(CH_2)_lO(CH_2)_mO(CH_2)_nCH_3$.

15. Procédé pour la préparation d'un composé de formule (3) selon la revendication 9, ou d'un de ses sels, caractérisé en ce qu'il consiste à faire réagir un composé de formule (2a) comme défini dans la revendication 8, avec du formaldéhyde et réduire, in situ, par hydrogénation catalytique ou réduction chimique à un pH de 4 à 6, pour obtenir un composé de formule (3) dans laquelle $R^{11}$ représente un groupe $-N(CH_3)R^7$ et, éventuellement, salifier le produit.

16. Utilisation d'un composé tel que défini dans la revendication 1 pour la fabrication d'un médicament en vue du traitement des infections provoquées par les bactéries gram-positives.